(19) Europäisches Patentamt
European Patent Office
Office européen des brevets

(11) EP 1 082 416 B1

(12) EUROPÄISCHE PATENTSCHRIFT

(45) Veröffentlichungstag und Bekanntmachung des Hinweises auf die Patenterteilung:
**28.03.2007 Patentblatt 2007/13**

(21) Anmeldenummer: **99929144.6**

(22) Anmeldetag: **04.06.1999**

(51) Int Cl.:
*C12N 9/10* (2006.01) *C12N 15/54* (2006.01)
*C07K 14/455* (2006.01) *C12N 15/10* (2006.01)
*C12Q 1/68* (2006.01) *G01N 33/53* (2006.01)
*A61K 48/00* (2006.01) *A01K 67/027* (2006.01)
*A61K 31/70* (2006.01) *A61K 38/45* (2006.01)

(86) Internationale Anmeldenummer:
**PCT/EP1999/003889**

(87) Internationale Veröffentlichungsnummer:
**WO 1999/064572 (16.12.1999 Gazette 1999/50)**

(54) **POLY(ADP-RIBOSE)POLYMERASE-GENE**
POLY(ADP-RIBOSE)POLYMERASE GENE
GENES DE POLY(ADP-RIBOSE)POLYMERASE

(84) Benannte Vertragsstaaten:
**AT BE CH DE DK ES FI FR GB GR IE IT LI LU NL PT SE**
Benannte Erstreckungsstaaten:
**RO SI**

(30) Priorität: **05.06.1998 DE 19825213**
**01.03.1999 DE 19908837**

(43) Veröffentlichungstag der Anmeldung:
**14.03.2001 Patentblatt 2001/11**

(73) Patentinhaber: **Abbott GmbH & Co. KG**
**65205 Wiesbaden (DE)**

(72) Erfinder:
- **KOCK, Michael**
**D-67105 Schifferstadt (DE)**
- **HÖGER, Thomas**
**D-68535 Edingen-Neckarhausen (DE)**
- **KRÖGER, Burkhard**
**D-67117 Limburgerhof (DE)**
- **OTTERBACH, Bernd**
**D-67061 Ludwigshafen (DE)**
- **LUBISCH, Wilfried**
**D-69115 Heidelberg (DE)**
- **LEMAIRE, Hans-Georg**
**D-67117 Limburgerhof (DE)**

(74) Vertreter: **Kinzebach, Werner et al**
**Ludwigsplatz 4**
**67059 Ludwigshafen (DE)**

(56) Entgegenhaltungen:
**WO-A-96/18737** **FR-A- 2 707 011**
**US-A- 5 272 057**

- **DATABASE EMBL NUCLEOTIDE AND PROTEIN SEQUENCES,23. Februar 1998 (1998-02-23), XP002129091 HINXTON, GB**
- **DATABASE EMBL NUCLEOTIDE AND PROTEIN SEQUENCES,15. Dezember 1996 (1996-12-15), XP002129092 HINXTON, GB**
- **GRIFFIN R J ET AL: "NOVEL POTENT INHIBITORS OF THE DNA REPAIR ENZYME POLY (ADP-RIBOSE)POLYMERASE (PARP)" ANTI-CANCER DRUG DESIGN,GB,BASINGSTOKE, Bd. 10, Nr. 6, 1995, Seite 507-514 XP002065156 ISSN: 0266-9536 in der Anmeldung erwähnt**
- **LEPINIEC L ET AL., : "Characterization of an Arabidopsis thaliana cDNA homologue to animal poly(ADP-ribose) polymerase" FEBS LETTERS, Bd. 364, 1995, Seiten 103-108, XP000867423 in der Anmeldung erwähnt**
- **BENEKE S. ET AL., : "Isolation of cDNA encoding full-length rat (Rattus norvegicus) poly (ADP-ribose) polymerase" BIOCHEM. MOL. BIOL. INT., Bd. 43, 1997, Seite 755-761 XP000870495**
- **WANG Z-Q ET AL., : "PARP is important for genomic stability but dispensable in apoptosis" GENES AND DEVELOPMENT, Bd. 11, 1997, Seiten 2347-2358, XP002129093**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- KUEPPER J-H AND BUERKLE A.: "EXPRESSION OF THE DNA-BINDING DOMAIN OF HUMAN POLY (ADP-RIBOSE) POLYMERASE AS A TRANS-DOMINANT INHIBITOR OF POLY(ADP-RIBOSYL) ATION IN TRANSFECTED EUCARYOTIC CELL LINES" 1992 , POIRIER, G.G. AND MOREAU P., EDS , SPRINGER, NEW YORK XP000568905 Seite 38-46 Seite 45
- JOHANSSON M.: "A human Poly(ADP-ribose) polymerase gene family (ADPRTL): cDNA cloning of two novel Poly(ADP-ribose) polymerase homologues" GENOMICS, Bd. 57(3), 1999, Seite 442-445 XP000870418
- BERGHAMMER H.A. ET AL., : "pADPRT-2: a novel mammalian polymerizing(ADP-ribosyl) transerase gene related to truncated pADPRT homologues in plant and Caernorhibditis elegans" FEBS LETT., Bd. 449, 23. April 1999 (1999-04-23), Seite 259-263 XP000867424
- AME J-C. ET AL., : "Identification and characterization of a new isoform of poly(ADP-ribose) polymerase: PARP-2" J. BIOL. CHEM. , Bd. 274, 18. Juni 1999 (1999-06-18), Seite 17860-17868 XP002129094

SEQUENZPROTOKOLL

(1) ALLGEMEINE ANGABEN:

(i) ANMELDER:

(A) NAME: BASF Aktiengesellschaft
(B) STRASSE:
(C) ORT: Ludwigshafen
(E) LAND: Deutschland
(F) POSTLEITZAHL: 67065

(ii) BEZEICHNUNG DER ERFINDUNG: Neue Poly ADP Ribose Polymerase Gene

(iii) ANZAHL DER SEQUENZEN: 28

(iv) COMPUTER LESBARE FASSUNG:

(A) DATENTRÄGER: Floppy disk
(B) COMPUTER: IBM PC compatible
(C) BETRIEBSSYSTEM: PC DOS/MS DOS
(D) SOFTWARE: PatentIn Release #1.0, Version #1.30 (EPA)

(2) ANGABEN ZU SEQ ID NO: 1:

(i) SEQUENZKENNZEICHEN:

(A) LÄNGE: 1843 Basenpaare
(B) ART: Nucleotid
(C) STRANGFORM: Einzelstrang
(D) TOPOLOGIE: linear

(ii) ART DES MOLEKÜLS: cDNA

(iii) HYPOTHETISCH: NEIN

(iv) ANTISENSE: NEIN

(vi) URSPRÜNLICHE HERKUNFT:

(F) GEWEBETYP: Brain

(ix) MERKMAL:

(A) NAME/SCHLÜSSEL: CDS
(B) LAGE:3..1715
(D) SONSTIGE ANGABEN:/product= "Poly ADP Ribose Polymerase"

(xi) SEQUENZBESCHREIBUNG: SEQ ID NO: 1:

```
CC ATG GCG GCG CGG CGG CGA CGG AGC ACC GGC GGC GGC AGG GCG AGA         47
   Met Ala Ala Arg Arg Arg Arg Ser Thr Gly Gly Gly Arg Ala Arg
     1               5                  10                  15

GCA TTA AAT GAA AGC AAA AGA GTT AAT AAT GGC AAC ACG GCT CCA GAA        95
Ala Leu Asn Glu Ser Lys Arg Val Asn Asn Gly Asn Thr Ala Pro Glu
                 20                  25                  30
```

```
GAC TCT TCC CCT GCC AAG AAA ACT CGT AGA TGC CAG AGA CAG GAG TCG      143
Asp Ser Ser Pro Ala Lys Lys Thr Arg Arg Cys Gln Arg Gln Glu Ser
             35                  40                  45

AAA AAG ATG CCT GTG GCT GGA GGA AAA GCT AAT AAG GAC AGG ACA GAA      191
Lys Lys Met Pro Val Ala Gly Gly Lys Ala Asn Lys Asp Arg Thr Glu
         50                  55                  60

GAC AAG CAA GAT GAA TCT GTG AAG GCC TTG CTG TTA AAG GGC AAA GCT      239
Asp Lys Gln Asp Glu Ser Val Lys Ala Leu Leu Leu Lys Gly Lys Ala
     65                  70                  75

CCT GTG GAC CCA GAG TGT ACA GCC AAG GTG GGG AAG GCT CAT GTG TAT      287
Pro Val Asp Pro Glu Cys Thr Ala Lys Val Gly Lys Ala His Val Tyr
 80                  85                  90                  95

TGT GAA GGA AAT GAT GTC TAT GAT GTC ATG CTA AAT CAG ACC AAT CTC      335
Cys Glu Gly Asn Asp Val Tyr Asp Val Met Leu Asn Gln Thr Asn Leu
                100                 105                 110

CAG TTC AAC AAC AAC AAG TAC TAT CTG ATT CAG CTA TTA GAA GAT GAT      383
Gln Phe Asn Asn Asn Lys Tyr Tyr Leu Ile Gln Leu Leu Glu Asp Asp
            115                 120                 125

GCC CAG AGG AAC TTC AGT GTT TGG ATG AGA TGG GGC CGA GTT GGG AAA      431
Ala Gln Arg Asn Phe Ser Val Trp Met Arg Trp Gly Arg Val Gly Lys
        130                 135                 140

ATG GGA CAG CAC AGC CTG GTG GCT TGT TCA GGC AAT CTC AAC AAG GCC      479
Met Gly Gln His Ser Leu Val Ala Cys Ser Gly Asn Leu Asn Lys Ala
    145                 150                 155

AAG GAA ATC TTT CAG AAG AAA TTC CTT GAC AAA ACG AAA AAC AAT TGG      527
Lys Glu Ile Phe Gln Lys Lys Phe Leu Asp Lys Thr Lys Asn Asn Trp
160                 165                 170                 175

GAA GAT CGA GAA AAG TTT GAG AAG GTG CCT GGA AAA TAT GAT ATG CTA      575
Glu Asp Arg Glu Lys Phe Glu Lys Val Pro Gly Lys Tyr Asp Met Leu
                180                 185                 190

CAG ATG GAC TAT GCC ACC AAT ACT CAG GAT GAA GAG GAA ACA AAG AAA      623
Gln Met Asp Tyr Ala Thr Asn Thr Gln Asp Glu Glu Glu Thr Lys Lys
            195                 200                 205

GAG GAA TCT CTT AAA TCT CCC TTG AAG CCA GAG TCA CAG CTA GAT CTT      671
Glu Glu Ser Leu Lys Ser Pro Leu Lys Pro Glu Ser Gln Leu Asp Leu
        210                 215                 220

CGG GTA CAG GAG TTA ATA AAG TTG ATC TGT AAT GTT CAG GCC ATG GAA      719
Arg Val Gln Glu Leu Ile Lys Leu Ile Cys Asn Val Gln Ala Met Glu
    225                 230                 235

GAA ATG ATG ATG GAA ATG AAG TAT AAT ACC AAG AAA GCC CCA CTT GGG      767
Glu Met Met Met Glu Met Lys Tyr Asn Thr Lys Lys Ala Pro Leu Gly
240                 245                 250                 255

AAG CTG ACA GTG GCA CAA ATC AAG GCA GGT TAC CAG TCT CTT AAG AAG      815
Lys Leu Thr Val Ala Gln Ile Lys Ala Gly Tyr Gln Ser Leu Lys Lys
```

```
                260                 265                 270

ATT GAG GAT TGT ATT CGG GCT GGC CAG CAT GGA CGA GCT CTC ATG GAA       863
Ile Glu Asp Cys Ile Arg Ala Gly Gln His Gly Arg Ala Leu Met Glu
            275                 280                 285

GCA TGC AAT GAA TTC TAC ACC AGG ATT CCG CAT GAC TTT GGA CTC CGT       911
Ala Cys Asn Glu Phe Tyr Thr Arg Ile Pro His Asp Phe Gly Leu Arg
        290                 295                 300

ACT CCT CCA CTA ATC CGG ACA CAG AAG GAA CTG TCA GAA AAA ATA CAA       959
Thr Pro Pro Leu Ile Arg Thr Gln Lys Glu Leu Ser Glu Lys Ile Gln
    305                 310                 315

TTA CTA GAG GCT TTG GGA GAC ATT GAA ATT GCT ATT AAG CTG GTG AAA      1007
Leu Leu Glu Ala Leu Gly Asp Ile Glu Ile Ala Ile Lys Leu Val Lys
320                 325                 330                 335

ACA GAG CTA CAA AGC CCA GAA CAC CCA TTG GAC CAA CAC TAT AGA AAC      1055
Thr Glu Leu Gln Ser Pro Glu His Pro Leu Asp Gln His Tyr Arg Asn
                340                 345                 350

CTA CAT TGT GCC TTG CGC CCC CTT GAC CAT GAA AGT TAC GAG TTC AAA      1103
Leu His Cys Ala Leu Arg Pro Leu Asp His Glu Ser Tyr Glu Phe Lys
            355                 360                 365

GTG ATT TCC CAG TAC CTA CAA TCT ACC CAT GCT CCC ACA CAC AGC GAC      1151
Val Ile Ser Gln Tyr Leu Gln Ser Thr His Ala Pro Thr His Ser Asp
        370                 375                 380

TAT ACC ATG ACC TTG CTG GAT TTG TTT GAA GTG GAG AAG GAT GGT GAG      1199
Tyr Thr Met Thr Leu Leu Asp Leu Phe Glu Val Glu Lys Asp Gly Glu
    385                 390                 395

AAA GAA GCC TTC AGA GAG GAC CTT CAT AAC AGG ATG CTT CTA TGG CAT      1247
Lys Glu Ala Phe Arg Glu Asp Leu His Asn Arg Met Leu Leu Trp His
400                 405                 410                 415

GGT TCC AGG ATG AGT AAC TGG GTG GGA ATC TTG AGC CAT GGG CTT CGA      1295
Gly Ser Arg Met Ser Asn Trp Val Gly Ile Leu Ser His Gly Leu Arg
                420                 425                 430

ATT GCC CCA CCT GAA GCT CCC ATC ACA GGT TAC ATG TTT GGG AAA GGA      1343
Ile Ala Pro Pro Glu Ala Pro Ile Thr Gly Tyr Met Phe Gly Lys Gly
            435                 440                 445

ATC TAC TTT GCT GAC ATG TCT TCC AAG AGT GCC AAT TAC TGC TTT GCC      1391
Ile Tyr Phe Ala Asp Met Ser Ser Lys Ser Ala Asn Tyr Cys Phe Ala
        450                 455                 460

TCT CGC CTA AAG AAT ACA GGA CTG CTG CTC TTA TCA GAG GTA GCT CTA      1439
Ser Arg Leu Lys Asn Thr Gly Leu Leu Leu Leu Ser Glu Val Ala Leu
    465                 470                 475

GGT CAG TGT AAT GAA CTA CTA GAG GCC AAT CCT AAG GCC GAA GGA TTG      1487
Gly Gln Cys Asn Glu Leu Leu Glu Ala Asn Pro Lys Ala Glu Gly Leu
480                 485                 490                 495

CTT CAA GGT AAA CAT AGC ACC AAG GGG CTG GGC AAG ATG GCT CCC AGT      1535
```

```
Leu Gln Gly Lys His Ser Thr Lys Gly Leu Gly Lys Met Ala Pro Ser
                500                 505                 510

TCT GCC CAC TTC GTC ACC CTG AAT GGG AGT ACA GTG CCA TTA GGA CCA      1583
Ser Ala His Phe Val Thr Leu Asn Gly Ser Thr Val Pro Leu Gly Pro
            515                 520                 525

GCA AGT GAC ACA GGA ATT CTG AAT CCA GAT GGT TAT ACC CTC AAC TAC      1631
Ala Ser Asp Thr Gly Ile Leu Asn Pro Asp Gly Tyr Thr Leu Asn Tyr
        530                 535                 540

AAT GAA TAT ATT GTA TAT AAC CCC AAC CAG GTC CGT ATG CGG TAC CTT      1679
Asn Glu Tyr Ile Val Tyr Asn Pro Asn Gln Val Arg Met Arg Tyr Leu
    545                 550                 555

TTA AAG GTT CAG TTT AAT TTC CTT CAG CTG TGG TGA ATGTTGATAT           1725
Leu Lys Val Gln Phe Asn Phe Leu Gln Leu Trp  *
560                 565                 570

TAAATAAACC AGAGATCTGA TCTTCAAGCA AGAAAATAAG CAGTGTTGTA CTTGTGAATT    1785

TTGTGATATT TTATGTAATA AAAACTGTAC AGGTCTAAAA AAAAAAAAAA AAAAAAAA      1843
```

(2) ANGABEN ZU SEQ ID NO: 2:

(i) SEQUENZKENNZEICHEN:

(A) LÄNGE: 571 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 2:

```
Met Ala Ala Arg Arg Arg Arg Ser Thr Gly Gly Gly Arg Ala Arg Ala
 1               5                  10                  15

Leu Asn Glu Ser Lys Arg Val Asn Asn Gly Asn Thr Ala Pro Glu Asp
            20                  25                  30

Ser Ser Pro Ala Lys Lys Thr Arg Arg Cys Gln Arg Gln Glu Ser Lys
        35                  40                  45

Lys Met Pro Val Ala Gly Gly Lys Ala Asn Lys Asp Arg Thr Glu Asp
    50                  55                  60

Lys Gln Asp Glu Ser Val Lys Ala Leu Leu Leu Lys Gly Lys Ala Pro
65                  70                  75                  80

Val Asp Pro Glu Cys Thr Ala Lys Val Gly Lys Ala His Val Tyr Cys
                85                  90                  95

Glu Gly Asn Asp Val Tyr Asp Val Met Leu Asn Gln Thr Asn Leu Gln
            100                 105                 110

Phe Asn Asn Asn Lys Tyr Tyr Leu Ile Gln Leu Leu Glu Asp Asp Ala
        115                 120                 125

Gln Arg Asn Phe Ser Val Trp Met Arg Trp Gly Arg Val Gly Lys Met
```

130 135 140

Gly Gln His Ser Leu Val Ala Cys Ser Gly Asn Leu Asn Lys Ala Lys
145 150 155 160

Glu Ile Phe Gln Lys Lys Phe Leu Asp Lys Thr Lys Asn Asn Trp Glu
165 170 175

Asp Arg Glu Lys Phe Glu Lys Val Pro Gly Lys Tyr Asp Met Leu Gln
180 185 190

Met Asp Tyr Ala Thr Asn Thr Gln Asp Glu Glu Glu Thr Lys Lys Glu
195 200 205

Glu Ser Leu Lys Ser Pro Leu Lys Pro Glu Ser Gln Leu Asp Leu Arg
210 215 220

Val Gln Glu Leu Ile Lys Leu Ile Cys Asn Val Gln Ala Met Glu Glu
225 230 235 240

Met Met Met Glu Met Lys Tyr Asn Thr Lys Lys Ala Pro Leu Gly Lys
245 250 255

Leu Thr Val Ala Gln Ile Lys Ala Gly Tyr Gln Ser Leu Lys Lys Ile
260 265 270

Glu Asp Cys Ile Arg Ala Gly Gln His Gly Arg Ala Leu Met Glu Ala
275 280 285

Cys Asn Glu Phe Tyr Thr Arg Ile Pro His Asp Phe Gly Leu Arg Thr
290 295 300

Pro Pro Leu Ile Arg Thr Gln Lys Glu Leu Ser Glu Lys Ile Gln Leu
305 310 315 320

Leu Glu Ala Leu Gly Asp Ile Glu Ile Ala Ile Lys Leu Val Lys Thr
325 330 335

Glu Leu Gln Ser Pro Glu His Pro Leu Asp Gln His Tyr Arg Asn Leu
340 345 350

His Cys Ala Leu Arg Pro Leu Asp His Glu Ser Tyr Glu Phe Lys Val
355 360 365

Ile Ser Gln Tyr Leu Gln Ser Thr His Ala Pro Thr His Ser Asp Tyr
370 375 380

Thr Met Thr Leu Leu Asp Leu Phe Glu Val Glu Lys Asp Gly Glu Lys
385 390 395 400

Glu Ala Phe Arg Glu Asp Leu His Asn Arg Met Leu Leu Trp His Gly
405 410 415

Ser Arg Met Ser Asn Trp Val Gly Ile Leu Ser His Gly Leu Arg Ile
420 425 430

Ala Pro Pro Glu Ala Pro Ile Thr Gly Tyr Met Phe Gly Lys Gly Ile
435 440 445

Tyr Phe Ala Asp Met Ser Ser Lys Ser Ala Asn Tyr Cys Phe Ala Ser

```
    450                 455                 460

Arg Leu Lys Asn Thr Gly Leu Leu Leu Leu Ser Glu Val Ala Leu Gly
465                 470                 475                 480

Gln Cys Asn Glu Leu Leu Glu Ala Asn Pro Lys Ala Glu Gly Leu Leu
                485                 490                 495

Gln Gly Lys His Ser Thr Lys Gly Leu Gly Lys Met Ala Pro Ser Ser
            500                 505                 510

Ala His Phe Val Thr Leu Asn Gly Ser Thr Val Pro Leu Gly Pro Ala
        515                 520                 525

Ser Asp Thr Gly Ile Leu Asn Pro Asp Gly Tyr Thr Leu Asn Tyr Asn
    530                 535                 540

Glu Tyr Ile Val Tyr Asn Pro Asn Gln Val Arg Met Arg Tyr Leu Leu
545                 550                 555                 560

Lys Val Gln Phe Asn Phe Leu Gln Leu Trp  *
                565                 570
```

(2) ANGABEN ZU SEQ ID NO: 3:

(i) SEQUENZKENNZEICHEN:

(A) LÄNGE: 2265 Basenpaare
(B) ART: Nucleotid
(C) STRANGFORM: Einzelstrang
(D) TOPOLOGIE: linear

(ii) ART DES MOLEKÜLS: cDNA

(iii) HYPOTHETISCH: NEIN

(iv) ANTISENSE: NEIN

(vi) URSPRÜNLICHE HERKUNFT:

(F) GEWEBETYP: Uterus

(ix) MERKMAL:

(A) NAME/SCHLÜSSEL: CDS
(B) LAGE:242..1843
(D) SONSTIGE ANGABEN:/product= "Poly ADP Ribose Polymerase"

(xi) SEQUENZBESCHREIBUNG: SEQ ID NO: 3:

```
TGGGACTGGT CGCCTGACTC GGCCTGCCCC AGCCTCTGCT TCACCCCACT GGTGGCCAAA      60

TAGCCGATGT CTAATCCCCC ACACAAGCTC ATCCCCGGCC TCTGGGATTG TTGGGAATTC     120

TCTCCCTAAT TCACGCCTGA GGCTCATGGA GAGTTGCTAG ACCTGGGACT GCCCTGGGAG     180

GCGCACACAA CCAGGCCGGG TGGCAGCCAG GACCTCTCCC ATGTCCCTGC TTTTCTTGGC     240

C ATG GCT CCA AAG CCG AAG CCC TGG GTA CAG ACT GAG GGC CCT GAG         286
  Met Ala Pro Lys Pro Lys Pro Trp Val Gln Thr Glu Gly Pro Glu
```

```
                575                 580                 585

AAG AAG AAG GGC CGG CAG GCA GGA AGG GAG GAG GAC CCC TTC CGC TCC          334
Lys Lys Lys Gly Arg Gln Ala Gly Arg Glu Glu Asp Pro Phe Arg Ser
            590                 595                 600

ACC GCT GAG GCC CTC AAG GCC ATA CCC GCA GAG AAG CGC ATA ATC CGC          382
Thr Ala Glu Ala Leu Lys Ala Ile Pro Ala Glu Lys Arg Ile Ile Arg
        605                 610                 615

GTG GAT CCA ACA TGT CCA CTC AGC AGC AAC CCC GGG ACC CAG GTG TAT          430
Val Asp Pro Thr Cys Pro Leu Ser Ser Asn Pro Gly Thr Gln Val Tyr
    620                 625                 630

GAG GAC TAC AAC TGC ACC CTG AAC CAG ACC AAC ATC GAG AAC AAC AAC          478
Glu Asp Tyr Asn Cys Thr Leu Asn Gln Thr Asn Ile Glu Asn Asn Asn
635                 640                 645                 650

AAC AAG TTC TAC ATC ATC CAG CTG CTC CAA GAC AGC AAC CGC TTC TTC          526
Asn Lys Phe Tyr Ile Ile Gln Leu Leu Gln Asp Ser Asn Arg Phe Phe
                655                 660                 665

ACC TGC TGG AAC CGC TGG GGC CGT GTG GGA GAG GTC GGC CAG TCA AAG          574
Thr Cys Trp Asn Arg Trp Gly Arg Val Gly Glu Val Gly Gln Ser Lys
            670                 675                 680

ATC AAC CAC TTC ACA AGG CTA GAA GAT GCA AAG AAG GAC TTT GAG AAG          622
Ile Asn His Phe Thr Arg Leu Glu Asp Ala Lys Lys Asp Phe Glu Lys
        685                 690                 695

AAA TTT CGG GAA AAG ACC AAG AAC AAC TGG GCA GAG CGG GAC CAC TTT          670
Lys Phe Arg Glu Lys Thr Lys Asn Asn Trp Ala Glu Arg Asp His Phe
    700                 705                 710

GTG TCT CAC CCG GGC AAG TAC ACA CTT ATC GAA GTA CAG GCA GAG GAT          718
Val Ser His Pro Gly Lys Tyr Thr Leu Ile Glu Val Gln Ala Glu Asp
715                 720                 725                 730

GAG GCC CAG GAA GCT GTG GTG AAG GTG GAC AGA GGC CCA GTG AGG ACT          766
Glu Ala Gln Glu Ala Val Val Lys Val Asp Arg Gly Pro Val Arg Thr
                735                 740                 745

GTG ACT AAG CGG GTG CAG CCC TGC TCC CTG GAC CCA GCC ACG CAG AAG          814
Val Thr Lys Arg Val Gln Pro Cys Ser Leu Asp Pro Ala Thr Gln Lys
            750                 755                 760

CTC ATC ACT AAC ATC TTC AGC AAG GAG ATG TTC AAG AAC ACC ATG GCC          862
Leu Ile Thr Asn Ile Phe Ser Lys Glu Met Phe Lys Asn Thr Met Ala
        765                 770                 775

CTC ATG GAC CTG GAT GTG AAG AAG ATG CCC CTG GGA AAG CTG AGC AAG          910
Leu Met Asp Leu Asp Val Lys Lys Met Pro Leu Gly Lys Leu Ser Lys
    780                 785                 790

CAA CAG ATT GCA CGG GGT TTC GAG GCC TTG GAG GCG CTG GAG GAG GCC          958
Gln Gln Ile Ala Arg Gly Phe Glu Ala Leu Glu Ala Leu Glu Glu Ala
795                 800                 805                 810

CTG AAA GGC CCC ACG GAT GGT GGC CAA AGC CTG GAG GAG CTG TCC TCA         1006
```

```
Leu Lys Gly Pro Thr Asp Gly Gly Gln Ser Leu Glu Glu Leu Ser Ser
                815                 820                 825

CAC TTT TAC ACC GTC ATC CCG CAC AAC TTC GGC CAC AGC CAG CCC CCG      1054
His Phe Tyr Thr Val Ile Pro His Asn Phe Gly His Ser Gln Pro Pro
            830                 835                 840

CCC ATC AAT TCC CCT GAG CTT CTG CAG GCC AAG AAG GAC ATG CTG CTG      1102
Pro Ile Asn Ser Pro Glu Leu Leu Gln Ala Lys Lys Asp Met Leu Leu
        845                 850                 855

GTG CTG GCG GAC ATC GAG CTG GCC CAG GCC CTG CAG GCA GTC TCT GAG      1150
Val Leu Ala Asp Ile Glu Leu Ala Gln Ala Leu Gln Ala Val Ser Glu
    860                 865                 870

CAG GAG AAG ACG GTG GAG GAG GTG CCA CAC CCC CTG GAC CGA GAC TAC      1198
Gln Glu Lys Thr Val Glu Glu Val Pro His Pro Leu Asp Arg Asp Tyr
875                 880                 885                 890

CAG CTT CTC AAG TGC CAG CTG CAG CTG CTA GAC TCT GGA GCA CCT GAG      1246
Gln Leu Leu Lys Cys Gln Leu Gln Leu Leu Asp Ser Gly Ala Pro Glu
                895                 900                 905

TAC AAG GTG ATA CAG ACC TAC TTA GAA CAG ACT GGC AGC AAC CAC AGG      1294
Tyr Lys Val Ile Gln Thr Tyr Leu Glu Gln Thr Gly Ser Asn His Arg
            910                 915                 920

TGC CCT ACA CTT CAA CAC ATC TGG AAA GTA AAC CAA GAA GGG GAG GAA      1342
Cys Pro Thr Leu Gln His Ile Trp Lys Val Asn Gln Glu Gly Glu Glu
        925                 930                 935

GAC AGA TTC CAG GCC CAC TCC AAA CTG GGT AAT CGG AAG CTG CTG TGG      1390
Asp Arg Phe Gln Ala His Ser Lys Leu Gly Asn Arg Lys Leu Leu Trp
    940                 945                 950

CAT GGC ACC AAC ATG GCC GTG GTG GCC GCC ATC CTC ACT AGT GGG CTC      1438
His Gly Thr Asn Met Ala Val Val Ala Ala Ile Leu Thr Ser Gly Leu
955                 960                 965                 970

CGC ATC ATG CCA CAT TCT GGT GGG CGT GTT GGC AAG GGC ATC TAC TTT      1486
Arg Ile Met Pro His Ser Gly Gly Arg Val Gly Lys Gly Ile Tyr Phe
                975                 980                 985

GCC TCA GAG AAC AGC AAG TCA GCT GGA TAT GTT ATT GGC ATG AAG TGT      1534
Ala Ser Glu Asn Ser Lys Ser Ala Gly Tyr Val Ile Gly Met Lys Cys
            990                 995                 1000

GGG GCC CAC CAT GTC GGC TAC ATG TTC CTG GGT GAG GTG GCC CTG GGC      1582
Gly Ala His His Val Gly Tyr Met Phe Leu Gly Glu Val Ala Leu Gly
        1005                1010                1015

AGA GAG CAC CAT ATC AAC ACG GAC AAC CCC AGC TTG AAG AGC CCA CCT      1630
Arg Glu His His Ile Asn Thr Asp Asn Pro Ser Leu Lys Ser Pro Pro
    1020                1025                1030

CCT GGC TTC GAC AGT GTC ATT GCC CGA GGC CAC ACC GAG CCT GAT CCG      1678
Pro Gly Phe Asp Ser Val Ile Ala Arg Gly His Thr Glu Pro Asp Pro
1035                1040                1045                1050
```

```
ACC CAG GAC ACT GAG TTG GAG CTG GAT GGC CAG CAA GTG GTG GTG CCC      1726
Thr Gln Asp Thr Glu Leu Glu Leu Asp Gly Gln Gln Val Val Val Pro
                1055                1060                1065

CAG GGC CAG CCT GTG CCC TGC CCA GAG TTC AGC AGC TCC ACA TTC TCC      1774
Gln Gly Gln Pro Val Pro Cys Pro Glu Phe Ser Ser Ser Thr Phe Ser
            1070                1075                1080

CAG AGC GAG TAC CTC ATC TAC CAG GAG AGC CAG TGT CGC CTG CGC TAC      1822
Gln Ser Glu Tyr Leu Ile Tyr Gln Glu Ser Gln Cys Arg Leu Arg Tyr
        1085                1090                1095

CTG CTG GAG GTC CAC CTC TGA GTGCCCGCCC TGTCCCCCGG GGTCCTGCAA         1873
Leu Leu Glu Val His Leu  *
    1100                1105

GGCTGGACTG TGATCTTCAA TCATCCTGCC CATCTCTGGT ACCCCTATAT CACTCCTTTT    1933

TTTCAAGAAT ACAATACGTT GTTGTTAACT ATAGTCACCA TGCTGTACAA GATCCCTGAA    1993

CTTATGCCTC CTAACTGAAA TTTTGTATTC TTTGACACAT CTGCCCAGTC CCTCTCCTCC    2053

CAGCCCATGG TAACCAGCAT TTGACTCTTT ACTTGTATAA GGGCAGCTTT TATAGGTTCC    2113

ACATGTAAGT GAGATCATGC AGTGTTTGTC TTTCTGTGCC TGGCTTATTT CACTCAGCAT    2173

AATGTGCACC GGGTTCACCC ATGTTTTCAT AAATGACAAG ATTTCCTCCT TTAAAAAAAA    2233

AAAAAAAAAA AAAAAAAAAA AAAAAAAAAA AA                                  2265
```

(2) ANGABEN ZU SEQ ID NO: 4:

(i) SEQUENZKENNZEICHEN:

(A) LÄNGE: 534 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 4:

```
Met Ala Pro Lys Pro Lys Pro Trp Val Gln Thr Glu Gly Pro Glu Lys
 1               5                  10                  15

Lys Lys Gly Arg Gln Ala Gly Arg Glu Glu Asp Pro Phe Arg Ser Thr
            20                  25                  30

Ala Glu Ala Leu Lys Ala Ile Pro Ala Glu Lys Arg Ile Ile Arg Val
        35                  40                  45

Asp Pro Thr Cys Pro Leu Ser Ser Asn Pro Gly Thr Gln Val Tyr Glu
    50                  55                  60

Asp Tyr Asn Cys Thr Leu Asn Gln Thr Asn Ile Glu Asn Asn Asn Asn
65                  70                  75                  80

Lys Phe Tyr Ile Ile Gln Leu Leu Gln Asp Ser Asn Arg Phe Phe Thr
                85                  90                  95

Cys Trp Asn Arg Trp Gly Arg Val Gly Glu Val Gly Gln Ser Lys Ile
```

```
                100                 105                 110

Asn His Phe Thr Arg Leu Glu Asp Ala Lys Lys Asp Phe Glu Lys Lys
        115                 120                 125

Phe Arg Glu Lys Thr Lys Asn Asn Trp Ala Glu Arg Asp His Phe Val
    130                 135                 140

Ser His Pro Gly Lys Tyr Thr Leu Ile Glu Val Gln Ala Glu Asp Glu
145                 150                 155                 160

Ala Gln Glu Ala Val Val Lys Val Asp Arg Gly Pro Val Arg Thr Val
                165                 170                 175

Thr Lys Arg Val Gln Pro Cys Ser Leu Asp Pro Ala Thr Gln Lys Leu
            180                 185                 190

Ile Thr Asn Ile Phe Ser Lys Glu Met Phe Lys Asn Thr Met Ala Leu
        195                 200                 205

Met Asp Leu Asp Val Lys Lys Met Pro Leu Gly Lys Leu Ser Lys Gln
    210                 215                 220

Gln Ile Ala Arg Gly Phe Glu Ala Leu Glu Ala Leu Glu Glu Ala Leu
225                 230                 235                 240

Lys Gly Pro Thr Asp Gly Gly Gln Ser Leu Glu Glu Leu Ser Ser His
                245                 250                 255

Phe Tyr Thr Val Ile Pro His Asn Phe Gly His Ser Gln Pro Pro Pro
            260                 265                 270

Ile Asn Ser Pro Glu Leu Leu Gln Ala Lys Lys Asp Met Leu Leu Val
        275                 280                 285

Leu Ala Asp Ile Glu Leu Ala Gln Ala Leu Gln Ala Val Ser Glu Gln
    290                 295                 300

Glu Lys Thr Val Glu Glu Val Pro His Pro Leu Asp Arg Asp Tyr Gln
305                 310                 315                 320

Leu Leu Lys Cys Gln Leu Gln Leu Leu Asp Ser Gly Ala Pro Glu Tyr
                325                 330                 335

Lys Val Ile Gln Thr Tyr Leu Glu Gln Thr Gly Ser Asn His Arg Cys
            340                 345                 350

Pro Thr Leu Gln His Ile Trp Lys Val Asn Gln Glu Gly Glu Glu Asp
        355                 360                 365

Arg Phe Gln Ala His Ser Lys Leu Gly Asn Arg Lys Leu Leu Trp His
    370                 375                 380

Gly Thr Asn Met Ala Val Val Ala Ala Ile Leu Thr Ser Gly Leu Arg
385                 390                 395                 400

Ile Met Pro His Ser Gly Gly Arg Val Gly Lys Gly Ile Tyr Phe Ala
                405                 410                 415

Ser Glu Asn Ser Lys Ser Ala Gly Tyr Val Ile Gly Met Lys Cys Gly
```

```
            420                     425                     430

Ala His His Val Gly Tyr Met Phe Leu Gly Glu Val Ala Leu Gly Arg
        435                     440                     445

Glu His His Ile Asn Thr Asp Asn Pro Ser Leu Lys Ser Pro Pro Pro
    450                     455                     460

Gly Phe Asp Ser Val Ile Ala Arg Gly His Thr Glu Pro Asp Pro Thr
465                     470                     475                     480

Gln Asp Thr Glu Leu Glu Leu Asp Gly Gln Gln Val Val Val Pro Gln
                485                     490                     495

Gly Gln Pro Val Pro Cys Pro Glu Phe Ser Ser Ser Thr Phe Ser Gln
            500                     505                     510

Ser Glu Tyr Leu Ile Tyr Gln Glu Ser Gln Cys Arg Leu Arg Tyr Leu
        515                     520                     525

Leu Glu Val His Leu  *
    530
```

(2) ANGABEN ZU SEQ ID NO: 5:

(i) SEQUENZKENNZEICHEN:

(A) LÄNGE: 2265 Basenpaare
(B) ART: Nucleotid
(C) STRANGFORM: Einzelstrang
(D) TOPOLOGIE: linear

(ii) ART DES MOLEKÜLS: cDNA

(iii) HYPOTHETISCH: NEIN

(iv) ANTISENSE: NEIN

(vi) URSPRÜNLICHE HERKUNFT:

(F) GEWEBETYP: Uterus

(ix) MERKMAL:

(A) NAME/SCHLÜSSEL: CDS
(B) LAGE:221..1843
(D) SONSTIGE ANGABEN:/product= "Poly ADP Ribose Polymerase"

(xi) SEQUENZBESCHREIBUNG: SEQ ID NO: 5:

```
TGGGACTGGT CGCCTGACTC GGCCTGCCCC AGCCTCTGCT TCACCCCACT GGTGGCCAAA      60

TAGCCGATGT CTAATCCCCC ACACAAGCTC ATCCCCGGCC TCTGGGATTG TTGGGAATTC     120

TCTCCCTAAT TCACGCCTGA GGCTCATGGA GAGTTGCTAG ACCTGGGACT GCCCTGGGAG     180

GCGCACACAA CCAGGCCGGG TGGCAGCCAG GACCTCTCCC ATG TCC CTG CTT TTC       235
                                            Met Ser Leu Leu Phe
                                            535

TTG GCC ATG GCT CCA AAG CCG AAG CCC TGG GTA CAG ACT GAG GGC CCT       283
```

```
Leu Ala Met Ala Pro Lys Pro Lys Pro Trp Val Gln Thr Glu Gly Pro
540                 545                 550                 555

GAG AAG AAG AAG GGC CGG CAG GCA GGA AGG GAG GAG GAC CCC TTC CGC        331
Glu Lys Lys Lys Gly Arg Gln Ala Gly Arg Glu Glu Asp Pro Phe Arg
                560                 565                 570

TCC ACC GCT GAG GCC CTC AAG GCC ATA CCC GCA GAG AAG CGC ATA ATC        379
Ser Thr Ala Glu Ala Leu Lys Ala Ile Pro Ala Glu Lys Arg Ile Ile
            575                 580                 585

CGC GTG GAT CCA ACA TGT CCA CTC AGC AGC AAC CCC GGG ACC CAG GTG        427
Arg Val Asp Pro Thr Cys Pro Leu Ser Ser Asn Pro Gly Thr Gln Val
        .590                595                 600

TAT GAG GAC TAC AAC TGC ACC CTG AAC CAG ACC AAC ATC GAG AAC AAC        475
Tyr Glu Asp Tyr Asn Cys Thr Leu Asn Gln Thr Asn Ile Glu Asn Asn
    605                 610                 615

AAC AAC AAG TTC TAC ATC ATC CAG CTG CTC CAA GAC AGC AAC CGC TTC        523
Asn Asn Lys Phe Tyr Ile Ile Gln Leu Leu Gln Asp Ser Asn Arg Phe
620                 625                 630                 635

TTC ACC TGC TGG AAC CGC TGG GGC CGT GTG GGA GAG GTC GGC CAG TCA        571
Phe Thr Cys Trp Asn Arg Trp Gly Arg Val Gly Glu Val Gly Gln Ser
                640                 645                 650

AAG ATC AAC CAC TTC ACA AGG CTA GAA GAT GCA AAG AAG GAC TTT GAG        619
Lys Ile Asn His Phe Thr Arg Leu Glu Asp Ala Lys Lys Asp Phe Glu
            655                 660                 665

AAG AAA TTT CGG GAA AAG ACC AAG AAC AAC TGG GCA GAG CGG GAC CAC        667
Lys Lys Phe Arg Glu Lys Thr Lys Asn Asn Trp Ala Glu Arg Asp His
        670                 675                 680

TTT GTG TCT CAC CCG GGC AAG TAC ACA CTT ATC GAA GTA CAG GCA GAG        715
Phe Val Ser His Pro Gly Lys Tyr Thr Leu Ile Glu Val Gln Ala Glu
    685                 690                 695

GAT GAG GCC CAG GAA GCT GTG GTG AAG GTG GAC AGA GGC CCA GTG AGG        763
Asp Glu Ala Gln Glu Ala Val Val Lys Val Asp Arg Gly Pro Val Arg
700                 705                 710                 715

ACT GTG ACT AAG CGG GTG CAG CCC TGC TCC CTG GAC CCA GCC ACG CAG        811
Thr Val Thr Lys Arg Val Gln Pro Cys Ser Leu Asp Pro Ala Thr Gln
                720                 725                 730

AAG CTC ATC ACT AAC ATC TTC AGC AAG GAG ATG TTC AAG AAC ACC ATG        859
Lys Leu Ile Thr Asn Ile Phe Ser Lys Glu Met Phe Lys Asn Thr Met
            735                 740                 745

GCC CTC ATG GAC CTG GAT GTG AAG AAG ATG CCC CTG GGA AAG CTG AGC        907
Ala Leu Met Asp Leu Asp Val Lys Lys Met Pro Leu Gly Lys Leu Ser
        750                 755                 760

AAG CAA CAG ATT GCA CGG GGT TTC GAG GCC TTG GAG GCG CTG GAG GAG        955
Lys Gln Gln Ile Ala Arg Gly Phe Glu Ala Leu Glu Ala Leu Glu Glu
    765                 770                 775
```

```
GCC CTG AAA GGC CCC ACG GAT GGT GGC CAA AGC CTG GAG GAG CTG TCC      1003
Ala Leu Lys Gly Pro Thr Asp Gly Gly Gln Ser Leu Glu Glu Leu Ser
780                 785                 790                 795

TCA CAC TTT TAC ACC GTC ATC CCG CAC AAC TTC GGC CAC AGC CAG CCC      1051
Ser His Phe Tyr Thr Val Ile Pro His Asn Phe Gly His Ser Gln Pro
                800                 805                 810

CCG CCC ATC AAT TCC CCT GAG CTT CTG CAG GCC AAG AAG GAC ATG CTG      1099
Pro Pro Ile Asn Ser Pro Glu Leu Leu Gln Ala Lys Lys Asp Met Leu
            815                 820                 825

CTG GTG CTG GCG GAC ATC GAG CTG GCC CAG GCC CTG CAG GCA GTC TCT      1147
Leu Val Leu Ala Asp Ile Glu Leu Ala Gln Ala Leu Gln Ala Val Ser
        830                 835                 840

GAG CAG GAG AAG ACG GTG GAG GAG GTG CCA CAC CCC CTG GAC CGA GAC      1195
Glu Gln Glu Lys Thr Val Glu Glu Val Pro His Pro Leu Asp Arg Asp
    845                 850                 855

TAC CAG CTT CTC AAG TGC CAG CTG CAG CTG CTA GAC TCT GGA GCA CCT      1243
Tyr Gln Leu Leu Lys Cys Gln Leu Gln Leu Leu Asp Ser Gly Ala Pro
860                 865                 870                 875

GAG TAC AAG GTG ATA CAG ACC TAC TTA GAA CAG ACT GGC AGC AAC CAC      1291
Glu Tyr Lys Val Ile Gln Thr Tyr Leu Glu Gln Thr Gly Ser Asn His
                880                 885                 890

AGG TGC CCT ACA CTT CAA CAC ATC TGG AAA GTA AAC CAA GAA GGG GAG      1339
Arg Cys Pro Thr Leu Gln His Ile Trp Lys Val Asn Gln Glu Gly Glu
            895                 900                 905

GAA GAC AGA TTC CAG GCC CAC TCC AAA CTG GGT AAT CGG AAG CTG CTG      1387
Glu Asp Arg Phe Gln Ala His Ser Lys Leu Gly Asn Arg Lys Leu Leu
        910                 915                 920

TGG CAT GGC ACC AAC ATG GCC GTG GTG GCC GCC ATC CTC ACT AGT GGG      1435
Trp His Gly Thr Asn Met Ala Val Val Ala Ala Ile Leu Thr Ser Gly
    925                 930                 935

CTC CGC ATC ATG CCA CAT TCT GGT GGG CGT GTT GGC AAG GGC ATC TAC      1483
Leu Arg Ile Met Pro His Ser Gly Gly Arg Val Gly Lys Gly Ile Tyr
940                 945                 950                 955

TTT GCC TCA GAG AAC AGC AAG TCA GCT GGA TAT GTT ATT GGC ATG AAG      1531
Phe Ala Ser Glu Asn Ser Lys Ser Ala Gly Tyr Val Ile Gly Met Lys
                960                 965                 970

TGT GGG GCC CAC CAT GTC GGC TAC ATG TTC CTG GGT GAG GTG GCC CTG      1579
Cys Gly Ala His His Val Gly Tyr Met Phe Leu Gly Glu Val Ala Leu
            975                 980                 985

GGC AGA GAG CAC CAT ATC AAC ACG GAC AAC CCC AGC TTG AAG AGC CCA      1627
Gly Arg Glu His His Ile Asn Thr Asp Asn Pro Ser Leu Lys Ser Pro
        990                 995                 1000

CCT CCT GGC TTC GAC AGT GTC ATT GCC CGA GGC CAC ACC GAG CCT GAT      1675
Pro Pro Gly Phe Asp Ser Val Ile Ala Arg Gly His Thr Glu Pro Asp
```

```
    1005                1010                1015

CCG ACC CAG GAC ACT GAG TTG GAG CTG GAT GGC CAG CAA GTG GTG GTG      1723
Pro Thr Gln Asp Thr Glu Leu Glu Leu Asp Gly Gln Gln Val Val Val
1020                1025                1030                1035

CCC CAG GGC CAG CCT GTG CCC TGC CCA GAG TTC AGC AGC TCC ACA TTC      1771
Pro Gln Gly Gln Pro Val Pro Cys Pro Glu Phe Ser Ser Ser Thr Phe
                1040                1045                1050

TCC CAG AGC GAG TAC CTC ATC TAC CAG GAG AGC CAG TGT CGC CTG CGC      1819
Ser Gln Ser Glu Tyr Leu Ile Tyr Gln Glu Ser Gln Cys Arg Leu Arg
            1055                1060                1065

TAC CTG CTG GAG GTC CAC CTC TGA GTGCCCGCCC TGTCCCCCGG GGTCCTGCAA      1873
Tyr Leu Leu Glu Val His Leu  *
        1070                1075

GGCTGGACTG TGATCTTCAA TCATCCTGCC CATCTCTGGT ACCCCTATAT CACTCCTTTT      1933

TTTCAAGAAT ACAATACGTT GTTGTTAACT ATAGTCACCA TGCTGTACAA GATCCCTGAA      1993

CTTATGCCTC CTAACTGAAA TTTTGTATTC TTTGACACAT CTGCCCAGTC CCTCTCCTCC      2053

CAGCCCATGG TAACCAGCAT TTGACTCTTT ACTTGTATAA GGGCAGCTTT TATAGGTTCC      2113

ACATGTAAGT GAGATCATGC AGTGTTTGTC TTTCTGTGCC TGGCTTATTT CACTCAGCAT      2173

AATGTGCACC GGGTTCACCC ATGTTTTCAT AAATGACAAG ATTTCCTCCT TTAAAAAAAA      2233

AAAAAAAAAA AAAAAAAAAA AAAAAAAAAA AA                                  2265
```

(2) ANGABEN ZU SEQ ID NO: 6:

(i) SEQUENZKENNZEICHEN:

(A) LÄNGE: 541 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 6:

Met Ser Leu Leu Phe Leu Ala Met Ala Pro Lys Pro Lys Pro Trp Val
1 5 10 15

Gln Thr Glu Gly Pro Glu Lys Lys Lys Gly Arg Gln Ala Gly Arg Glu
20 25 30

Glu Asp Pro Phe Arg Ser Thr Ala Glu Ala Leu Lys Ala Ile Pro Ala
35 40 45

Glu Lys Arg Ile Ile Arg Val Asp Pro Thr Cys Pro Leu Ser Ser Asn
50 55 60

Pro Gly Thr Gln Val Tyr Glu Asp Tyr Asn Cys Thr Leu Asn Gln Thr
65 70 75 80

Asn Ile Glu Asn Asn Asn Asn Lys Phe Tyr Ile Ile Gln Leu Leu Gln

```
                        85                  90                  95
Asp Ser Asn Arg Phe Phe Thr Cys Trp Asn Arg Trp Gly Arg Val Gly
            100                 105                 110
Glu Val Gly Gln Ser Lys Ile Asn His Phe Thr Arg Leu Glu Asp Ala
        115                 120                 125
Lys Lys Asp Phe Glu Lys Lys Phe Arg Glu Lys Thr Lys Asn Asn Trp
    130                 135                 140
Ala Glu Arg Asp His Phe Val Ser His Pro Gly Lys Tyr Thr Leu Ile
145                 150                 155                 160
Glu Val Gln Ala Glu Asp Glu Ala Gln Glu Ala Val Val Lys Val Asp
                165                 170                 175
Arg Gly Pro Val Arg Thr Val Thr Lys Arg Val Gln Pro Cys Ser Leu
            180                 185                 190
Asp Pro Ala Thr Gln Lys Leu Ile Thr Asn Ile Phe Ser Lys Glu Met
        195                 200                 205
Phe Lys Asn Thr Met Ala Leu Met Asp Leu Asp Val Lys Lys Met Pro
    210                 215                 220
Leu Gly Lys Leu Ser Lys Gln Gln Ile Ala Arg Gly Phe Glu Ala Leu
225                 230                 235                 240
Glu Ala Leu Glu Glu Ala Leu Lys Gly Pro Thr Asp Gly Gly Gln Ser
                245                 250                 255
Leu Glu Glu Leu Ser Ser His Phe Tyr Thr Val Ile Pro His Asn Phe
            260                 265                 270
Gly His Ser Gln Pro Pro Pro Ile Asn Ser Pro Glu Leu Leu Gln Ala
        275                 280                 285
Lys Lys Asp Met Leu Leu Val Leu Ala Asp Ile Glu Leu Ala Gln Ala
    290                 295                 300
Leu Gln Ala Val Ser Glu Gln Glu Lys Thr Val Glu Glu Val Pro His
305                 310                 315                 320
Pro Leu Asp Arg Asp Tyr Gln Leu Leu Lys Cys Gln Leu Gln Leu Leu
                325                 330                 335
Asp Ser Gly Ala Pro Glu Tyr Lys Val Ile Gln Thr Tyr Leu Glu Gln
            340                 345                 350
Thr Gly Ser Asn His Arg Cys Pro Thr Leu Gln His Ile Trp Lys Val
        355                 360                 365
Asn Gln Glu Gly Glu Glu Asp Arg Phe Gln Ala His Ser Lys Leu Gly
    370                 375                 380
Asn Arg Lys Leu Leu Trp His Gly Thr Asn Met Ala Val Val Ala Ala
385                 390                 395                 400
Ile Leu Thr Ser Gly Leu Arg Ile Met Pro His Ser Gly Gly Arg Val
```

```
                405                 410                 415
Gly Lys Gly Ile Tyr Phe Ala Ser Glu Asn Ser Lys Ser Ala Gly Tyr
            420                 425                 430

Val Ile Gly Met Lys Cys Gly Ala His His Val Gly Tyr Met Phe Leu
        435                 440                 445

Gly Glu Val Ala Leu Gly Arg Glu His His Ile Asn Thr Asp Asn Pro
    450                 455                 460

Ser Leu Lys Ser Pro Pro Pro Gly Phe Asp Ser Val Ile Ala Arg Gly
465                 470                 475                 480

His Thr Glu Pro Asp Pro Thr Gln Asp Thr Glu Leu Glu Leu Asp Gly
                485                 490                 495

Gln Gln Val Val Val Pro Gln Gly Gln Pro Val Pro Cys Pro Glu Phe
            500                 505                 510

Ser Ser Ser Thr Phe Ser Gln Ser Glu Tyr Leu Ile Tyr Gln Glu Ser
        515                 520                 525

Gln Cys Arg Leu Arg Tyr Leu Leu Glu Val His Leu  *
    530                 535                 540
```

(2) ANGABEN ZU SEQ ID NO: 7:

(i) SEQUENZKENNZEICHEN:

(A) LÄNGE: 1740 Basenpaare
(B) ART: Nucleotid
(C) STRANGFORM: Einzelstrang
(D) TOPOLOGIE: linear

(ii) ART DES MOLEKÜLS: cDNA

(iii) HYPOTHETISCH: NEIN

(iv) ANTISENSE: NEIN

(vi) URSPRÜNLICHE HERKUNFT:

(A) ORGANISMUS: Mus musculus

(ix) MERKMAL:

(A) NAME/SCHLÜSSEL: CDS
(B) LAGE:112..1710

(xi) SEQUENZBESCHREIBUNG: SEQ ID NO: 7:

```
CCCGGCTTTC ACTTTTTCTG CTGCCTCGGG GAACACCTCG AGCCAACTGC TTCCTAACTC       60

AGGGTGGGCA GAACTGACGG GATCTAAGCT TCTGCATCTC TGAGGAGAAC C ATG GCT       117
                                                         Met Ala

CCA AAA CGA AAG GCC TCT GTG CAG ACT GAG GGC TCC AAG AAG CAG CGA       165
Pro Lys Arg Lys Ala Ser Val Gln Thr Glu Gly Ser Lys Lys Gln Arg
    545                 550                 555
```

CAA GGG ACA GAG GAG GAG GAC AGC TTC CGG TCC ACT GCC GAG GCT CTC 213
Gln Gly Thr Glu Glu Glu Asp Ser Phe Arg Ser Thr Ala Glu Ala Leu
560 565 570 575

AGA GCA GCA CCT GCT GAT AAT CGG GTC ATC CGT GTG GAC CCC TCA TGT 261
Arg Ala Ala Pro Ala Asp Asn Arg Val Ile Arg Val Asp Pro Ser Cys
580 585 590

CCA TTC AGC CGG AAC CCC GGG ATA CAG GTC CAC GAG GAC TAT GAC TGT 309
Pro Phe Ser Arg Asn Pro Gly Ile Gln Val His Glu Asp Tyr Asp Cys
595 600 605

ACC CTG AAC CAG ACC AAC ATC GGC AAC AAC AAC AAC AAG TTC TAT ATT 357
Thr Leu Asn Gln Thr Asn Ile Gly Asn Asn Asn Asn Lys Phe Tyr Ile
610 615 620

ATC CAA CTG CTG GAG GAG GGT AGT CGC TTC TTC TGC TGG AAT CGC TGG 405
Ile Gln Leu Leu Glu Glu Gly Ser Arg Phe Phe Cys Trp Asn Arg Trp
625 630 635

GGC CGC GTG GGA GAG GTG GGC CAG AGC AAG ATG AAC CAC TTC ACC TGC 453
Gly Arg Val Gly Glu Val Gly Gln Ser Lys Met Asn His Phe Thr Cys
640 645 650 655

CTG GAA GAT GCA AAG AAG GAC TTT AAG AAG AAA TTT TGG GAG AAG ACT 501
Leu Glu Asp Ala Lys Lys Asp Phe Lys Lys Lys Phe Trp Glu Lys Thr
660 665 670

AAA AAC AAA TGG GAG GAG CGG GAC CGT TTT GTG GCC CAG CCC AAC AAG 549
Lys Asn Lys Trp Glu Glu Arg Asp Arg Phe Val Ala Gln Pro Asn Lys
675 680 685

TAC ACA CTT ATA GAA GTC CAG GGA GAA GCA GAG AGC CAA GAG GCT GTA 597
Tyr Thr Leu Ile Glu Val Gln Gly Glu Ala Glu Ser Gln Glu Ala Val
690 695 700

GTG AAG GCC TTA TCT CCC CAG GTG GAC AGC GGC CCT GTG AGG ACC GTG 645
Val Lys Ala Leu Ser Pro Gln Val Asp Ser Gly Pro Val Arg Thr Val
705 710 715

GTC AAG CCC TGC TCC CTA GAC CCT GCC ACC CAG AAC CTT ATC ACC AAC 693
Val Lys Pro Cys Ser Leu Asp Pro Ala Thr Gln Asn Leu Ile Thr Asn
720 725 730 735

ATC TTC AGC AAA GAG ATG TTC AAG AAC GCA ATG ACC CTC ATG AAC CTG 741
Ile Phe Ser Lys Glu Met Phe Lys Asn Ala Met Thr Leu Met Asn Leu
740 745 750

GAT GTG AAG AAG ATG CCC TTG GGA AAG CTG ACC AAG CAG CAG ATT GCC 789
Asp Val Lys Lys Met Pro Leu Gly Lys Leu Thr Lys Gln Gln Ile Ala
755 760 765

CGT GGC TTC GAG GCC TTG GAA GCT CTA GAG GAG GCC ATG AAA AAC CCC 837
Arg Gly Phe Glu Ala Leu Glu Ala Leu Glu Glu Ala Met Lys Asn Pro
770 775 780

ACA GGG GAT GGC CAG AGC CTG GAA GAG CTC TCC TCC TGC TTC TAC ACT 885
Thr Gly Asp Gly Gln Ser Leu Glu Glu Leu Ser Ser Cys Phe Tyr Thr

```
    785                 790                 795

GTC ATC CCA CAC AAC TTC GGC CGC AGC CGA CCC CCG CCC ATC AAC TCC          933
Val Ile Pro His Asn Phe Gly Arg Ser Arg Pro Pro Pro Ile Asn Ser
800                 805                 810                 815

CCT GAT GTG CTT CAG GCC AAG AAG GAC ATG CTG CTG GTG CTA GCG GAC          981
Pro Asp Val Leu Gln Ala Lys Lys Asp Met Leu Leu Val Leu Ala Asp
                820                 825                 830

ATC GAG TTG GCG CAG ACC TTG CAG GCA GCC CCT GGG GAG GAG GAG GAG         1029
Ile Glu Leu Ala Gln Thr Leu Gln Ala Ala Pro Gly Glu Glu Glu Glu
            835                 840                 845

AAA GTG GAA GAG GTG CCA CAC CCA CTG GAT CGA GAC TAC CAG CTC CTC         1077
Lys Val Glu Glu Val Pro His Pro Leu Asp Arg Asp Tyr Gln Leu Leu
        850                 855                 860

AGG TGC CAG CTT CAA CTG CTG GAC TCC GGG GAG TCC GAG TAC AAG GCA         1125
Arg Cys Gln Leu Gln Leu Leu Asp Ser Gly Glu Ser Glu Tyr Lys Ala
    865                 870                 875

ATA CAG ACC TAC CTG AAA CAG ACT GGC AAC AGC TAC AGG TGC CCA AAC         1173
Ile Gln Thr Tyr Leu Lys Gln Thr Gly Asn Ser Tyr Arg Cys Pro Asn
880                 885                 890                 895

CTG CGG CAT GTT TGG AAA GTG AAC CGA GAA GGG GAG GGA GAC AGG TTC         1221
Leu Arg His Val Trp Lys Val Asn Arg Glu Gly Glu Gly Asp Arg Phe
                900                 905                 910

CAG GCC CAC TCC AAA CTG GGC AAT CGG AGG CTG CTG TGG CAC GGC ACC         1269
Gln Ala His Ser Lys Leu Gly Asn Arg Arg Leu Leu Trp His Gly Thr
            915                 920                 925

AAT GTG GCC GTG GTG GCT GCC ATC CTC ACC AGT GGG CTC CGA ATC ATG         1317
Asn Val Ala Val Val Ala Ala Ile Leu Thr Ser Gly Leu Arg Ile Met
        930                 935                 940

CCA CAC TCG GGT GGT CGT GTT GGC AAG GGT ATT TAT TTT GCC TCT GAG         1365
Pro His Ser Gly Gly Arg Val Gly Lys Gly Ile Tyr Phe Ala Ser Glu
    945                 950                 955

AAC AGC AAG TCA GCT GGC TAT GTT ACC ACC ATG CAC TGT GGG GGC CAC         1413
Asn Ser Lys Ser Ala Gly Tyr Val Thr Thr Met His Cys Gly Gly His
960                 965                 970                 975

CAG GTG GGC TAC ATG TTC CTG GGC GAG GTG GCC CTC GGC AAA GAG CAC         1461
Gln Val Gly Tyr Met Phe Leu Gly Glu Val Ala Leu Gly Lys Glu His
                980                 985                 990

CAC ATC ACC ATC GAT GAC CCC AGC TTG AAG AGT CCA CCC CCT GGC TTT         1509
His Ile Thr Ile Asp Asp Pro Ser Leu Lys Ser Pro Pro Pro Gly Phe
            995                1000                1005

GAC AGC GTC ATC GCC CGA GGC CAA ACC GAG CCG GAT CCC GCC CAG GAC         1557
Asp Ser Val Ile Ala Arg Gly Gln Thr Glu Pro Asp Pro Ala Gln Asp
       1010                1015                1020

ATT GAA CTT GAA CTG GAT GGG CAG CCG GTG GTG GTG CCC CAA GGC CCG         1605
```

```
Ile Glu Leu Glu Leu Asp Gly Gln Pro Val Val Val Pro Gln Gly Pro
    1025                1030                1035

CCT GTG CAG TGC CCG TCA TTC AAA AGC TCC AGC TTC AGC CAG AGT GAA      1653
Pro Val Gln Cys Pro Ser Phe Lys Ser Ser Ser Phe Ser Gln Ser Glu
1040                1045                1050                1055

TAC CTC ATA TAC AAG GAG AGC CAG TGT CGC CTG CGC TAC CTG CTG GAG      1701
Tyr Leu Ile Tyr Lys Glu Ser Gln Cys Arg Leu Arg Tyr Leu Leu Glu
                1060                1065                1070

ATT CAC CTC TAAGCTGCTT GCCCTCCCTA GGTCCAAGCC                         1740
Ile His Leu
```

(2) ANGABEN ZU SEQ ID NO: 8:

(i) SEQUENZKENNZEICHEN:

(A) LÄNGE: 533 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 8:

```
Met Ala Pro Lys Arg Lys Ala Ser Val Gln Thr Glu Gly Ser Lys Lys
 1               5                  10                  15

Gln Arg Gln Gly Thr Glu Glu Glu Asp Ser Phe Arg Ser Thr Ala Glu
            20                  25                  30

Ala Leu Arg Ala Ala Pro Ala Asp Asn Arg Val Ile Arg Val Asp Pro
        35                  40                  45

Ser Cys Pro Phe Ser Arg Asn Pro Gly Ile Gln Val His Glu Asp Tyr
    50                  55                  60

Asp Cys Thr Leu Asn Gln Thr Asn Ile Gly Asn Asn Asn Asn Lys Phe
65                  70                  75                  80

Tyr Ile Ile Gln Leu Leu Glu Glu Gly Ser Arg Phe Phe Cys Trp Asn
                85                  90                  95

Arg Trp Gly Arg Val Gly Glu Val Gly Gln Ser Lys Met Asn His Phe
            100                 105                 110

Thr Cys Leu Glu Asp Ala Lys Lys Asp Phe Lys Lys Lys Phe Trp Glu
        115                 120                 125

Lys Thr Lys Asn Lys Trp Glu Glu Arg Asp Arg Phe Val Ala Gln Pro
    130                 135                 140

Asn Lys Tyr Thr Leu Ile Glu Val Gln Gly Glu Ala Glu Ser Gln Glu
145                 150                 155                 160

Ala Val Val Lys Ala Leu Ser Pro Gln Val Asp Ser Gly Pro Val Arg
                165                 170                 175

Thr Val Val Lys Pro Cys Ser Leu Asp Pro Ala Thr Gln Asn Leu Ile
```

180 185 190

Thr Asn Ile Phe Ser Lys Glu Met Phe Lys Asn Ala Met Thr Leu Met
195 200 205

Asn Leu Asp Val Lys Lys Met Pro Leu Gly Lys Leu Thr Lys Gln Gln
210 215 220

Ile Ala Arg Gly Phe Glu Ala Leu Glu Ala Leu Glu Glu Ala Met Lys
225 230 235 240

Asn Pro Thr Gly Asp Gly Gln Ser Leu Glu Glu Leu Ser Ser Cys Phe
245 250 255

Tyr Thr Val Ile Pro His Asn Phe Gly Arg Ser Arg Pro Pro Pro Ile
260 265 270

Asn Ser Pro Asp Val Leu Gln Ala Lys Lys Asp Met Leu Leu Val Leu
275 280 285

Ala Asp Ile Glu Leu Ala Gln Thr Leu Gln Ala Ala Pro Gly Glu Glu
290 295 300

Glu Glu Lys Val Glu Glu Val Pro His Pro Leu Asp Arg Asp Tyr Gln
305 310 315 320

Leu Leu Arg Cys Gln Leu Gln Leu Leu Asp Ser Gly Glu Ser Glu Tyr
325 330 335

Lys Ala Ile Gln Thr Tyr Leu Lys Gln Thr Gly Asn Ser Tyr Arg Cys
340 345 350

Pro Asn Leu Arg His Val Trp Lys Val Asn Arg Glu Gly Glu Gly Asp
355 360 365

Arg Phe Gln Ala His Ser Lys Leu Gly Asn Arg Arg Leu Leu Trp His
370 375 380

Gly Thr Asn Val Ala Val Val Ala Ala Ile Leu Thr Ser Gly Leu Arg
385 390 395 400

Ile Met Pro His Ser Gly Gly Arg Val Gly Lys Gly Ile Tyr Phe Ala
405 410 415

Ser Glu Asn Ser Lys Ser Ala Gly Tyr Val Thr Thr Met His Cys Gly
420 425 430

Gly His Gln Val Gly Tyr Met Phe Leu Gly Glu Val Ala Leu Gly Lys
435 440 445

Glu His His Ile Thr Ile Asp Asp Pro Ser Leu Lys Ser Pro Pro Pro
450 455 460

Gly Phe Asp Ser Val Ile Ala Arg Gly Gln Thr Glu Pro Asp Pro Ala
465 470 475 480

Gln Asp Ile Glu Leu Glu Leu Asp Gly Gln Pro Val Val Val Pro Gln
485 490 495

Gly Pro Pro Val Gln Cys Pro Ser Phe Lys Ser Ser Ser Phe Ser Gln

```
            500                 505                 510
Ser Glu Tyr Leu Ile Tyr Lys Glu Ser Gln Cys Arg Leu Arg Tyr Leu
        515                 520                 525
Leu Glu Ile His Leu
    530
```

(2) ANGABEN ZU SEQ ID NO: 9:

(i) SEQUENZ KENNZEICHEN:

(A) LÄNGE: 1587 Basenpaare
(B) ART: Nucleotid
(C) STRANGFORM: Einzelstrang
(D) TOPOLOGIE: linear

(ii) ART DES MOLEKÜLS: cDNA

(iii) HYPOTHETISCH: NEIN

(iv) ANTISENSE: NEIN

(vi) URSPRÜNLICHE HERKUNFT:

(A) ORGANISMUS: Mus musculus

(ix) MERKMAL:

(A) NAME/SCHLÜSSEL: CDS
(B) LAGE:1..1584

(xi) SEQUENZBESCHREIBUNG: SEQ ID NO: 9:

```
ATG GCT CCA AAA CGA AAG GCC TCT GTG CAG ACT GAG GGC TCC AAG AAG          48
Met Ala Pro Lys Arg Lys Ala Ser Val Gln Thr Glu Gly Ser Lys Lys
    535                 540                 545

CAG CGA CAA GGG ACA GAG GAG GAG GAC AGC TTC CGG TCC ACT GCC GAG          96
Gln Arg Gln Gly Thr Glu Glu Glu Asp Ser Phe Arg Ser Thr Ala Glu
550                 555                 560                 565

GCT CTC AGA GCA GCA CCT GCT GAT AAT CGG GTC ATC CGT GTG GAC CCC         144
Ala Leu Arg Ala Ala Pro Ala Asp Asn Arg Val Ile Arg Val Asp Pro
                570                 575                 580

TCA TGT CCA TTC AGC CGG AAC CCC GGG ATA CAG GTC CAC GAG GAC TAT         192
Ser Cys Pro Phe Ser Arg Asn Pro Gly Ile Gln Val His Glu Asp Tyr
            585                 590                 595

GAC TGT ACC CTG AAC CAG ACC AAC ATC GGC AAC AAC AAC AAC AAG TTC         240
Asp Cys Thr Leu Asn Gln Thr Asn Ile Gly Asn Asn Asn Asn Lys Phe
        600                 605                 610

TAT ATT ATC CAA CTG CTG GAG GAG GGT AGT CGC TTC TTC TGC TGG AAT         288
Tyr Ile Ile Gln Leu Leu Glu Glu Gly Ser Arg Phe Phe Cys Trp Asn
    615                 620                 625

CGC TGG GGC CGC GTG GGA GAG GTG GGC CAG AGC AAG ATG AAC CAC TTC         336
Arg Trp Gly Arg Val Gly Glu Val Gly Gln Ser Lys Met Asn His Phe
630                 635                 640                 645
```

```
ACC TGC CTG GAA GAT GCA AAG AAG GAC TTT AAG AAG AAA TTT TGG GAG        384
Thr Cys Leu Glu Asp Ala Lys Lys Asp Phe Lys Lys Lys Phe Trp Glu
                650                 655                 660

AAG ACT AAA AAC AAA TGG GAG GAG CGG GAC CGT TTT GTG GCC CAG CCC        432
Lys Thr Lys Asn Lys Trp Glu Glu Arg Asp Arg Phe Val Ala Gln Pro
            665                 670                 675

AAC AAG TAC ACA CTT ATA GAA GTC CAG GGA GAA GCA GAG AGC CAA GAG        480
Asn Lys Tyr Thr Leu Ile Glu Val Gln Gly Glu Ala Glu Ser Gln Glu
        680                 685                 690

GCT GTA GTG AAG GTG GAC AGC GGC CCT GTG AGG ACC GTG GTC AAG CCC        528
Ala Val Val Lys Val Asp Ser Gly Pro Val Arg Thr Val Val Lys Pro
    695                 700                 705

TGC TCC CTA GAC CCT GCC ACC CAG AAC CTT ATC ACC AAC ATC TTC AGC        576
Cys Ser Leu Asp Pro Ala Thr Gln Asn Leu Ile Thr Asn Ile Phe Ser
710                 715                 720                 725

AAA GAG ATG TTC AAG AAC GCA ATG ACC CTC ATG AAC CTG GAT GTG AAG        624
Lys Glu Met Phe Lys Asn Ala Met Thr Leu Met Asn Leu Asp Val Lys
                730                 735                 740

AAG ATG CCC TTG GGA AAG CTG ACC AAG CAG CAG ATT GCC CGT GGC TTC        672
Lys Met Pro Leu Gly Lys Leu Thr Lys Gln Gln Ile Ala Arg Gly Phe
            745                 750                 755

GAG GCC TTG GAA GCT CTA GAG GAG GCC ATG AAA AAC CCC ACA GGG GAT        720
Glu Ala Leu Glu Ala Leu Glu Glu Ala Met Lys Asn Pro Thr Gly Asp
        760                 765                 770

GGC CAG AGC CTG GAA GAG CTC TCC TCC TGC TTC TAC ACT GTC ATC CCA        768
Gly Gln Ser Leu Glu Glu Leu Ser Ser Cys Phe Tyr Thr Val Ile Pro
    775                 780                 785

CAC AAC TTC GGC CGC AGC CGA CCC CCG CCC ATC AAC TCC CCT GAT GTG        816
His Asn Phe Gly Arg Ser Arg Pro Pro Pro Ile Asn Ser Pro Asp Val
790                 795                 800                 805

CTT CAG GCC AAG AAG GAC ATG CTG CTG GTG CTA GCG GAC ATC GAG TTG        864
Leu Gln Ala Lys Lys Asp Met Leu Leu Val Leu Ala Asp Ile Glu Leu
                810                 815                 820

GCG CAG ACC TTG CAG GCA GCC CCT GGG GAG GAG GAG GAG AAA GTG GAA        912
Ala Gln Thr Leu Gln Ala Ala Pro Gly Glu Glu Glu Glu Lys Val Glu
            825                 830                 835

GAG GTG CCA CAC CCA CTG GAT CGA GAC TAC CAG CTC CTC AGG TGC CAG        960
Glu Val Pro His Pro Leu Asp Arg Asp Tyr Gln Leu Leu Arg Cys Gln
        840                 845                 850

CTT CAA CTG CTG GAC TCC GGG GAG TCC GAG TAC AAG GCA ATA CAG ACC       1008
Leu Gln Leu Leu Asp Ser Gly Glu Ser Glu Tyr Lys Ala Ile Gln Thr
    855                 860                 865

TAC CTG AAA CAG ACT GGC AAC AGC TAC AGG TGC CCA AAC CTG CGG CAT       1056
Tyr Leu Lys Gln Thr Gly Asn Ser Tyr Arg Cys Pro Asn Leu Arg His
```

```
870                 875                 880                 885

GTT TGG AAA GTG AAC CGA GAA GGG GAG GGA GAC AGG TTC CAG GCC CAC     1104
Val Trp Lys Val Asn Arg Glu Gly Glu Gly Asp Arg Phe Gln Ala His
                890                 895                 900

TCC AAA CTG GGC AAT CGG AGG CTG CTG TGG CAC GGC ACC AAT GTG GCC     1152
Ser Lys Leu Gly Asn Arg Arg Leu Leu Trp His Gly Thr Asn Val Ala
            905                 910                 915

GTG GTG GCT GCC ATC CTC ACC AGT GGG CTC CGA ATC ATG CCA CAC TCG     1200
Val Val Ala Ala Ile Leu Thr Ser Gly Leu Arg Ile Met Pro His Ser
        920                 925                 930

GGT GGT CGT GTT GGC AAG GGT ATT TAT TTT GCC TCT GAG AAC AGC AAG     1248
Gly Gly Arg Val Gly Lys Gly Ile Tyr Phe Ala Ser Glu Asn Ser Lys
    935                 940                 945

TCA GCT GGC TAT GTT ACC ACC ATG CAC TGT GGG GGC CAC CAG GTG GGC     1296
Ser Ala Gly Tyr Val Thr Thr Met His Cys Gly Gly His Gln Val Gly
950                 955                 960                 965

TAC ATG TTC CTG GGC GAG GTG GCC CTC GGC AAA GAG CAC CAC ATC ACC     1344
Tyr Met Phe Leu Gly Glu Val Ala Leu Gly Lys Glu His His Ile Thr
                970                 975                 980

ATC GAT GAC CCC AGC TTG AAG AGT CCA CCC CCT GGC TTT GAC AGC GTC     1392
Ile Asp Asp Pro Ser Leu Lys Ser Pro Pro Pro Gly Phe Asp Ser Val
            985                 990                 995

ATC GCC CGA GGC CAA ACC GAG CCG GAT CCC GCC CAG GAC ATT GAA CTT     1440
Ile Ala Arg Gly Gln Thr Glu Pro Asp Pro Ala Gln Asp Ile Glu Leu
        1000                1005                1010

GAA CTG GAT GGG CAG CCG GTG GTG GTG CCC CAA GGC CCG CCT GTG CAG     1488
Glu Leu Asp Gly Gln Pro Val Val Val Pro Gln Gly Pro Pro Val Gln
    1015                1020                1025

TGC CCG TCA TTC AAA AGC TCC AGC TTC AGC CAG AGT GAA TAC CTC ATA     1536
Cys Pro Ser Phe Lys Ser Ser Ser Phe Ser Gln Ser Glu Tyr Leu Ile
1030                1035                1040                1045

TAC AAG GAG AGC CAG TGT CGC CTG CGC TAC CTG CTG GAG ATT CAC CTC     1584
Tyr Lys Glu Ser Gln Cys Arg Leu Arg Tyr Leu Leu Glu Ile His Leu
                1050                1055                1060

TAA                                                                 1587
```

(2) ANGABEN ZU SEQ ID NO: 10:

(i) SEQUENZKENNZEICHEN:

(A) LÄNGE: 528 Aminosäuren
(B) ART: Aminosäure
(D) TOPOLOGIE: linear

(ii) ART DES MOLEKÜLS: Protein
(xi) SEQUENZBESCHREIBUNG: SEQ ID NO: 10:

Met Ala Pro Lys Arg Lys Ala Ser Val Gln Thr Glu Gly Ser Lys Lys

```
1               5                   10                  15
Gln Arg Gln Gly Thr Glu Glu Glu Asp Ser Phe Arg Ser Thr Ala Glu
            20                  25                  30
Ala Leu Arg Ala Ala Pro Ala Asp Asn Arg Val Ile Arg Val Asp Pro
        35                  40                  45
Ser Cys Pro Phe Ser Arg Asn Pro Gly Ile Gln Val His Glu Asp Tyr
    50                  55                  60
Asp Cys Thr Leu Asn Gln Thr Asn Ile Gly Asn Asn Asn Asn Lys Phe
65                  70                  75                  80
Tyr Ile Ile Gln Leu Leu Glu Glu Gly Ser Arg Phe Phe Cys Trp Asn
                85                  90                  95
Arg Trp Gly Arg Val Gly Glu Val Gly Gln Ser Lys Met Asn His Phe
            100                 105                 110
Thr Cys Leu Glu Asp Ala Lys Lys Asp Phe Lys Lys Lys Phe Trp Glu
        115                 120                 125
Lys Thr Lys Asn Lys Trp Glu Glu Arg Asp Arg Phe Val Ala Gln Pro
    130                 135                 140
Asn Lys Tyr Thr Leu Ile Glu Val Gln Gly Glu Ala Glu Ser Gln Glu
145                 150                 155                 160
Ala Val Val Lys Val Asp Ser Gly Pro Val Arg Thr Val Val Lys Pro
                165                 170                 175
Cys Ser Leu Asp Pro Ala Thr Gln Asn Leu Ile Thr Asn Ile Phe Ser
            180                 185                 190
Lys Glu Met Phe Lys Asn Ala Met Thr Leu Met Asn Leu Asp Val Lys
        195                 200                 205
Lys Met Pro Leu Gly Lys Leu Thr Lys Gln Gln Ile Ala Arg Gly Phe
    210                 215                 220
Glu Ala Leu Glu Ala Leu Glu Glu Ala Met Lys Asn Pro Thr Gly Asp
225                 230                 235                 240
Gly Gln Ser Leu Glu Glu Leu Ser Ser Cys Phe Tyr Thr Val Ile Pro
                245                 250                 255
His Asn Phe Gly Arg Ser Arg Pro Pro Pro Ile Asn Ser Pro Asp Val
            260                 265                 270
Leu Gln Ala Lys Lys Asp Met Leu Leu Val Leu Ala Asp Ile Glu Leu
        275                 280                 285
Ala Gln Thr Leu Gln Ala Ala Pro Gly Glu Glu Glu Glu Lys Val Glu
    290                 295                 300
Glu Val Pro His Pro Leu Asp Arg Asp Tyr Gln Leu Leu Arg Cys Gln
305                 310                 315                 320
Leu Gln Leu Leu Asp Ser Gly Glu Ser Glu Tyr Lys Ala Ile Gln Thr
```

```
                    325                 330                 335
Tyr Leu Lys Gln Thr Gly Asn Ser Tyr Arg Cys Pro Asn Leu Arg His
            340                 345                 350
Val Trp Lys Val Asn Arg Glu Gly Glu Gly Asp Arg Phe Gln Ala His
        355                 360                 365
Ser Lys Leu Gly Asn Arg Arg Leu Leu Trp His Gly Thr Asn Val Ala
    370                 375                 380
Val Val Ala Ala Ile Leu Thr Ser Gly Leu Arg Ile Met Pro His Ser
385                 390                 395                 400
Gly Gly Arg Val Gly Lys Gly Ile Tyr Phe Ala Ser Glu Asn Ser Lys
                405                 410                 415
Ser Ala Gly Tyr Val Thr Thr Met His Cys Gly Gly His Gln Val Gly
            420                 425                 430
Tyr Met Phe Leu Gly Glu Val Ala Leu Gly Lys Glu His His Ile Thr
        435                 440                 445
Ile Asp Asp Pro Ser Leu Lys Ser Pro Pro Pro Gly Phe Asp Ser Val
    450                 455                 460
Ile Ala Arg Gly Gln Thr Glu Pro Asp Pro Ala Gln Asp Ile Glu Leu
465                 470                 475                 480
Glu Leu Asp Gly Gln Pro Val Val Val Pro Gln Gly Pro Pro Val Gln
                485                 490                 495
Cys Pro Ser Phe Lys Ser Ser Ser Phe Ser Gln Ser Glu Tyr Leu Ile
            500                 505                 510
Tyr Lys Glu Ser Gln Cys Arg Leu Arg Tyr Leu Leu Glu Ile His Leu
        515                 520                 525
```

(2) ANGABEN ZU SEQ ID NO: 11:

(i) SEQUENZKENNZEICHEN:

(A) LÄNGE: 14 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: JA

(ix) MERKMAL:

(A) NAME/SCHLÜSSEL: Region
(B) LAGE:2
(D) SONSTIGE ANGABEN:/note= "Xaa steht fuer 1 bis 5 andere Aminosaeuren"

(ix) MERKMAL:

(A) NAME/SCHLÜSSEL: Region
(B) LAGE:3
(D) SONSTIGE ANGABEN:/note= "Xaa steht fuer Ser oder Thr"

(xi) SEQUENZBESCHREIBUNG: SEQ ID NO: 11:

```
Pro Xaa Xaa Gly Xaa Xaa Xaa Gly Lys Gly Ile Tyr Phe Ala
1               5                   10
```

(2) ANGABEN ZU SEQ ID NO: 12:

(i) SEQUENZKENNZEICHEN:

(A) LÄNGE: 21 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: JA

(ix) MERKMAL:

(A) NAME/SCHLÜSSEL: Region
(B) LAGE:1
(D) SONSTIGE ANGABEN:/note= "Xaa steht fuer Ser oder Thr"

(ix) MERKMAL:

(A) NAME/SCHLÜSSEL: Region
(B) LAGE:6
(D) SONSTIGE ANGABEN:/note= "Xaa steht fuer Ile oder Val"

(ix) MERKMAL:

(A) NAME/SCHLÜSSEL: Region
(B) LAGE:9
(D) SONSTIGE ANGABEN:/note= "Xaa steht fuer 1 bis 5 andere Aminosaeuren"

(ix) MERKMAL:

(A) NAME/SCHLÜSSEL: Region
(B) LAGE:10
(D) SONSTIGE ANGABEN:/note= "Xaa steht fuer Ser oder Thr"

(xi) SEQUENZBESCHREIBUNG: SEQ ID NO: 12:

```
Xaa Xaa Gly Leu Arg Xaa Xaa Pro Xaa Xaa Gly Xaa Xaa Xaa Gly Lys
1               5                   10                  15

Gly Ile Tyr Phe Ala
            20
```

(2) ANGABEN ZU SEQ ID NO: 13:

(i) SEQUENZKENNZEICHEN:

(A) LÄNGE: 45 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: JA

(ix) MERKMAL:

(A) NAME/SCHLÜSSEL: Region
(B) LAGE:16
(D) SONSTIGE ANGABEN:/note= "Xaa steht fuer Ser oder Thr"

(ix) MERKMAL:

(A) NAME/SCHLÜSSEL: Region
(B) LAGE:21
(D) SONSTIGE ANGABEN:/note= "Xaa steht fuer Ile oder Val"

(ix) MERKMAL:

(A) NAME/SCHLÜSSEL: Region
(B) LAGE:24
(D) SONSTIGE ANGABEN:/note= "Xaa steht fuer 1 bis 5 andere Aminosaeuren"

(ix) MERKMAL:

(A) NAME/SCHLÜSSEL: Region
(B) LAGE:25
(D) SONSTIGE ANGABEN:/note= "Xaa steht fuer Ser oder Thr"

(ix) MERKMAL:

(A) NAME/SCHLÜSSEL: Region
(B) LAGE:6
(D) SONSTIGE ANGABEN:/note= "Xaa steht fuer Ser oder Thr"

(xi) SEQUENZBESCHREIBUNG: SEQ ID NO: 13:

```
Leu Leu Trp His Gly Xaa Xaa Xaa Xaa Xaa Xaa Xaa Xaa Ile Leu Xaa
1               5                   10                  15

Xaa Gly Leu Arg Xaa Xaa Pro Xaa Xaa Gly Xaa Xaa Xaa Gly Lys Gly
            20                  25                  30

Ile Tyr Phe Ala Xaa Xaa Xaa Ser Lys Ser Ala Xaa Tyr
        35                  40                  45
```

(2) ANGABEN ZU SEQ ID NO: 14:

(i) SEQUENZKENNZEICHEN:

(A) LÄNGE: 22 Aminosäuren

(B) ART: Aminosäure
(C) STRANGFORM: Einzelstrang
(D) TOPOLOGIE: linear

(ii) ART DES MOLEKÜLS: Peptid

(iii) HYPOTHETISCH: JA

(ix) MERKMAL:

(A) NAME/SCHLÜSSEL: Region
(B) LAGE:1
(D) SONSTIGE ANGABEN:/note= "Xaa steht fuer Leu oder Val"

(xi) SEQUENZBESCHREIBUNG: SEQ ID NO: 14:

```
Xaa Xaa Xaa Xaa Xaa Xaa Xaa Leu Xaa Xaa Xaa Xaa Xaa Xaa Leu Xaa
1               5                   10                  15

Xaa Xaa Xaa Xaa Xaa Leu
            20
```

(2) ANGABEN ZU SEQ ID NO: 15:

(i) SEQUENZKENNZEICHEN:

(A) LÄNGE: 27 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: JA

(ix) MERKMAL:

(A) NAME/SCHLÜSSEL: Region
(B) LAGE:21
(D) SONSTIGE ANGABEN:/note= "Xaa steht fuer Asp oder Glu"

(ix) MERKMAL:

(A) NAME/SCHLÜSSEL: Region
(B) LAGE:22
(D) SONSTIGE ANGABEN:/note= "Xaa steht fuer 10 oder 11 andere Aminosaeuren"

(xi) SEQUENZBESCHREIBUNG: SEQ ID NO: 15:

```
Leu Xaa Xaa Xaa Xaa Xaa Xaa Xaa Xaa Xaa Asn Xaa Xaa Tyr Xaa Xaa
1               5                   10                  15

Gln Leu Leu Xaa Xaa Xaa Trp Gly Arg Val Gly
            20                  25
```

(2) ANGABEN ZU SEQ ID NO: 16:

(i) SEQUENZKENNZEICHEN:

(A) LÄNGE: 29 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: JA

(xi) SEQUENZBESCHREIBUNG: SEQ ID NO: 16:

```
Ala Xaa Xaa Xaa Phe Xaa Lys Xaa Xaa Xaa Xaa Lys Thr Xaa Asn Xaa
1               5                   10                  15

Trp Xaa Xaa Xaa Xaa Xaa Phe Xaa Xaa Xaa Pro Xaa Lys
            20                  25
```

(2) ANGABEN ZU SEQ ID NO: 17:

(i) SEQUENZKENNZEICHEN:

(A) LÄNGE: 44 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: JA

(ix) MERKMAL:

(A) NAME/SCHLÜSSEL: Region
(B) LAGE:4
(D) SONSTIGE ANGABEN:/note= "Xaa steht fuer Ile oder Leu"

(xi) SEQUENZBESCHREIBUNG: SEQ ID NO: 17:

```
Gln Xaa Leu Xaa Xaa Xaa Ile Xaa Xaa Xaa Xaa Xaa Xaa Xaa Xaa Xaa
1               5                   10                  15

Met Xaa Xaa Xaa Xaa Xaa Xaa Xaa Xaa Xaa Xaa Pro Leu Gly Lys Leu
            20                  25                  30

Xaa Xaa Xaa Gln Ile Xaa Xaa Xaa Xaa Xaa Xaa Leu
        35                  40
```

(2) ANGABEN ZU SEQ ID NO: 18:

(i) SEQUENZKENNZEICHEN:

(A) LÄNGE: 15 Aminosäuren

(B) ART: Aminosäure
(C) STRANGFORM: Einzelstrang
(D) TOPOLOGIE: linear

(ii) ART DES MOLEKÜLS: Peptid

(iii) HYPOTHETISCH: JA

(xi) SEQUENZBESCHREIBUNG: SEQ ID NO: 18:

```
Phe Tyr Thr Xaa Ile Pro His Xaa Phe Gly Xaa Xaa Xaa Pro Pro
1               5                   10                  15
```

(2) ANGABEN ZU SEQ ID NO: 19:

(i) SEQUENZKENNZEICHEN:

(A) LÄNGE: 17 Aminosäuren
(B) ART: Aminosäure
(C) STRANGFORM: Einzelstrang
(D) TOPOLOGIE: linear

(ii) ART DES MOLEKÜLS: Peptid
(iii) HYPOTHETISCH: JA
(xi) SEQUENZBESCHREIBUNG: SEQ ID NO: 19:

```
Lys Xaa Xaa Xaa Leu Xaa Xaa Leu Xaa Asp Ile Glu Xaa Ala Xaa Xaa
1               5                   10                  15

Leu
```

(2) ANGABEN ZU SEQ ID NO: 20:

(i) SEQUENZKENNZEICHEN:

(A) LÄNGE: 11 Aminosäuren
(B) ART: Aminosäure
(C) STRANGFORM: Einzelstrang
(D) TOPOLOGIE: linear

(ii) ART DES MOLEKÜLS: Peptid
(iii) HYPOTHETISCH: JA
(xi) SEQUENZBESCHREIBUNG: SEQ ID NO: 20:

```
Gly Xaa Xaa Xaa Leu Xaa Glu Val Ala Leu Gly
1               5                   10
```

(2) ANGABEN ZU SEQ ID NO: 21:

(i) SEQUENZKENNZEICHEN:

(A) LÄNGE: 20 Aminosäuren
(B) ART: Aminosäure
(C) STRANGFORM: Einzelstrang

(D) TOPOLOGIE: linear

(ii) ART DES MOLEKÜLS: Peptid
(iii) HYPOTHETISCH: JA
(ix) MERKMAL:

(A) NAME/SCHLÜSSEL: Region
(B) LAGE:14
(D) SONSTIGE ANGABEN:/note= "Xaa steht fuer 7 bis 9 andere Aminosaeuren"

(xi) SEQUENZBESCHREIBUNG: SEQ ID NO: 21:

```
Gly Xaa Xaa Ser Xaa Xaa Xaa Xaa Gly Xaa Xaa Xaa Pro Xaa Leu Xaa
1               5                   10                  15

Gly Xaa Xaa Val
            20
```

(2) ANGABEN ZU SEQ ID NO: 22:

(i) SEQUENZKENNZEICHEN:

(A) LÄNGE: 16 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: JA

(ix) MERKMAL:

(A) NAME/SCHLÜSSEL: Region
(B) LAGE:2
(D) SONSTIGE ANGABEN:/note= "Xaa steht fuer Tyr oder Phe"

(xi) SEQUENZBESCHREIBUNG: SEQ ID NO: 22:

```
Glu Xaa Xaa Xaa Tyr Xaa Xaa Xaa Gln Xaa Xaa Xaa Xaa Tyr Leu Leu
1               5                   10                  15
```

(2) ANGABEN ZU SEQ ID NO: 23:

(i) SEQUENZKENNZEICHEN:

(A) LÄNGE: 20 Aminosäuren
(B) ART: Aminosäure
(C) STRANGFORM: Einzelstrang
(D) TOPOLOGIE: linear

(ii) ART DES MOLEKÜLS: Peptid

(iii) HYPOTHETISCH: NEIN

(xi) SEQUENZBESCHREIBUNG: SEQ ID NO: 23:

```
Met Ala Ala Arg Arg Arg Arg Ser Thr Gly Gly Gly Arg Ala Arg Ala
1               5                   10                  15

Leu Asn Glu Ser
```

(2) ANGABEN ZU SEQ ID NO: 24:

(i) SEQUENZKENNZEICHEN:

(A) LÄNGE: 20 Aminosäuren
(B) ART: Aminosäure
(C) STRANGFORM: Einzelstrang
(D) TOPOLOGIE: linear

(ii) ART DES MOLEKÜLS: Peptid
(iii) HYPOTHETISCH: NEIN
(xi) SEQUENZBESCHREIBUNG: SEQ ID NO: 24:

```
Lys Thr Glu Leu Gln Ser Pro Glu His Pro Leu Asp Gln His Tyr Arg
1               5                   10                  15

Asn Leu His Cys
            20
```

(2) ANGABEN ZU SEQ ID NO: 25:

(i) SEQUENZKENNZEICHEN:

(A) LÄNGE: 21 Aminosäuren
(B) ART: Aminosäure
(C) STRANGFORM: Einzelstrang
(D) TOPOLOGIE: linear

(ii) ART DES MOLEKÜLS: Peptid
(iii) HYPOTHETISCH: NEIN
(xi) SEQUENZBESCHREIBUNG: SEQ ID NO: 25:

```
Cys Lys Gly Arg Gln Ala Gly Arg Glu Glu Asp Pro Phe Arg Ser Thr
1               5                   10                  15

Ala Glu Ala Leu Lys
            20
```

(2) ANGABEN ZU SEQ ID NO: 26:

(i) SEQUENZKENNZEICHEN:

(A) LÄNGE: 20 Aminosäuren
(B) ART: Aminosäure
(C) STRANGFORM: Einzelstrang
(D) TOPOLOGIE: linear

(ii) ART DES MOLEKÜLS: Peptid
(iii) HYPOTHETISCH: NEIN

(xi) SEQUENZBESCHREIBUNG: SEQ ID NO: 26:

```
Cys Lys Gln Gln Ile Ala Arg Gly Phe Glu Ala Leu Glu Ala Leu Glu
1               5                   10                  15

Glu Ala Leu Lys
            20
```

(2) ANGABEN ZU SEQ ID NO: 27:

(i) SEQUENZKENNZEICHEN:

(A) LÄNGE: 19 Aminosäuren
(B) ART: Aminosäure
(C) STRANGFORM: Einzelstrang
(D) TOPOLOGIE: linear

(ii) ART DES MOLEKÜLS: Peptid
(iii) HYPOTHETISCH: NEIN
(xi) SEQUENZBESCHREIBUNG: SEQ ID NO: 27:

```
Lys Gln Gln Ile Ala Arg Gly Phe Glu Ala Leu Glu Ala Leu Glu Glu
1               5                   10                  15

Ala Leu Lys
```

(2) ANGABEN ZU SEQ ID NO: 28:

(i) SEQUENZKENNZEICHEN:

(A) LÄNGE: 19 Aminosäuren
(B) ART: Aminosäure
(C) STRANGFORM: Einzelstrang
(D) TOPOLOGIE: linear

(ii) ART DES MOLEKÜLS: Peptid
(iii) HYPOTHETISCH: NEIN
(xi) SEQUENZBESCHREIBUNG: SEQ ID NO: 28:

```
Lys Gln Gln Ile Ala Arg Gly Phe Glu Ala Leu Glu Ala Leu Glu Glu
1               5                   10                  15

Ala Met Lys
```

SEQUENZPROTOKOLL

<110> BASF Aktiengesellschaft

<120> Neue Poly(ADP-ribose)polymerase-Gene

<130> M/39113-PCT

<140> PCT/EP 99/03889
<141> 1999-06-04

<160> 28

<170> Patent In Ver. 2.1

<210> 1
<211> 1843
<212> DNA
<213> Brain

<220>
<221> CDS
<222> (3)..(1715)
<223> Product= Poly ADP Ribose Polymerase

<400> 1

```
cc atg gcg gcg cgg cgg cga cgg agc acc ggc ggc ggc agg gcg aga     47
   Met Ala Ala Arg Arg Arg Arg Ser Thr Gly Gly Gly Arg Ala Arg
     1               5                  10                  15

gca tta aat gaa agc aaa aga gtt aat aat ggc aac acg gct cca gaa    95
Ala Leu Asn Glu Ser Lys Arg Val Asn Asn Gly Asn Thr Ala Pro Glu
                 20                  25                  30

gac tct tcc cct gcc aag aaa act cgt aga tgc cag aga cag gag tcg   143
Asp Ser Ser Pro Ala Lys Lys Thr Arg Arg Cys Gln Arg Gln Glu Ser
             35                  40                  45

aaa aag atg cct gtg gct gga gga aaa gct aat aag gac agg aca gaa   191
Lys Lys Met Pro Val Ala Gly Gly Lys Ala Asn Lys Asp Arg Thr Glu
         50                  55                  60

gac aag caa gat gaa tct gtg aag gcc ttg ctg tta aag ggc aaa gct   239
Asp Lys Gln Asp Glu Ser Val Lys Ala Leu Leu Leu Lys Gly Lys Ala
     65                  70                  75

cct gtg gac cca gag tgt aca gcc aag gtg ggg aag gct cat gtg tat   287
Pro Val Asp Pro Glu Cys Thr Ala Lys Val Gly Lys Ala His Val Tyr
 80                  85                  90                  95

tgt gaa gga aat gat gtc tat gat gtc atg cta aat cag acc aat ctc   335
Cys Glu Gly Asn Asp Val Tyr Asp Val Met Leu Asn Gln Thr Asn Leu
                100                 105                 110

cag ttc aac aac aac aag tac tat ctg att cag cta tta gaa gat gat   383
Gln Phe Asn Asn Asn Lys Tyr Tyr Leu Ile Gln Leu Leu Glu Asp Asp
            115                 120                 125

gcc cag agg aac ttc agt gtt tgg atg aga tgg ggc cga gtt ggg aaa   431
Ala Gln Arg Asn Phe Ser Val Trp Met Arg Trp Gly Arg Val Gly Lys
        130                 135                 140
```

```
atg gga cag cac agc ctg gtg gct tgt tca ggc aat ctc aac aag gcc    479
Met Gly Gln His Ser Leu Val Ala Cys Ser Gly Asn Leu Asn Lys Ala
    145                 150                 155

aag gaa atc ttt cag aag aaa ttc ctt gac aaa acg aaa aac aat tgg    527
Lys Glu Ile Phe Gln Lys Lys Phe Leu Asp Lys Thr Lys Asn Asn Trp
160                 165                 170                 175

gaa gat cga gaa aag ttt gag aag gtg cct gga aaa tat gat atg cta    575
Glu Asp Arg Glu Lys Phe Glu Lys Val Pro Gly Lys Tyr Asp Met Leu
                180                 185                 190

cag atg gac tat gcc acc aat act cag gat gaa gag gaa aca aag aaa    623
Gln Met Asp Tyr Ala Thr Asn Thr Gln Asp Glu Glu Glu Thr Lys Lys
            195                 200                 205

gag gaa tct ctt aaa tct ccc ttg aag cca gag tca cag cta gat ctt    671
Glu Glu Ser Leu Lys Ser Pro Leu Lys Pro Glu Ser Gln Leu Asp Leu
        210                 215                 220

cgg gta cag gag tta ata aag ttg atc tgt aat gtt cag gcc atg gaa    719
Arg Val Gln Glu Leu Ile Lys Leu Ile Cys Asn Val Gln Ala Met Glu
    225                 230                 235

gaa atg atg atg gaa atg aag tat aat acc aag aaa gcc cca ctt ggg    767
Glu Met Met Met Glu Met Lys Tyr Asn Thr Lys Lys Ala Pro Leu Gly
240                 245                 250                 255

aag ctg aca gtg gca caa atc aag gca ggt tac cag tct ctt aag aag    815
Lys Leu Thr Val Ala Gln Ile Lys Ala Gly Tyr Gln Ser Leu Lys Lys
                260                 265                 270

att gag gat tgt att cgg gct ggc cag cat gga cga gct ctc atg gaa    863
Ile Glu Asp Cys Ile Arg Ala Gly Gln His Gly Arg Ala Leu Met Glu
            275                 280                 285

gca tgc aat gaa ttc tac acc agg att ccg cat gac ttt gga ctc cgt    911
Ala Cys Asn Glu Phe Tyr Thr Arg Ile Pro His Asp Phe Gly Leu Arg
        290                 295                 300

act cct cca cta atc cgg aca cag aag gaa ctg tca gaa aaa ata caa    959
Thr Pro Pro Leu Ile Arg Thr Gln Lys Glu Leu Ser Glu Lys Ile Gln
    305                 310                 315

tta cta gag gct ttg gga gac att gaa att gct att aag ctg gtg aaa    1007
Leu Leu Glu Ala Leu Gly Asp Ile Glu Ile Ala Ile Lys Leu Val Lys
320                 325                 330                 335

aca gag cta caa agc cca gaa cac cca ttg gac caa cac tat aga aac    1055
Thr Glu Leu Gln Ser Pro Glu His Pro Leu Asp Gln His Tyr Arg Asn
                340                 345                 350

cta cat tgt gcc ttg cgc ccc ctt gac cat gaa agt tac gag ttc aaa    1103
Leu His Cys Ala Leu Arg Pro Leu Asp His Glu Ser Tyr Glu Phe Lys
            355                 360                 365

gtg att tcc cag tac cta caa tct acc cat gct ccc aca cac agc gac    1151
Val Ile Ser Gln Tyr Leu Gln Ser Thr His Ala Pro Thr His Ser Asp
        370                 375                 380

tat acc atg acc ttg ctg gat ttg ttt gaa gtg gag aag gat ggt gag    1199
Tyr Thr Met Thr Leu Leu Asp Leu Phe Glu Val Glu Lys Asp Gly Glu
    385                 390                 395
```

```
aaa gaa gcc ttc aga gag gac ctt cat aac agg atg ctt cta tgg cat    1247
Lys Glu Ala Phe Arg Glu Asp Leu His Asn Arg Met Leu Leu Trp His
400                 405                 410                 415

ggt tcc agg atg agt aac tgg gtg gga atc ttg agc cat ggg ctt cga    1295
Gly Ser Arg Met Ser Asn Trp Val Gly Ile Leu Ser His Gly Leu Arg
                420                 425                 430

att gcc cca cct gaa gct ccc atc aca ggt tac atg ttt ggg aaa gga    1343
Ile Ala Pro Pro Glu Ala Pro Ile Thr Gly Tyr Met Phe Gly Lys Gly
            435                 440                 445

atc tac ttt gct gac atg tct tcc aag agt gcc aat tac tgc ttt gcc    1391
Ile Tyr Phe Ala Asp Met Ser Ser Lys Ser Ala Asn Tyr Cys Phe Ala
        450                 455                 460

tct cgc cta aag aat aca gga ctg ctg ctc tta tca gag gta gct cta    1439
Ser Arg Leu Lys Asn Thr Gly Leu Leu Leu Leu Ser Glu Val Ala Leu
    465                 470                 475

ggt cag tgt aat gaa cta cta gag gcc aat cct aag gcc gaa gga ttg    1487
Gly Gln Cys Asn Glu Leu Leu Glu Ala Asn Pro Lys Ala Glu Gly Leu
480                 485                 490                 495

ctt caa ggt aaa cat agc acc aag ggg ctg ggc aag atg gct ccc agt    1535
Leu Gln Gly Lys His Ser Thr Lys Gly Leu Gly Lys Met Ala Pro Ser
                500                 505                 510

tct gcc cac ttc gtc acc ctg aat ggg agt aca gtg cca tta gga cca    1583
Ser Ala His Phe Val Thr Leu Asn Gly Ser Thr Val Pro Leu Gly Pro
            515                 520                 525

gca agt gac aca gga att ctg aat cca gat ggt tat acc ctc aac tac    1631
Ala Ser Asp Thr Gly Ile Leu Asn Pro Asp Gly Tyr Thr Leu Asn Tyr
        530                 535                 540

aat gaa tat att gta tat aac ccc aac cag gtc cgt atg cgg tac ctt    1679
Asn Glu Tyr Ile Val Tyr Asn Pro Asn Gln Val Arg Met Arg Tyr Leu
    545                 550                 555

tta aag gtt cag ttt aat ttc ctt cag ctg tgg tga atgttgatat         1725
Leu Lys Val Gln Phe Asn Phe Leu Gln Leu Trp
560                 565                 570

taaataaacc agagatctga tcttcaagca agaaaataag cagtgttgta cttgtgaatt 1785

ttgtgatatt ttatgtaata aaaactgtac aggtctaaaa aaaaaaaaaa aaaaaaa    1843
```

<210> 2
<211> 570
<212> PRT
<213> Brain

<400> 2

Met Ala Ala Arg Arg Arg Arg Ser Thr Gly Gly Gly Arg Ala Arg Ala
1 5 10 15

Leu Asn Glu Ser Lys Arg Val Asn Asn Gly Asn Thr Ala Pro Glu Asp
20 25 30

```
Ser Ser Pro Ala Lys Lys Thr Arg Arg Cys Gln Arg Gln Glu Ser Lys
        35                  40                  45

Lys Met Pro Val Ala Gly Gly Lys Ala Asn Lys Asp Arg Thr Glu Asp
    50                  55                  60

Lys Gln Asp Glu Ser Val Lys Ala Leu Leu Leu Lys Gly Lys Ala Pro
65                  70                  75                  80

Val Asp Pro Glu Cys Thr Ala Lys Val Gly Lys Ala His Val Tyr Cys
                85                  90                  95

Glu Gly Asn Asp Val Tyr Asp Val Met Leu Asn Gln Thr Asn Leu Gln
            100                 105                 110

Phe Asn Asn Asn Lys Tyr Tyr Leu Ile Gln Leu Leu Glu Asp Asp Ala
        115                 120                 125

Gln Arg Asn Phe Ser Val Trp Met Arg Trp Gly Arg Val Gly Lys Met
    130                 135                 140

Gly Gln His Ser Leu Val Ala Cys Ser Gly Asn Leu Asn Lys Ala Lys
145                 150                 155                 160

Glu Ile Phe Gln Lys Lys Phe Leu Asp Lys Thr Lys Asn Asn Trp Glu
                165                 170                 175

Asp Arg Glu Lys Phe Glu Lys Val Pro Gly Lys Tyr Asp Met Leu Gln
            180                 185                 190

Met Asp Tyr Ala Thr Asn Thr Gln Asp Glu Glu Glu Thr Lys Lys Glu
        195                 200                 205

Glu Ser Leu Lys Ser Pro Leu Lys Pro Glu Ser Gln Leu Asp Leu Arg
    210                 215                 220

Val Gln Glu Leu Ile Lys Leu Ile Cys Asn Val Gln Ala Met Glu Glu
225                 230                 235                 240

Met Met Met Glu Met Lys Tyr Asn Thr Lys Lys Ala Pro Leu Gly Lys
                245                 250                 255

Leu Thr Val Ala Gln Ile Lys Ala Gly Tyr Gln Ser Leu Lys Lys Ile
            260                 265                 270

Glu Asp Cys Ile Arg Ala Gly Gln His Gly Arg Ala Leu Met Glu Ala
        275                 280                 285

Cys Asn Glu Phe Tyr Thr Arg Ile Pro His Asp Phe Gly Leu Arg Thr
    290                 295                 300

Pro Pro Leu Ile Arg Thr Gln Lys Glu Leu Ser Glu Lys Ile Gln Leu
305                 310                 315                 320

Leu Glu Ala Leu Gly Asp Ile Glu Ile Ala Ile Lys Leu Val Lys Thr
                325                 330                 335

Glu Leu Gln Ser Pro Glu His Pro Leu Asp Gln His Tyr Arg Asn Leu
            340                 345                 350

His Cys Ala Leu Arg Pro Leu Asp His Glu Ser Tyr Glu Phe Lys Val
        355                 360                 365
```

```
Ile Ser Gln Tyr Leu Gln Ser Thr His Ala Pro Thr His Ser Asp Tyr
    370                 375                 380

Thr Met Thr Leu Leu Asp Leu Phe Glu Val Glu Lys Asp Gly Glu Lys
385                 390                 395                 400

Glu Ala Phe Arg Glu Asp Leu His Asn Arg Met Leu Leu Trp His Gly
                405                 410                 415

Ser Arg Met Ser Asn Trp Val Gly Ile Leu Ser His Gly Leu Arg Ile
            420                 425                 430

Ala Pro Pro Glu Ala Pro Ile Thr Gly Tyr Met Phe Gly Lys Gly Ile
        435                 440                 445

Tyr Phe Ala Asp Met Ser Ser Lys Ser Ala Asn Tyr Cys Phe Ala Ser
    450                 455                 460

Arg Leu Lys Asn Thr Gly Leu Leu Leu Leu Ser Glu Val Ala Leu Gly
465                 470                 475                 480

Gln Cys Asn Glu Leu Leu Glu Ala Asn Pro Lys Ala Glu Gly Leu Leu
                485                 490                 495

Gln Gly Lys His Ser Thr Lys Gly Leu Gly Lys Met Ala Pro Ser Ser
            500                 505                 510

Ala His Phe Val Thr Leu Asn Gly Ser Thr Val Pro Leu Gly Pro Ala
        515                 520                 525

Ser Asp Thr Gly Ile Leu Asn Pro Asp Gly Tyr Thr Leu Asn Tyr Asn
    530                 535                 540

Glu Tyr Ile Val Tyr Asn Pro Asn Gln Val Arg Met Arg Tyr Leu Leu
545                 550                 555                 560

Lys Val Gln Phe Asn Phe Leu Gln Leu Trp
                565                 570
```

<210> 3
<211> 2265
<212> DNA
<213> Uterus

<220>
<221> CDS
<222> (242)..(1843)
<223> Product=Poly ADP Ribose Polymerase

<400> 3

```
tgggactggt cgcctgactc ggcctgcccc agcctctgct tcaccccact ggtggccaaa 60

tagccgatgt ctaatccccc acacaagctc atccccggcc tctgggattg ttgggaattc 120

tctccctaat tcacgcctga ggctcatgga gagttgctag acctgggact gccctgggag 180

gcgcacacaa ccaggccggg tggcagccag gacctctccc atgtccctgc ttttcttggc 240

c atg gct cca aag ccg aag ccc tgg gta cag act gag ggc cct gag aag 289
  Met Ala Pro Lys Pro Lys Pro Trp Val Gln Thr Glu Gly Pro Glu Lys
    1               5                  10                  15
```

```
aag aag ggc cgg cag gca gga agg gag gag gac ccc ttc cgc tcc acc    337
Lys Lys Gly Arg Gln Ala Gly Arg Glu Glu Asp Pro Phe Arg Ser Thr
            20                  25                  30

gct gag gcc ctc aag gcc ata ccc gca gag aag cgc ata atc cgc gtg    385
Ala Glu Ala Leu Lys Ala Ile Pro Ala Glu Lys Arg Ile Ile Arg Val
        35                  40                  45

gat cca aca tgt cca ctc agc agc aac ccc ggg acc cag gtg tat gag    433
Asp Pro Thr Cys Pro Leu Ser Ser Asn Pro Gly Thr Gln Val Tyr Glu
    50                  55                  60

gac tac aac tgc acc ctg aac cag acc aac atc gag aac aac aac aac    481
Asp Tyr Asn Cys Thr Leu Asn Gln Thr Asn Ile Glu Asn Asn Asn Asn
65                  70                  75                  80

aag ttc tac atc atc cag ctg ctc caa gac agc aac cgc ttc ttc acc    529
Lys Phe Tyr Ile Ile Gln Leu Leu Gln Asp Ser Asn Arg Phe Phe Thr
                85                  90                  95

tgc tgg aac cgc tgg ggc cgt gtg gga gag gtc ggc cag tca aag atc    577
Cys Trp Asn Arg Trp Gly Arg Val Gly Glu Val Gly Gln Ser Lys Ile
            100                 105                 110

aac cac ttc aca agg cta gaa gat gca aag aag gac ttt gag aag aaa    625
Asn His Phe Thr Arg Leu Glu Asp Ala Lys Lys Asp Phe Glu Lys Lys
        115                 120                 125

ttt cgg gaa aag acc aag aac aac tgg gca gag cgg gac cac ttt gtg    673
Phe Arg Glu Lys Thr Lys Asn Asn Trp Ala Glu Arg Asp His Phe Val
    130                 135                 140

tct cac ccg ggc aag tac aca ctt atc gaa gta cag gca gag gat gag    721
Ser His Pro Gly Lys Tyr Thr Leu Ile Glu Val Gln Ala Glu Asp Glu
145                 150                 155                 160

gcc cag gaa gct gtg gtg aag gtg gac aga ggc cca gtg agg act gtg    769
Ala Gln Glu Ala Val Val Lys Val Asp Arg Gly Pro Val Arg Thr Val
                165                 170                 175

act aag cgg gtg cag ccc tgc tcc ctg gac cca gcc acg cag aag ctc    817
Thr Lys Arg Val Gln Pro Cys Ser Leu Asp Pro Ala Thr Gln Lys Leu
            180                 185                 190

atc act aac atc ttc agc aag gag atg ttc aag aac acc atg gcc ctc    865
Ile Thr Asn Ile Phe Ser Lys Glu Met Phe Lys Asn Thr Met Ala Leu
        195                 200                 205

atg gac ctg gat gtg aag aag atg ccc ctg gga aag ctg agc aag caa    913
Met Asp Leu Asp Val Lys Lys Met Pro Leu Gly Lys Leu Ser Lys Gln
    210                 215                 220

cag att gca cgg ggt ttc gag gcc ttg gag gcg ctg gag gag gcc ctg    961
Gln Ile Ala Arg Gly Phe Glu Ala Leu Glu Ala Leu Glu Glu Ala Leu
225                 230                 235                 240

aaa ggc ccc acg gat ggt ggc caa agc ctg gag gag ctg tcc tca cac    1009
Lys Gly Pro Thr Asp Gly Gly Gln Ser Leu Glu Glu Leu Ser Ser His
                245                 250                 255
```

```
ttt tac acc gtc atc ccg cac aac ttc ggc cac agc cag ccc ccg ccc    1057
Phe Tyr Thr Val Ile Pro His Asn Phe Gly His Ser Gln Pro Pro Pro
            260                 265                 270

atc aat tcc cct gag ctt ctg cag gcc aag aag gac atg ctg ctg gtg    1105
Ile Asn Ser Pro Glu Leu Leu Gln Ala Lys Lys Asp Met Leu Leu Val
        275                 280                 285

ctg gcg gac atc gag ctg gcc cag gcc ctg cag gca gtc tct gag cag    1153
Leu Ala Asp Ile Glu Leu Ala Gln Ala Leu Gln Ala Val Ser Glu Gln
    290                 295                 300

gag aag acg gtg gag gag gtg cca cac ccc ctg gac cga gac tac cag    1201
Glu Lys Thr Val Glu Glu Val Pro His Pro Leu Asp Arg Asp Tyr Gln
305                 310                 315                 320

ctt ctc aag tgc cag ctg cag ctg cta gac tct gga gca cct gag tac    1249
Leu Leu Lys Cys Gln Leu Gln Leu Leu Asp Ser Gly Ala Pro Glu Tyr
                325                 330                 335

aag gtg ata cag acc tac tta gaa cag act ggc agc aac cac agg tgc    1297
Lys Val Ile Gln Thr Tyr Leu Glu Gln Thr Gly Ser Asn His Arg Cys
            340                 345                 350

cct aca ctt caa cac atc tgg aaa gta aac caa gaa ggg gag gaa gac    1345
Pro Thr Leu Gln His Ile Trp Lys Val Asn Gln Glu Gly Glu Glu Asp
        355                 360                 365

aga ttc cag gcc cac tcc aaa ctg ggt aat cgg aag ctg ctg tgg cat    1393
Arg Phe Gln Ala His Ser Lys Leu Gly Asn Arg Lys Leu Leu Trp His
    370                 375                 380

ggc acc aac atg gcc gtg gtg gcc gcc atc ctc act agt ggg ctc cgc    1441
Gly Thr Asn Met Ala Val Val Ala Ala Ile Leu Thr Ser Gly Leu Arg
385                 390                 395                 400

atc atg cca cat tct ggt ggg cgt gtt ggc aag ggc atc tac ttt gcc    1489
Ile Met Pro His Ser Gly Gly Arg Val Gly Lys Gly Ile Tyr Phe Ala
                405                 410                 415

tca gag aac agc aag tca gct gga tat gtt att ggc atg aag tgt ggg    1537
Ser Glu Asn Ser Lys Ser Ala Gly Tyr Val Ile Gly Met Lys Cys Gly
            420                 425                 430

gcc cac cat gtc ggc tac atg ttc ctg ggt gag gtg gcc ctg ggc aga    1585
Ala His His Val Gly Tyr Met Phe Leu Gly Glu Val Ala Leu Gly Arg
        435                 440                 445

gag cac cat atc aac acg gac aac ccc agc ttg aag agc cca cct cct    1633
Glu His His Ile Asn Thr Asp Asn Pro Ser Leu Lys Ser Pro Pro Pro
    450                 455                 460

ggc ttc gac agt gtc att gcc cga ggc cac acc gag cct gat ccg acc    1681
Gly Phe Asp Ser Val Ile Ala Arg Gly His Thr Glu Pro Asp Pro Thr
465                 470                 475                 480

cag gac act gag ttg gag ctg gat ggc cag caa gtg gtg gtg ccc cag    1729
Gln Asp Thr Glu Leu Glu Leu Asp Gly Gln Gln Val Val Val Pro Gln
                485                 490                 495

ggc cag cct gtg ccc tgc cca gag ttc agc agc tcc aca ttc tcc cag    1777
Gly Gln Pro Val Pro Cys Pro Glu Phe Ser Ser Ser Thr Phe Ser Gln
            500                 505                 510
```

```
agc gag tac ctc atc tac cag gag agc cag tgt cgc ctg cgc tac ctg    1825
Ser Glu Tyr Leu Ile Tyr Gln Glu Ser Gln Cys Arg Leu Arg Tyr Leu
        515                 520                 525

ctg gag gtc cac ctc tga gtgcccgccc tgtcccccgg ggtcctgcaa           1873
Leu Glu Val His Leu
    530

ggctggactg tgatcttcaa tcatcctgcc catctctggt acccctatat cactcctttt 1933

tttcaagaat acaatacgtt gttgttaact atagtcacca tgctgtacaa gatccctgaa 1993

cttatgcctc ctaactgaaa ttttgtattc tttgacacat ctgcccagtc cctctcctcc 2053

cagcccatgg taaccagcat ttgactcttt acttgtataa gggcagcttt tataggttcc 2113

acatgtaagt gagatcatgc agtgtttgtc tttctgtgcc tggcttattt cactcagcat 2173

aatgtgcacc gggttcaccc atgttttcat aaatgacaag atttcctcct ttaaaaaaaa 2233

aaaaaaaaaa aaaaaaaaaa aaaaaaaaaa aa                                2265
```

<210> 4
<211> 533
<212> PRT
<213> Uterus

<400> 4

```
Met Ala Pro Lys Pro Lys Pro Trp Val Gln Thr Glu Gly Pro Glu Lys
1               5                   10                  15

Lys Lys Gly Arg Gln Ala Gly Arg Glu Glu Asp Pro Phe Arg Ser Thr
            20                  25                  30

Ala Glu Ala Leu Lys Ala Ile Pro Ala Glu Lys Arg Ile Ile Arg Val
        35                  40                  45

Asp Pro Thr Cys Pro Leu Ser Ser Asn Pro Gly Thr Gln Val Tyr Glu
    50                  55                  60

Asp Tyr Asn Cys Thr Leu Asn Gln Thr Asn Ile Glu Asn Asn Asn Asn
65                  70                  75                  80

Lys Phe Tyr Ile Ile Gln Leu Leu Gln Asp Ser Asn Arg Phe Phe Thr
                85                  90                  95

Cys Trp Asn Arg Trp Gly Arg Val Gly Glu Val Gly Gln Ser Lys Ile
            100                 105                 110

Asn His Phe Thr Arg Leu Glu Asp Ala Lys Lys Asp Phe Glu Lys Lys
        115                 120                 125

Phe Arg Glu Lys Thr Lys Asn Asn Trp Ala Glu Arg Asp His Phe Val
    130                 135                 140

Ser His Pro Gly Lys Tyr Thr Leu Ile Glu Val Gln Ala Glu Asp Glu
145                 150                 155                 160

Ala Gln Glu Ala Val Val Lys Val Asp Arg Gly Pro Val Arg Thr Val
                165                 170                 175
```

Thr Lys Arg Val Gln Pro Cys Ser Leu Asp Pro Ala Thr Gln Lys Leu
180 185 190

Ile Thr Asn Ile Phe Ser Lys Glu Met Phe Lys Asn Thr Met Ala Leu
195 200 205

Met Asp Leu Asp Val Lys Lys Met Pro Leu Gly Lys Leu Ser Lys Gln
210 215 220

Gln Ile Ala Arg Gly Phe Glu Ala Leu Glu Ala Leu Glu Glu Ala Leu
225 230 235 240

Lys Gly Pro Thr Asp Gly Gly Gln Ser Leu Glu Glu Leu Ser Ser His
245 250 255

Phe Tyr Thr Val Ile Pro His Asn Phe Gly His Ser Gln Pro Pro Pro
260 265 270

Ile Asn Ser Pro Glu Leu Leu Gln Ala Lys Lys Asp Met Leu Leu Val
275 280 285

Leu Ala Asp Ile Glu Leu Ala Gln Ala Leu Gln Ala Val Ser Glu Gln
290 295 300

Glu Lys Thr Val Glu Glu Val Pro His Pro Leu Asp Arg Asp Tyr Gln
305 310 315 320

Leu Leu Lys Cys Gln Leu Gln Leu Leu Asp Ser Gly Ala Pro Glu Tyr
325 330 335

Lys Val Ile Gln Thr Tyr Leu Glu Gln Thr Gly Ser Asn His Arg Cys
340 345 350

Pro Thr Leu Gln His Ile Trp Lys Val Asn Gln Glu Gly Glu Glu Asp
355 360 365

Arg Phe Gln Ala His Ser Lys Leu Gly Asn Arg Lys Leu Leu Trp His
370 375 380

Gly Thr Asn Met Ala Val Val Ala Ala Ile Leu Thr Ser Gly Leu Arg
385 390 395 400

Ile Met Pro His Ser Gly Gly Arg Val Gly Lys Gly Ile Tyr Phe Ala
405 410 415

Ser Glu Asn Ser Lys Ser Ala Gly Tyr Val Ile Gly Met Lys Cys Gly
420 425 430

Ala His His Val Gly Tyr Met Phe Leu Gly Glu Val Ala Leu Gly Arg
435 440 445

Glu His His Ile Asn Thr Asp Asn Pro Ser Leu Lys Ser Pro Pro Pro
450 455 460

Gly Phe Asp Ser Val Ile Ala Arg Gly His Thr Glu Pro Asp Pro Thr
465 470 475 480

Gln Asp Thr Glu Leu Glu Leu Asp Gly Gln Gln Val Val Val Pro Gln
485 490 495

Gly Gln Pro Val Pro Cys Pro Glu Phe Ser Ser Ser Thr Phe Ser Gln
500 505 510

```
Ser Glu Tyr Leu Ile Tyr Gln Glu Ser Gln Cys Arg Leu Arg Tyr Leu
        515                 520                 525

Leu Glu Val His Leu
    530
```

<210> 5
<211> 2265
<212> DNA
<213> Uterus

<220>
<221> CDS
<222> (221)..(1843)
<223> Product=Poly ADP Ribose Polymerase

<400> 5

```
tgggactggt cgcctgactc ggcctgcccc agcctctgct tcaccccact ggtggccaaa 60

tagccgatgt ctaatccccc acacaagctc atccccggcc tctgggattg ttgggaattc 120

tctccctaat tcacgcctga ggctcatgga gagttgctag acctgggact gccctgggag 180

gcgcacacaa ccaggccggg tggcagccag gacctctccc atg tcc ctg ctt ttc   235
                                            Met Ser Leu Leu Phe
                                             1               5

ttg gcc atg gct cca aag ccg aag ccc tgg gta cag act gag ggc cct   283
Leu Ala Met Ala Pro Lys Pro Lys Pro Trp Val Gln Thr Glu Gly Pro
                10                  15                  20

gag aag aag aag ggc cgg cag gca gga agg gag gag gac ccc ttc cgc   331
Glu Lys Lys Lys Gly Arg Gln Ala Gly Arg Glu Glu Asp Pro Phe Arg
            25                  30                  35

tcc acc gct gag gcc ctc aag gcc ata ccc gca gag aag cgc ata atc   379
Ser Thr Ala Glu Ala Leu Lys Ala Ile Pro Ala Glu Lys Arg Ile Ile
        40                  45                  50

cgc gtg gat cca aca tgt cca ctc agc agc aac ccc ggg acc cag gtg   427
Arg Val Asp Pro Thr Cys Pro Leu Ser Ser Asn Pro Gly Thr Gln Val
    55                  60                  65

tat gag gac tac aac tgc acc ctg aac cag acc aac atc gag aac aac   475
Tyr Glu Asp Tyr Asn Cys Thr Leu Asn Gln Thr Asn Ile Glu Asn Asn
70                  75                  80                  85

aac aac aag ttc tac atc atc cag ctg ctc caa gac agc aac cgc ttc   523
Asn Asn Lys Phe Tyr Ile Ile Gln Leu Leu Gln Asp Ser Asn Arg Phe
                90                  95                  100

ttc acc tgc tgg aac cgc tgg ggc cgt gtg gga gag gtc ggc cag tca   571
Phe Thr Cys Trp Asn Arg Trp Gly Arg Val Gly Glu Val Gly Gln Ser
            105                 110                 115

aag atc aac cac ttc aca agg cta gaa gat gca aag aag gac ttt gag   619
Lys Ile Asn His Phe Thr Arg Leu Glu Asp Ala Lys Lys Asp Phe Glu
        120                 125                 130
```

```
aag aaa ttt cgg gaa aag acc aag aac aac tgg gca gag cgg gac cac    667
Lys Lys Phe Arg Glu Lys Thr Lys Asn Asn Trp Ala Glu Arg Asp His
    135                 140                 145

ttt gtg tct cac ccg ggc aag tac aca ctt atc gaa gta cag gca gag    715
Phe Val Ser His Pro Gly Lys Tyr Thr Leu Ile Glu Val Gln Ala Glu
150                 155                 160                 165

gat gag gcc cag gaa gct gtg gtg aag gtg gac aga ggc cca gtg agg    763
Asp Glu Ala Gln Glu Ala Val Val Lys Val Asp Arg Gly Pro Val Arg
                170                 175                 180

act gtg act aag cgg gtg cag ccc tgc tcc ctg gac cca gcc acg cag    811
Thr Val Thr Lys Arg Val Gln Pro Cys Ser Leu Asp Pro Ala Thr Gln
            185                 190                 195

aag ctc atc act aac atc ttc agc aag gag atg ttc aag aac acc atg    859
Lys Leu Ile Thr Asn Ile Phe Ser Lys Glu Met Phe Lys Asn Thr Met
        200                 205                 210

gcc ctc atg gac ctg gat gtg aag aag atg ccc ctg gga aag ctg agc    907
Ala Leu Met Asp Leu Asp Val Lys Lys Met Pro Leu Gly Lys Leu Ser
    215                 220                 225

aag caa cag att gca cgg ggt ttc gag gcc ttg gag gcg ctg gag gag    955
Lys Gln Gln Ile Ala Arg Gly Phe Glu Ala Leu Glu Ala Leu Glu Glu
230                 235                 240                 245

gcc ctg aaa ggc ccc acg gat ggt ggc caa agc ctg gag gag ctg tcc    1003
Ala Leu Lys Gly Pro Thr Asp Gly Gly Gln Ser Leu Glu Glu Leu Ser
                250                 255                 260

tca cac ttt tac acc gtc atc ccg cac aac ttc ggc cac agc cag ccc    1051
Ser His Phe Tyr Thr Val Ile Pro His Asn Phe Gly His Ser Gln Pro
            265                 270                 275

ccg ccc atc aat tcc cct gag ctt ctg cag gcc aag aag gac atg ctg    1099
Pro Pro Ile Asn Ser Pro Glu Leu Leu Gln Ala Lys Lys Asp Met Leu
        280                 285                 290

ctg gtg ctg gcg gac atc gag ctg gcc cag gcc ctg cag gca gtc tct    1147
Leu Val Leu Ala Asp Ile Glu Leu Ala Gln Ala Leu Gln Ala Val Ser
    295                 300                 305

gag cag gag aag acg gtg gag gag gtg cca cac ccc ctg gac cga gac    1195
Glu Gln Glu Lys Thr Val Glu Glu Val Pro His Pro Leu Asp Arg Asp
310                 315                 320                 325

tac cag ctt ctc aag tgc cag ctg cag ctg cta gac tct gga gca cct    1243
Tyr Gln Leu Leu Lys Cys Gln Leu Gln Leu Leu Asp Ser Gly Ala Pro
                330                 335                 340

gag tac aag gtg ata cag acc tac tta gaa cag act ggc agc aac cac    1291
Glu Tyr Lys Val Ile Gln Thr Tyr Leu Glu Gln Thr Gly Ser Asn His
            345                 350                 355

agg tgc cct aca ctt caa cac atc tgg aaa gta aac caa gaa ggg gag    1339
Arg Cys Pro Thr Leu Gln His Ile Trp Lys Val Asn Gln Glu Gly Glu
        360                 365                 370

gaa gac aga ttc cag gcc cac tcc aaa ctg ggt aat cgg aag ctg ctg    1387
Glu Asp Arg Phe Gln Ala His Ser Lys Leu Gly Asn Arg Lys Leu Leu
    375                 380                 385
```

```
tgg cat ggc acc aac atg gcc gtg gtg gcc gcc atc ctc act agt ggg     1435
Trp His Gly Thr Asn Met Ala Val Val Ala Ala Ile Leu Thr Ser Gly
390                 395                 400                 405

ctc cgc atc atg cca cat tct ggt ggg cgt gtt ggc aag ggc atc tac     1483
Leu Arg Ile Met Pro His Ser Gly Gly Arg Val Gly Lys Gly Ile Tyr
                410                 415                 420

ttt gcc tca gag aac agc aag tca gct gga tat gtt att ggc atg aag     1531
Phe Ala Ser Glu Asn Ser Lys Ser Ala Gly Tyr Val Ile Gly Met Lys
            425                 430                 435

tgt ggg gcc cac cat gtc ggc tac atg ttc ctg ggt gag gtg gcc ctg     1579
Cys Gly Ala His His Val Gly Tyr Met Phe Leu Gly Glu Val Ala Leu
        440                 445                 450

ggc aga gag cac cat atc aac acg gac aac ccc agc ttg aag agc cca     1627
Gly Arg Glu His His Ile Asn Thr Asp Asn Pro Ser Leu Lys Ser Pro
    455                 460                 465

cct cct ggc ttc gac agt gtc att gcc cga ggc cac acc gag cct gat     1675
Pro Pro Gly Phe Asp Ser Val Ile Ala Arg Gly His Thr Glu Pro Asp
470                 475                 480                 485

ccg acc cag gac act gag ttg gag ctg gat ggc cag caa gtg gtg gtg     1723
Pro Thr Gln Asp Thr Glu Leu Glu Leu Asp Gly Gln Gln Val Val Val
                490                 495                 500

ccc cag ggc cag cct gtg ccc tgc cca gag ttc agc agc tcc aca ttc     1771
Pro Gln Gly Gln Pro Val Pro Cys Pro Glu Phe Ser Ser Ser Thr Phe
            505                 510                 515

tcc cag agc gag tac ctc atc tac cag gag agc cag tgt cgc ctg cgc     1819
Ser Gln Ser Glu Tyr Leu Ile Tyr Gln Glu Ser Gln Cys Arg Leu Arg
        520                 525                 530

tac ctg ctg gag gtc cac ctc tga gtgcccgccc tgtcccccgg ggtcctgcaa    1873
Tyr Leu Leu Glu Val His Leu
    535                 540

ggctggactg tgatcttcaa tcatcctgcc catctctggt acccctatat cactcctttt 1933

tttcaagaat acaatacgtt gttgttaact atagtcacca tgctgtacaa gatccctgaa 1993

cttatgcctc ctaactgaaa ttttgtattc tttgacacat ctgcccagtc cctctcctcc 2053

cagcccatgg taaccagcat ttgactcttt acttgtataa gggcagcttt tataggttcc 2113

acatgtaagt gagatcatgc agtgtttgtc tttctgtgcc tggcttattt cactcagcat 2173

aatgtgcacc gggttcaccc atgttttcat aaatgacaag atttcctcct ttaaaaaaaa 2233

aaaaaaaaaa aaaaaaaaaa aaaaaaaaaa aa                               2265
```

<210> 6
<211> 540
<212> PRT
<213> Uterus

<400> 6

```
Met Ser Leu Leu Phe Leu Ala Met Ala Pro Lys Pro Lys Pro Trp Val
 1               5                  10                  15

Gln Thr Glu Gly Pro Glu Lys Lys Lys Gly Arg Gln Ala Gly Arg Glu
            20                  25                  30

Glu Asp Pro Phe Arg Ser Thr Ala Glu Ala Leu Lys Ala Ile Pro Ala
        35                  40                  45

Glu Lys Arg Ile Ile Arg Val Asp Pro Thr Cys Pro Leu Ser Ser Asn
    50                  55                  60

Pro Gly Thr Gln Val Tyr Glu Asp Tyr Asn Cys Thr Leu Asn Gln Thr
65                  70                  75                  80

Asn Ile Glu Asn Asn Asn Asn Lys Phe Tyr Ile Ile Gln Leu Leu Gln
                85                  90                  95

Asp Ser Asn Arg Phe Phe Thr Cys Trp Asn Arg Trp Gly Arg Val Gly
            100                 105                 110

Glu Val Gly Gln Ser Lys Ile Asn His Phe Thr Arg Leu Glu Asp Ala
        115                 120                 125

Lys Lys Asp Phe Glu Lys Lys Phe Arg Glu Lys Thr Lys Asn Asn Trp
    130                 135                 140

Ala Glu Arg Asp His Phe Val Ser His Pro Gly Lys Tyr Thr Leu Ile
145                 150                 155                 160

Glu Val Gln Ala Glu Asp Glu Ala Gln Glu Ala Val Val Lys Val Asp
                165                 170                 175

Arg Gly Pro Val Arg Thr Val Thr Lys Arg Val Gln Pro Cys Ser Leu
            180                 185                 190

Asp Pro Ala Thr Gln Lys Leu Ile Thr Asn Ile Phe Ser Lys Glu Met
        195                 200                 205

Phe Lys Asn Thr Met Ala Leu Met Asp Leu Asp Val Lys Lys Met Pro
    210                 215                 220

Leu Gly Lys Leu Ser Lys Gln Gln Ile Ala Arg Gly Phe Glu Ala Leu
225                 230                 235                 240

Glu Ala Leu Glu Glu Ala Leu Lys Gly Pro Thr Asp Gly Gly Gln Ser
                245                 250                 255

Leu Glu Glu Leu Ser Ser His Phe Tyr Thr Val Ile Pro His Asn Phe
            260                 265                 270

Gly His Ser Gln Pro Pro Pro Ile Asn Ser Pro Glu Leu Leu Gln Ala
        275                 280                 285

Lys Lys Asp Met Leu Leu Val Leu Ala Asp Ile Glu Leu Ala Gln Ala
    290                 295                 300

Leu Gln Ala Val Ser Glu Gln Glu Lys Thr Val Glu Glu Val Pro His
305                 310                 315                 320

Pro Leu Asp Arg Asp Tyr Gln Leu Leu Lys Cys Gln Leu Gln Leu Leu
                325                 330                 335
```

```
Asp Ser Gly Ala Pro Glu Tyr Lys Val Ile Gln Thr Tyr Leu Glu Gln
            340                 345                 350

Thr Gly Ser Asn His Arg Cys Pro Thr Leu Gln His Ile Trp Lys Val
        355                 360                 365

Asn Gln Glu Gly Glu Glu Asp Arg Phe Gln Ala His Ser Lys Leu Gly
    370                 375                 380

Asn Arg Lys Leu Leu Trp His Gly Thr Asn Met Ala Val Val Ala Ala
385                 390                 395                 400

Ile Leu Thr Ser Gly Leu Arg Ile Met Pro His Ser Gly Gly Arg Val
                405                 410                 415

Gly Lys Gly Ile Tyr Phe Ala Ser Glu Asn Ser Lys Ser Ala Gly Tyr
            420                 425                 430

Val Ile Gly Met Lys Cys Gly Ala His His Val Gly Tyr Met Phe Leu
        435                 440                 445

Gly Glu Val Ala Leu Gly Arg Glu His His Ile Asn Thr Asp Asn Pro
    450                 455                 460

Ser Leu Lys Ser Pro Pro Pro Gly Phe Asp Ser Val Ile Ala Arg Gly
465                 470                 475                 480

His Thr Glu Pro Asp Pro Thr Gln Asp Thr Glu Leu Glu Leu Asp Gly
                485                 490                 495

Gln Gln Val Val Val Pro Gln Gly Gln Pro Val Pro Cys Pro Glu Phe
            500                 505                 510

Ser Ser Ser Thr Phe Ser Gln Ser Glu Tyr Leu Ile Tyr Gln Glu Ser
        515                 520                 525

Gln Cys Arg Leu Arg Tyr Leu Leu Glu Val His Leu
    530                 535                 540
```

<210> 7
<211> 1740
<212> DNA
<213> Mus musculus

<220>
<221> CDS
<222> (112)..(1710)

<400> 7

```
cccggctttc actttttctg ctgcctcggg gaacacctcg agccaactgc ttcctaactc 60

agggtgggca gaactgacgg gatctaagct tctgcatctc tgaggagaac c atg gct  117
                                                         Met Ala
                                                           1

cca aaa cga aag gcc tct gtg cag act gag ggc tcc aag aag cag cga   165
Pro Lys Arg Lys Ala Ser Val Gln Thr Glu Gly Ser Lys Lys Gln Arg
        5                  10                  15
```

```
caa ggg aca gag gag gag gac agc ttc cgg tcc act gcc gag gct ctc    213
Gln Gly Thr Glu Glu Glu Asp Ser Phe Arg Ser Thr Ala Glu Ala Leu
     20                  25                  30

aga gca gca cct gct gat aat cgg gtc atc cgt gtg gac ccc tca tgt    261
Arg Ala Ala Pro Ala Asp Asn Arg Val Ile Arg Val Asp Pro Ser Cys
 35                  40                  45                  50

cca ttc agc cgg aac ccc ggg ata cag gtc cac gag gac tat gac tgt    309
Pro Phe Ser Arg Asn Pro Gly Ile Gln Val His Glu Asp Tyr Asp Cys
                 55                  60                  65

acc ctg aac cag acc aac atc ggc aac aac aac aac aag ttc tat att    357
Thr Leu Asn Gln Thr Asn Ile Gly Asn Asn Asn Asn Lys Phe Tyr Ile
             70                  75                  80

atc caa ctg ctg gag gag ggt agt cgc ttc ttc tgc tgg aat cgc tgg    405
Ile Gln Leu Leu Glu Glu Gly Ser Arg Phe Phe Cys Trp Asn Arg Trp
         85                  90                  95

ggc cgc gtg gga gag gtg ggc cag agc aag atg aac cac ttc acc tgc    453
Gly Arg Val Gly Glu Val Gly Gln Ser Lys Met Asn His Phe Thr Cys
    100                 105                 110

ctg gaa gat gca aag aag gac ttt aag aag aaa ttt tgg gag aag act    501
Leu Glu Asp Ala Lys Lys Asp Phe Lys Lys Lys Phe Trp Glu Lys Thr
115                 120                 125                 130

aaa aac aaa tgg gag gag cgg gac cgt ttt gtg gcc cag ccc aac aag    549
Lys Asn Lys Trp Glu Glu Arg Asp Arg Phe Val Ala Gln Pro Asn Lys
                135                 140                 145

tac aca ctt ata gaa gtc cag gga gaa gca gag agc caa gag gct gta    597
Tyr Thr Leu Ile Glu Val Gln Gly Glu Ala Glu Ser Gln Glu Ala Val
            150                 155                 160

gtg aag gcc tta tct ccc cag gtg gac agc ggc cct gtg agg acc gtg    645
Val Lys Ala Leu Ser Pro Gln Val Asp Ser Gly Pro Val Arg Thr Val
        165                 170                 175

gtc aag ccc tgc tcc cta gac cct gcc acc cag aac ctt atc acc aac    693
Val Lys Pro Cys Ser Leu Asp Pro Ala Thr Gln Asn Leu Ile Thr Asn
    180                 185                 190

atc ttc agc aaa gag atg ttc aag aac gca atg acc ctc atg aac ctg    741
Ile Phe Ser Lys Glu Met Phe Lys Asn Ala Met Thr Leu Met Asn Leu
195                 200                 205                 210

gat gtg aag aag atg ccc ttg gga aag ctg acc aag cag cag att gcc    789
Asp Val Lys Lys Met Pro Leu Gly Lys Leu Thr Lys Gln Gln Ile Ala
                215                 220                 225

cgt ggc ttc gag gcc ttg gaa gct cta gag gag gcc atg aaa aac ccc    837
Arg Gly Phe Glu Ala Leu Glu Ala Leu Glu Glu Ala Met Lys Asn Pro
            230                 235                 240

aca ggg gat ggc cag agc ctg gaa gag ctc tcc tcc tgc ttc tac act    885
Thr Gly Asp Gly Gln Ser Leu Glu Glu Leu Ser Ser Cys Phe Tyr Thr
        245                 250                 255

gtc atc cca cac aac ttc ggc cgc agc cga ccc ccg ccc atc aac tcc    933
Val Ile Pro His Asn Phe Gly Arg Ser Arg Pro Pro Pro Ile Asn Ser
    260                 265                 270
```

```
cct gat gtg ctt cag gcc aag aag gac atg ctg ctg gtg cta gcg gac     981
Pro Asp Val Leu Gln Ala Lys Lys Asp Met Leu Leu Val Leu Ala Asp
275                 280                 285                 290

atc gag ttg gcg cag acc ttg cag gca gcc cct ggg gag gag gag gag    1029
Ile Glu Leu Ala Gln Thr Leu Gln Ala Ala Pro Gly Glu Glu Glu Glu
                295                 300                 305

aaa gtg gaa gag gtg cca cac cca ctg gat cga gac tac cag ctc ctc    1077
Lys Val Glu Glu Val Pro His Pro Leu Asp Arg Asp Tyr Gln Leu Leu
            310                 315                 320

agg tgc cag ctt caa ctg ctg gac tcc ggg gag tcc gag tac aag gca    1125
Arg Cys Gln Leu Gln Leu Leu Asp Ser Gly Glu Ser Glu Tyr Lys Ala
        325                 330                 335

ata cag acc tac ctg aaa cag act ggc aac agc tac agg tgc cca aac    1173
Ile Gln Thr Tyr Leu Lys Gln Thr Gly Asn Ser Tyr Arg Cys Pro Asn
    340                 345                 350

ctg cgg cat gtt tgg aaa gtg aac cga gaa ggg gag gga gac agg ttc    1221
Leu Arg His Val Trp Lys Val Asn Arg Glu Gly Glu Gly Asp Arg Phe
355                 360                 365                 370

cag gcc cac tcc aaa ctg ggc aat cgg agg ctg ctg tgg cac ggc acc    1269
Gln Ala His Ser Lys Leu Gly Asn Arg Arg Leu Leu Trp His Gly Thr
                375                 380                 385

aat gtg gcc gtg gtg gct gcc atc ctc acc agt ggg ctc cga atc atg    1317
Asn Val Ala Val Val Ala Ala Ile Leu Thr Ser Gly Leu Arg Ile Met
            390                 395                 400

cca cac tcg ggt ggt cgt gtt ggc aag ggt att tat ttt gcc tct gag    1365
Pro His Ser Gly Gly Arg Val Gly Lys Gly Ile Tyr Phe Ala Ser Glu
        405                 410                 415

aac agc aag tca gct ggc tat gtt acc acc atg cac tgt ggg ggc cac    1413
Asn Ser Lys Ser Ala Gly Tyr Val Thr Thr Met His Cys Gly Gly His
    420                 425                 430

cag gtg ggc tac atg ttc ctg ggc gag gtg gcc ctc ggc aaa gag cac    1461
Gln Val Gly Tyr Met Phe Leu Gly Glu Val Ala Leu Gly Lys Glu His
435                 440                 445                 450

cac atc acc atc gat gac ccc agc ttg aag agt cca ccc cct ggc ttt    1509
His Ile Thr Ile Asp Asp Pro Ser Leu Lys Ser Pro Pro Pro Gly Phe
                455                 460                 465

gac agc gtc atc gcc cga ggc caa acc gag ccg gat ccc gcc cag gac    1557
Asp Ser Val Ile Ala Arg Gly Gln Thr Glu Pro Asp Pro Ala Gln Asp
            470                 475                 480

att gaa ctt gaa ctg gat ggg cag ccg gtg gtg gtg ccc caa ggc ccg    1605
Ile Glu Leu Glu Leu Asp Gly Gln Pro Val Val Val Pro Gln Gly Pro
        485                 490                 495

cct gtg cag tgc ccg tca ttc aaa agc tcc agc ttc agc cag agt gaa    1653
Pro Val Gln Cys Pro Ser Phe Lys Ser Ser Ser Phe Ser Gln Ser Glu
    500                 505                 510
```

```
tac ctc ata tac aag gag agc cag tgt cgc ctg cgc tac ctg ctg gag    1701
Tyr Leu Ile Tyr Lys Glu Ser Gln Cys Arg Leu Arg Tyr Leu Leu Glu
515                 520                 525                 530

att cac ctc taagctgctt gccctcccta ggtccaagcc                       1740
Ile His Leu
```

<210> 8
<211> 533
<212> PRT
<213> Mus musculus

<400> 8

```
Met Ala Pro Lys Arg Lys Ala Ser Val Gln Thr Glu Gly Ser Lys Lys
1               5                   10                  15

Gln Arg Gln Gly Thr Glu Glu Glu Asp Ser Phe Arg Ser Thr Ala Glu
            20                  25                  30

Ala Leu Arg Ala Ala Pro Ala Asp Asn Arg Val Ile Arg Val Asp Pro
        35                  40                  45

Ser Cys Pro Phe Ser Arg Asn Pro Gly Ile Gln Val His Glu Asp Tyr
    50                  55                  60

Asp Cys Thr Leu Asn Gln Thr Asn Ile Gly Asn Asn Asn Asn Lys Phe
65                  70                  75                  80

Tyr Ile Ile Gln Leu Leu Glu Glu Gly Ser Arg Phe Phe Cys Trp Asn
                85                  90                  95

Arg Trp Gly Arg Val Gly Glu Val Gly Gln Ser Lys Met Asn His Phe
            100                 105                 110

Thr Cys Leu Glu Asp Ala Lys Lys Asp Phe Lys Lys Lys Phe Trp Glu
        115                 120                 125

Lys Thr Lys Asn Lys Trp Glu Glu Arg Asp Arg Phe Val Ala Gln Pro
    130                 135                 140

Asn Lys Tyr Thr Leu Ile Glu Val Gln Gly Glu Ala Glu Ser Gln Glu
145                 150                 155                 160

Ala Val Val Lys Ala Leu Ser Pro Gln Val Asp Ser Gly Pro Val Arg
                165                 170                 175

Thr Val Val Lys Pro Cys Ser Leu Asp Pro Ala Thr Gln Asn Leu Ile
            180                 185                 190

Thr Asn Ile Phe Ser Lys Glu Met Phe Lys Asn Ala Met Thr Leu Met
        195                 200                 205

Asn Leu Asp Val Lys Lys Met Pro Leu Gly Lys Leu Thr Lys Gln Gln
    210                 215                 220

Ile Ala Arg Gly Phe Glu Ala Leu Glu Ala Leu Glu Glu Ala Met Lys
225                 230                 235                 240

Asn Pro Thr Gly Asp Gly Gln Ser Leu Glu Glu Leu Ser Ser Cys Phe
                245                 250                 255
```

```
Tyr Thr Val Ile Pro His Asn Phe Gly Arg Ser Arg Pro Pro Pro Ile
            260                 265                 270

Asn Ser Pro Asp Val Leu Gln Ala Lys Lys Asp Met Leu Leu Val Leu
        275                 280                 285

Ala Asp Ile Glu Leu Ala Gln Thr Leu Gln Ala Ala Pro Gly Glu Glu
    290                 295                 300

Glu Glu Lys Val Glu Glu Val Pro His Pro Leu Asp Arg Asp Tyr Gln
305                 310                 315                 320

Leu Leu Arg Cys Gln Leu Gln Leu Leu Asp Ser Gly Glu Ser Glu Tyr
                325                 330                 335

Lys Ala Ile Gln Thr Tyr Leu Lys Gln Thr Gly Asn Ser Tyr Arg Cys
            340                 345                 350

Pro Asn Leu Arg His Val Trp Lys Val Asn Arg Glu Gly Glu Gly Asp
        355                 360                 365

Arg Phe Gln Ala His Ser Lys Leu Gly Asn Arg Arg Leu Leu Trp His
    370                 375                 380

Gly Thr Asn Val Ala Val Val Ala Ala Ile Leu Thr Ser Gly Leu Arg
385                 390                 395                 400

Ile Met Pro His Ser Gly Gly Arg Val Gly Lys Gly Ile Tyr Phe Ala
                405                 410                 415

Ser Glu Asn Ser Lys Ser Ala Gly Tyr Val Thr Thr Met His Cys Gly
            420                 425                 430

Gly His Gln Val Gly Tyr Met Phe Leu Gly Glu Val Ala Leu Gly Lys
        435                 440                 445

Glu His His Ile Thr Ile Asp Asp Pro Ser Leu Lys Ser Pro Pro Pro
    450                 455                 460

Gly Phe Asp Ser Val Ile Ala Arg Gly Gln Thr Glu Pro Asp Pro Ala
465                 470                 475                 480

Gln Asp Ile Glu Leu Glu Leu Asp Gly Gln Pro Val Val Val Pro Gln
                485                 490                 495

Gly Pro Pro Val Gln Cys Pro Ser Phe Lys Ser Ser Ser Phe Ser Gln
            500                 505                 510

Ser Glu Tyr Leu Ile Tyr Lys Glu Ser Gln Cys Arg Leu Arg Tyr Leu
        515                 520                 525

Leu Glu Ile His Leu
    530
```

<210> 9
<211> 1587
<212> DNA
<213> Mus musculus

<220>
<221> CDS
<222> (1)..(1584)

<400> 9

```
atg gct cca aaa cga aag gcc tct gtg cag act gag ggc tcc aag aag    48
Met Ala Pro Lys Arg Lys Ala Ser Val Gln Thr Glu Gly Ser Lys Lys
  1               5                  10                  15

cag cga caa ggg aca gag gag gag gac agc ttc cgg tcc act gcc gag    96
Gln Arg Gln Gly Thr Glu Glu Glu Asp Ser Phe Arg Ser Thr Ala Glu
             20                  25                  30

gct ctc aga gca gca cct gct gat aat cgg gtc atc cgt gtg gac ccc   144
Ala Leu Arg Ala Ala Pro Ala Asp Asn Arg Val Ile Arg Val Asp Pro
         35                  40                  45

tca tgt cca ttc agc cgg aac ccc ggg ata cag gtc cac gag gac tat   192
Ser Cys Pro Phe Ser Arg Asn Pro Gly Ile Gln Val His Glu Asp Tyr
     50                  55                  60

gac tgt acc ctg aac cag acc aac atc ggc aac aac aac aac aag ttc   240
Asp Cys Thr Leu Asn Gln Thr Asn Ile Gly Asn Asn Asn Asn Lys Phe
 65                  70                  75                  80

tat att atc caa ctg ctg gag gag ggt agt cgc ttc ttc tgc tgg aat   288
Tyr Ile Ile Gln Leu Leu Glu Glu Gly Ser Arg Phe Phe Cys Trp Asn
                 85                  90                  95

cgc tgg ggc cgc gtg gga gag gtg ggc cag agc aag atg aac cac ttc   336
Arg Trp Gly Arg Val Gly Glu Val Gly Gln Ser Lys Met Asn His Phe
            100                 105                 110

acc tgc ctg gaa gat gca aag aag gac ttt aag aag aaa ttt tgg gag   384
Thr Cys Leu Glu Asp Ala Lys Lys Asp Phe Lys Lys Lys Phe Trp Glu
        115                 120                 125

aag act aaa aac aaa tgg gag gag cgg gac cgt ttt gtg gcc cag ccc   432
Lys Thr Lys Asn Lys Trp Glu Glu Arg Asp Arg Phe Val Ala Gln Pro
    130                 135                 140

aac aag tac aca ctt ata gaa gtc cag gga gaa gca gag agc caa gag   480
Asn Lys Tyr Thr Leu Ile Glu Val Gln Gly Glu Ala Glu Ser Gln Glu
145                 150                 155                 160

gct gta gtg aag gtg gac agc ggc cct gtg agg acc gtg gtc aag ccc   528
Ala Val Val Lys Val Asp Ser Gly Pro Val Arg Thr Val Val Lys Pro
                165                 170                 175

tgc tcc cta gac cct gcc acc cag aac ctt atc acc aac atc ttc agc   576
Cys Ser Leu Asp Pro Ala Thr Gln Asn Leu Ile Thr Asn Ile Phe Ser
            180                 185                 190

aaa gag atg ttc aag aac gca atg acc ctc atg aac ctg gat gtg aag   624
Lys Glu Met Phe Lys Asn Ala Met Thr Leu Met Asn Leu Asp Val Lys
        195                 200                 205

aag atg ccc ttg gga aag ctg acc aag cag cag att gcc cgt ggc ttc   672
Lys Met Pro Leu Gly Lys Leu Thr Lys Gln Gln Ile Ala Arg Gly Phe
    210                 215                 220

gag gcc ttg gaa gct cta gag gag gcc atg aaa aac ccc aca ggg gat   720
Glu Ala Leu Glu Ala Leu Glu Glu Ala Met Lys Asn Pro Thr Gly Asp
225                 230                 235                 240
```

```
ggc cag agc ctg gaa gag ctc tcc tcc tgc ttc tac act gtc atc cca      768
Gly Gln Ser Leu Glu Glu Leu Ser Ser Cys Phe Tyr Thr Val Ile Pro
                245                 250                 255

cac aac ttc ggc cgc agc cga ccc ccg ccc atc aac tcc cct gat gtg      816
His Asn Phe Gly Arg Ser Arg Pro Pro Pro Ile Asn Ser Pro Asp Val
            260                 265                 270

ctt cag gcc aag aag gac atg ctg ctg gtg cta gcg gac atc gag ttg      864
Leu Gln Ala Lys Lys Asp Met Leu Leu Val Leu Ala Asp Ile Glu Leu
        275                 280                 285

gcg cag acc ttg cag gca gcc cct ggg gag gag gag gag aaa gtg gaa      912
Ala Gln Thr Leu Gln Ala Ala Pro Gly Glu Glu Glu Glu Lys Val Glu
    290                 295                 300

gag gtg cca cac cca ctg gat cga gac tac cag ctc ctc agg tgc cag      960
Glu Val Pro His Pro Leu Asp Arg Asp Tyr Gln Leu Leu Arg Cys Gln
305                 310                 315                 320

ctt caa ctg ctg gac tcc ggg gag tcc gag tac aag gca ata cag acc     1008
Leu Gln Leu Leu Asp Ser Gly Glu Ser Glu Tyr Lys Ala Ile Gln Thr
                325                 330                 335

tac ctg aaa cag act ggc aac agc tac agg tgc cca aac ctg cgg cat     1056
Tyr Leu Lys Gln Thr Gly Asn Ser Tyr Arg Cys Pro Asn Leu Arg His
            340                 345                 350

gtt tgg aaa gtg aac cga gaa ggg gag gga gac agg ttc cag gcc cac     1104
Val Trp Lys Val Asn Arg Glu Gly Glu Gly Asp Arg Phe Gln Ala His
        355                 360                 365

tcc aaa ctg ggc aat cgg agg ctg ctg tgg cac ggc acc aat gtg gcc     1152
Ser Lys Leu Gly Asn Arg Arg Leu Leu Trp His Gly Thr Asn Val Ala
    370                 375                 380

gtg gtg gct gcc atc ctc acc agt ggg ctc cga atc atg cca cac tcg     1200
Val Val Ala Ala Ile Leu Thr Ser Gly Leu Arg Ile Met Pro His Ser
385                 390                 395                 400

ggt ggt cgt gtt ggc aag ggt att tat ttt gcc tct gag aac agc aag     1248
Gly Gly Arg Val Gly Lys Gly Ile Tyr Phe Ala Ser Glu Asn Ser Lys
                405                 410                 415

tca gct ggc tat gtt acc acc atg cac tgt ggg ggc cac cag gtg ggc     1296
Ser Ala Gly Tyr Val Thr Thr Met His Cys Gly Gly His Gln Val Gly
            420                 425                 430

tac atg ttc ctg ggc gag gtg gcc ctc ggc aaa gag cac cac atc acc     1344
Tyr Met Phe Leu Gly Glu Val Ala Leu Gly Lys Glu His His Ile Thr
        435                 440                 445

atc gat gac ccc agc ttg aag agt cca ccc cct ggc ttt gac agc gtc     1392
Ile Asp Asp Pro Ser Leu Lys Ser Pro Pro Pro Gly Phe Asp Ser Val
    450                 455                 460

atc gcc cga ggc caa acc gag ccg gat ccc gcc cag gac att gaa ctt     1440
Ile Ala Arg Gly Gln Thr Glu Pro Asp Pro Ala Gln Asp Ile Glu Leu
465                 470                 475                 480

gaa ctg gat ggg cag ccg gtg gtg gtg ccc caa ggc ccg cct gtg cag     1488
Glu Leu Asp Gly Gln Pro Val Val Val Pro Gln Gly Pro Pro Val Gln
                485                 490                 495
```

```
tgc ccg tca ttc aaa agc tcc agc ttc agc cag agt gaa tac ctc ata    1536
Cys Pro Ser Phe Lys Ser Ser Ser Phe Ser Gln Ser Glu Tyr Leu Ile
            500                 505                 510

tac aag gag agc cag tgt cgc ctg cgc tac ctg ctg gag att cac ctc    1584
Tyr Lys Glu Ser Gln Cys Arg Leu Arg Tyr Leu Leu Glu Ile His Leu
        515                 520                 525

taa                                                                1587
```

<210> 10
<211> 528
<212> PRT
<213> Mus musculus

<400> 10

```
Met Ala Pro Lys Arg Lys Ala Ser Val Gln Thr Glu Gly Ser Lys Lys
1               5                   10                  15

Gln Arg Gln Gly Thr Glu Glu Glu Asp Ser Phe Arg Ser Thr Ala Glu
            20                  25                  30

Ala Leu Arg Ala Ala Pro Ala Asp Asn Arg Val Ile Arg Val Asp Pro
        35                  40                  45

Ser Cys Pro Phe Ser Arg Asn Pro Gly Ile Gln Val His Glu Asp Tyr
    50                  55                  60

Asp Cys Thr Leu Asn Gln Thr Asn Ile Gly Asn Asn Asn Asn Lys Phe
65                  70                  75                  80

Tyr Ile Ile Gln Leu Leu Glu Glu Gly Ser Arg Phe Phe Cys Trp Asn
                85                  90                  95

Arg Trp Gly Arg Val Gly Glu Val Gly Gln Ser Lys Met Asn His Phe
            100                 105                 110

Thr Cys Leu Glu Asp Ala Lys Lys Asp Phe Lys Lys Lys Phe Trp Glu
        115                 120                 125

Lys Thr Lys Asn Lys Trp Glu Glu Arg Asp Arg Phe Val Ala Gln Pro
    130                 135                 140

Asn Lys Tyr Thr Leu Ile Glu Val Gln Gly Glu Ala Glu Ser Gln Glu
145                 150                 155                 160

Ala Val Val Lys Val Asp Ser Gly Pro Val Arg Thr Val Val Lys Pro
                165                 170                 175

Cys Ser Leu Asp Pro Ala Thr Gln Asn Leu Ile Thr Asn Ile Phe Ser
            180                 185                 190

Lys Glu Met Phe Lys Asn Ala Met Thr Leu Met Asn Leu Asp Val Lys
        195                 200                 205

Lys Met Pro Leu Gly Lys Leu Thr Lys Gln Gln Ile Ala Arg Gly Phe
    210                 215                 220

Glu Ala Leu Glu Ala Leu Glu Glu Ala Met Lys Asn Pro Thr Gly Asp
225                 230                 235                 240
```

```
Gly Gln Ser Leu Glu Glu Leu Ser Ser Cys Phe Tyr Thr Val Ile Pro
                245                 250                 255

His Asn Phe Gly Arg Ser Arg Pro Pro Pro Ile Asn Ser Pro Asp Val
            260                 265                 270

Leu Gln Ala Lys Lys Asp Met Leu Leu Val Leu Ala Asp Ile Glu Leu
        275                 280                 285

Ala Gln Thr Leu Gln Ala Ala Pro Gly Glu Glu Glu Glu Lys Val Glu
    290                 295                 300

Glu Val Pro His Pro Leu Asp Arg Asp Tyr Gln Leu Leu Arg Cys Gln
305                 310                 315                 320

Leu Gln Leu Leu Asp Ser Gly Glu Ser Glu Tyr Lys Ala Ile Gln Thr
                325                 330                 335

Tyr Leu Lys Gln Thr Gly Asn Ser Tyr Arg Cys Pro Asn Leu Arg His
            340                 345                 350

Val Trp Lys Val Asn Arg Glu Gly Glu Gly Asp Arg Phe Gln Ala His
        355                 360                 365

Ser Lys Leu Gly Asn Arg Arg Leu Leu Trp His Gly Thr Asn Val Ala
    370                 375                 380

Val Val Ala Ala Ile Leu Thr Ser Gly Leu Arg Ile Met Pro His Ser
385                 390                 395                 400

Gly Gly Arg Val Gly Lys Gly Ile Tyr Phe Ala Ser Glu Asn Ser Lys
                405                 410                 415

Ser Ala Gly Tyr Val Thr Thr Met His Cys Gly Gly His Gln Val Gly
            420                 425                 430

Tyr Met Phe Leu Gly Glu Val Ala Leu Gly Lys Glu His His Ile Thr
        435                 440                 445

Ile Asp Asp Pro Ser Leu Lys Ser Pro Pro Pro Gly Phe Asp Ser Val
    450                 455                 460

Ile Ala Arg Gly Gln Thr Glu Pro Asp Pro Ala Gln Asp Ile Glu Leu
465                 470                 475                 480

Glu Leu Asp Gly Gln Pro Val Val Val Pro Gln Gly Pro Pro Val Gln
                485                 490                 495

Cys Pro Ser Phe Lys Ser Ser Ser Phe Ser Gln Ser Glu Tyr Leu Ile
            500                 505                 510

Tyr Lys Glu Ser Gln Cys Arg Leu Arg Tyr Leu Leu Glu Ile His Leu
        515                 520                 525
```

<210> 11
<211> 14
<212> PRT
<213> Künstliche Sequenz

<220>
<221> SITE
<222> (2)
<223> Xaa is 1 to 5 other amino acids

<220>
<221> SITE
<222> (3)
<223> Xaa is Thr or Ser

<220>
<223> Beschreibung der künstlichen Sequenz: peptide

<400> 11

```
Pro Xaa Xaa Gly Xaa Xaa Xaa Gly Lys Gly Ile Tyr Phe Ala
  1               5                  10
```

<210> 12
<211> 21
<212> PRT
<213> Künstliche Sequenz

<220>
<221> SITE
<222> (1)
<223> Xaa is Ser or Thr

<220>
<221> SITE
<222> (6)
<223> Xaa is Ile or Val

<220>
<221> SITE
<222> (9)
<223> Xaa is 1 to 5 other amino acids

<220>
<221> SITE
<222> (10)
<223> Xaa is Ser or Thr

<220>
<223> Beschreibung der künstlichen Sequenz: peptide

<400> 12

```
Xaa Xaa Gly Leu Arg Xaa Xaa Pro Xaa Xaa Gly Xaa Xaa Xaa Gly Lys
  1               5                  10                  15

Gly Ile Tyr Phe Ala
             20
```

<210> 13

<211> 45
<212> PRT
<213> Künstliche Sequenz

<220>
<223> Beschreibung der künstlichen Sequenz: peptide

<220>
<221> SITE
<222> (6)
<223> Xaa is Ser or Thr

<220>
<221> SITE
<222> (16)
<223> Xaa is Ser or Thr

<220>
<221> SITE
<222> (21)
<223> Xaa is Ile or Val

<220>
<221> SITE
<222> (24)
<223> Xaa is 1 to 5 other amino acids

<220>
<221> SITE
<222> (25)
<223> Xaa is Ser or Thr

<400> 13

```
Leu Leu Trp His Gly Xaa Xaa Xaa Xaa Xaa Xaa Xaa Xaa Ile Leu Xaa
  1               5                  10                  15

Xaa Gly Leu Arg Xaa Xaa Pro Xaa Xaa Gly Xaa Xaa Xaa Gly Lys Gly
            20                  25                  30

Ile Tyr Phe Ala Xaa Xaa Xaa Ser Lys Ser Ala Xaa Tyr
        35                  40                  45
```

<210> 14
<211> 22
<212> PRT
<213> Künstliche Sequenz

<220>
<221> SITE
<222> (1)
<223> Xaa is Leu or Val

<220>
<223> Beschreibung der künstlichen Sequenz: peptide

<400> 14

```
Xaa Xaa Xaa Xaa Xaa Xaa Xaa Leu Xaa Xaa Xaa Xaa Xaa Xaa Leu Xaa
 1               5                  10                  15

Xaa Xaa Xaa Xaa Xaa Leu
            20
```

<210> 15
<211> 27
<212> PRT
<213> Künstliche Sequenz

<220>
<221> SITE
<222> (21)
<223> Xaa is Asp or Glu

<220>
<221> SITE
<222> (22)
<223> Xaa is 10 or 11 other amino acids

<220>
<223> Beschreibung der künstlichen Sequenz: peptide

<400> 15

```
Leu Xaa Xaa Xaa Xaa Xaa Xaa Xaa Xaa Xaa Asn Xaa Xaa Tyr Xaa Xaa
 1               5                  10                  15

Gln Leu Leu Xaa Xaa Xaa Trp Gly Arg Val Gly
            20                  25
```

<210> 16
<211> 29
<212> PRT
<213> Künstliche Sequenz

<220>
<223> Beschreibung der künstlichen Sequenz: peptide

<400> 16

```
Ala Xaa Xaa Xaa Phe Xaa Lys Xaa Xaa Xaa Xaa Lys Thr Xaa Asn Xaa
 1               5                  10                  15

Trp Xaa Xaa Xaa Xaa Xaa Phe Xaa Xaa Xaa Pro Xaa Lys
            20                  25
```

<210> 17
<211> 44
<212> PRT
<213> Künstliche Sequenz

<220>
<221> SITE

<222> (4)
<223> Xaa is Ile or Leu

<220>
<223> Beschreibung der künstlichen Sequenz: peptide

<400> 17

```
Gln Xaa Leu Xaa Xaa Xaa Ile Xaa Xaa Xaa Xaa Xaa Xaa Xaa Xaa Xaa
 1               5                  10                  15

Met Xaa Xaa Xaa Xaa Xaa Xaa Xaa Xaa Xaa Xaa Pro Leu Gly Lys Leu
            20                  25                  30

Xaa Xaa Xaa Gln Ile Xaa Xaa Xaa Xaa Xaa Xaa Leu
        35                  40
```

<210> 18
<211> 15
<212> PRT
<213> Künstliche Sequenz

<220>
<223> Beschreibung der künstlichen Sequenz: peptide

<400> 18

```
Phe Tyr Thr Xaa Ile Pro His Xaa Phe Gly Xaa Xaa Xaa Pro Pro
 1               5                  10                  15
```

<210> 19
<211> 17
<212> PRT
<213> Künstliche Sequenz

<220>
<223> Beschreibung der künstlichen Sequenz: peptide

<400> 19

```
Lys Xaa Xaa Xaa Leu Xaa Xaa Leu Xaa Asp Ile Glu Xaa Ala Xaa Xaa
 1               5                  10                  15

Leu
```

<210> 20
<211> 11
<212> PRT
<213> Künstliche Sequenz

<220>
<223> Beschreibung der künstlichen Sequenz: peptide

<400> 20

Gly Xaa Xaa Xaa Leu Xaa Glu Val Ala Leu Gly
1 5 10

<210> 21
<211> 20
<212> PRT
<213> Künstliche Sequenz

<220>
<223> Beschreibung der künstlichen Sequenz: peptide

<220>
<221> SITE
<222> (14)
<223> Xaa is 7 to 9 other amino acids

<400> 21

Gly Xaa Xaa Ser Xaa Xaa Xaa Xaa Gly Xaa Xaa Xaa Pro Xaa Leu Xaa
1 5 10 15

Gly Xaa Xaa Val
20

<210> 22
<211> 16
<212> PRT
<213> Künstliche Sequenz

<220>
<223> Beschreibung der künstlichen Sequenz: peptide

<220>
<221> SITE
<222> (2)
<223> Xaa is Tyr or Phe

<400> 22

Glu Xaa Xaa Xaa Tyr Xaa Xaa Xaa Gln Xaa Xaa Xaa Xaa Tyr Leu Leu
1 5 10 15

<210> 23
<211> 20
<212> PRT
<213> Künstliche Sequenz

<220>
<223> Beschreibung der künstlichen Sequenz: peptide

<400> 23

Met Ala Ala Arg Arg Arg Arg Ser Thr Gly Gly Gly Arg Ala Arg Ala
1 5 10 15

Leu Asn Glu Ser
20

<210> 24
<211> 20
<212> PRT
<213> Künstliche Sequenz

<220>
<223> Beschreibung der künstlichen Sequenz: peptide

<400> 24

Lys Thr Glu Leu Gln Ser Pro Glu His Pro Leu Asp Gln His Tyr Arg
1 5 10 15

Asn Leu His Cys
20

<210> 25
<211> 21
<212> PRT
<213> Künstliche Sequenz

<220>
<223> Beschreibung der künstlichen Sequenz: peptide

<400> 25

Cys Lys Gly Arg Gln Ala Gly Arg Glu Glu Asp Pro Phe Arg Ser Thr
1 5 10 15

Ala Glu Ala Leu Lys
20

<210> 26
<211> 20
<212> PRT
<213> Künstliche Sequenz

<220>
<223> Beschreibung der künstlichen Sequenz: peptide

<400> 26

Cys Lys Gln Gln Ile Ala Arg Gly Phe Glu Ala Leu Glu Ala Leu Glu
1 5 10 15

Glu Ala Leu Lys
20

<210> 27
<211> 19
<212> PRT
<213> Künstliche Sequenz

<220>
<223> Beschreibung der künstlichen Sequenz: peptide

<400> 27

```
Lys Gln Gln Ile Ala Arg Gly Phe Glu Ala Leu Glu Ala Leu Glu Glu
 1               5                  10                  15

Ala Leu Lys
```

<210> 28
<211> 19
<212> PRT
<213> Künstliche Sequenz

<220>
<223> Beschreibung der künstlichen Sequenz: peptide

<400> 28

```
Lys Gln Gln Ile Ala Arg Gly Phe Glu Ala Leu Glu Ala Leu Glu Glu
 1               5                  10                  15

Ala Met Lys
```

## Beschreibung

Die vorliegende Erfindung betrifft neue Poly(ADP-ribose)polymerase (PARP) Gene und die von ihnen abgeleiteten Proteine; Antikörper mit Spezifität für die neuen Proteine; pharmazeutische und gentherapeutische Mittel, welche erfindungsgemäße Produkte enthalten; Verfahren zur analytischen Bestimmung der erfindungsgemäßen Proteine und Nukleinsäuren; Verfahren zur Identifizierung von Effektoren oder Bindungspartnern der erfindungsgemäßen Proteine; Verfahren zur Bestimmung der Wirksamkeit solcher Effektoren sowie deren Anwendung zur Diagnose oder Therapie von Krankheitszuständen.

1966 entdeckten Chambon und Mitarbeiter ein 116 kD Enzym, das in den darauffolgenden Jahren näher charakterisiert wurde und heute die Namen PARP (EC 2.4.2.30) (Poly(adenosin-5'-diphospho-ribose) polymerase), PARS (Poly(adenosin-5'-diphospho-ribose) synthetase) oder ADPRT (Adenosin-5'-diphospho-ribose-transferase) trägt. Im Pflanzenreich (Arabidopsis thaliana) wurde 1995 ein 72 kD (637 Aminosäuren) PARP gefunden (Lepiniec, L. et al., FEBS Lett. 1995:364(2):103-8). Es war nicht klar, ob es sich bei dieser kürzeren Form von PARP um eine pflanzenspezifische Besonderheit oder ein Artefakt ("Splice"-Variante o. ä.) handelt. Bis dato war das 116 kD PARP-Enzym mit seiner infolge beschriebenen Aktivität im Tierreich und im Menschen einzigartig. Der Eindeutigkeit halber wird es im Folgenden mit PARP1 bezeichnet.

Die primäre, physiologische Funktion von PARP1 scheint in seiner Beteiligung an einem komplexen Reparaturmechanismus zu bestehen, den Zellen zur Reparatur von DNA-Strangbrüchen entwickelt haben. Die primäre zelluläre Reaktion auf einen DNA-Strangbruch scheint dabei in der PARP1-katalysierten Synthese von Poly(ADP-ribose) aus $NAD^+$ zu bestehen (vgl. De Murcia, G. et al. (1994) TIBS, 19, 172).

PARP1 besitzt eine modular aufgebaute Molekülstruktur. Drei funktionale Hauptelemente wurden bisher identifiziert: eine N-terminale 46 kD große DNA-Bindungsdomäne; eine zentrale 22 kD Automodifikationsdomäne, an welche Poly(ADP-ribose) angehängt wird, wobei die Enzymaktivität von PARP1 mit zunehmender Elongation abnimmt; und eine C-terminale 54 kD große $NAD^+$-Bindungsdomäne. Lediglich in PARP aus Drosophila wurde eine Leucin-Zipper-Region innerhalb der Automodifikationsdomäne festgestellt, welche auf mögliche Protein-Protein-Interaktionen hinweist. Alle bisher bekannten PARPs sind vermutlich als Homodimere aktiv.

Der hohe Organisationsgrad des Moleküls spiegelt sich wider in der starken Konservierung der Aminosäuresequenz. So wurde für PARP1 aus Mensch, Maus, Rind und Huhn eine 62%-ige Konservierung der Aminosäuresequenz festgestellt. Größere strukturelle Unterschiede bestehen zu PARP aus Drosophila. Die einzelnen Domänen selbst weisen wiederum Cluster mit erhöhter Konservierung auf. So enthält die DNA-Bindungsregion zwei sogenannte Zink-Finger als Unterdomänen (umfassend Motive des Typs $CX_2CX_{28/30}HX_2C$), die an der $Zn^{2+}$-abhängigen Erkennung von DNA-Einzelstrangbrüchen oder einzelsträngigen DNA-Überhängen (z.B. an den Chromosomenenden, den Telomeren) beteiligt sind. Die C-terminale katalytische Domäne umfaßt einen Block von etwa 50 Aminosäuren (Reste 859-908), der unter Vertebraten zu etwa 100% konserviert ist (PARP-"Signature"). Dieser Block bindet das natürliche Substrat $NAD^+$ und bedingt somit die Synthese von Poly(ADP-ribose) (vgl. de Murcia, a.a.O.). Insbesondere das $GX_3GKG$-Motiv ist für PARPs in diesem Block charakteristisch.

Der oben beschriebenen positiven Funktion steht eine pathologische in zahlreichen Krankheiten (Schlaganfall, Herzinfarkt, Sepsis etc.) gegenüber. PARP ist in den Zelltod infolge von Ischämien des Gehirns (Choi, D.W., (1997) Nature Medicine, 3, 10, 1073), des Myokards (Zingarelli, B., et al (1997), Cardiovascular Research, 36, 205) und auch des Auges (Lam, T.T. (1997), Res. Comm. in Molecular Pathology and Pharmacology, 95, 3, 241) involviert. Auch bei septischem Schock wurde eine durch Entzündungs-Mediatoren ausgelöste PARP-Aktivierung beobachtet (Szabo, C., et al. (1997), Journal of Clinical Investigation, 100, 3, 723). Dabei geht eine Aktivierung von PARP mit einem starken Verbrauch an $NAD^+$ einher. Da für die Biosynthese von einem Mol $NAD^+$ vier Mole ATP verbraucht werden, nimmt die zelluläre Energieversorgung drastisch ab. Zelltod ist die Folge.

Als PARP1-Inhibitoren werden in der oben genannten Fachliteratur Nicotinamid und 3-Aminobenzamid beschrieben. 3,4-Dihydro-5-[4-(1-piperidinyl)butoxy]-1(2H)-isochinolon ist aus Takahashi, K., et al (1997), Journal of Cerebral Blood Flow and Metabolism 17, 1137 bekannt. Weitere Inhibitoren werden z.B. beschrieben in Banasik, M., et al. (1992) J. Biol. Chem., 267, 3, 1569 und Griffin, R.J., et al. (1995), Anti-Cancer Drug Design, 10, 507.

Als hochmolekulare Bindungspartner für humanes PARP1 ist unter anderem das Base Excision Repair (BER) Protein XRCC1 (X-ray repair cross-complementing 1) beschrieben worden, das über ein Zink-Finger-Motiv und ein BRCT (BRCA1 C-Terminus) Modul (Aminosäuren 372-524) bindet (Masson, M., et al., (1998) Molecular and

Cellular Biology, 18,6, 3563).

Lepiniec, L. et al. beschreiben in FEBS Letters 364 (1995)103-108 ein PARP-Homologes aus *Arabidopsis thaliana,* deren katalytische Domäne große Ähnlichkeit mit Vertebraten-PARP aufweist.

Aufgrund der vielfältigen physiologischen und pathologischen Funktionen von PARP stellte sich die Aufgabe, neue PARP-Homologe bereitzustellen. Die Bereitstellung von homologen PARP's wäre nämlich von besonderer Bedeutung für die Entwicklung neuer Wirkstofftargets bzw. neuer Wirkstoffe, um Diagnose und/oder Therapie von Krankheitszuständen zu verbessern, in welche PARP, PARP-Homologe oder davon abgeleitete Substanzen involviert sind.

Diese Aufgabe wurde überraschenderweise gelöst durch Bereitstellung von PARP-Homologen vorzugsweise aus menschlichen oder nichtmenschlichen Säugern, und funktionalen Äquivalenten davon, gekennzeichnet durch eine Aminosäuresequenz, die

a) eine funktionale $NAD^+$-Bindungsdomäne, d. h. eine PARP-"Signature"-Sequenz mit dem charakteristischen $GX_3GKG$-Motiv; und
b) insbesondere im N-terminalen Sequenzbereich, d.h. im Bereich der ersten 200, wie z.B. im Bereich der ersten 100, N-terminalen Aminosäuren, keine PARP-Zink-Finger-Sequenzmotive der allgemeinen Formel

$$CX_2CX_mHX_2C$$

aufweist, worin
m für einen ganzzahligen Wert von 28 oder 30 steht und die Reste X unanbhängig voneinander für eine beliebige Aminosäure stehen; und wobei die funktionale $NAD^+$-Bindungsdomäne das in Anspruch 1 definierte Sequenzmotiv aufweist.

Da die erfindungsgemäßen PARP-Moleküle insbesondere funktionale Homologe darstellen, besitzen sie natürlich außerdem eine Poly-(ADP-ribose)-synthetisierende Aktivität. Die NAD-Bindungsdomäne entspricht im Wesentlichen dieser Aktivität und ist C-terminal lokalisiert.
wesentliches Kennzeichen der erfindungsgemäßen PARPs ist somit das Vorhandensein einer funktionalen $NAD^+$-Bindungsdomäne (PARP-Signature), welche im C-terminalen Bereich der Aminosäuresequenz (d.h. etwa im Bereich der letzten 400, wie z.B. der letzten 350 oder 300, C-terminalen Aminosäuren) liegt, in Kombination mit einer keine Zink-Finger-Motive aufweisenden N-terminalen Sequenz. Da die Zink-Finger-Motive in bekannten PARPs vermutlich zur Erkennung der DNA-Bruchstellen beitragen, ist anzunehmen, daß die erfindungsgemäßen Proteine mit DNA nicht oder in anderer weise wechselwirken. Mit entsprechenden biochemischen Tests konnte nachgewiesen werden, daß das erfindungsgemäße PARP2 durch "aktivierte DNA" (d.h. limitiert DNaseI verdaute DNA) aktiviert werden kann. Daraus kann ferner geschlossen werden, daß das erfindungsgemäße PARP2 DNA bindende Eigenschaften hat. Der Mechanismus der DNA-Bindung und Enzym-Aktivierung bei den erfindungsgemäßen PARPs ist jedoch unterschiedlich zu dem von PARP1. Dessen DNA-Bindung und Enzym-Aktivierung wird wie erwähnt durch ein charakteristisches Zink-Finger-Motiv vermittelt. Derartige Motive sind in den erfindungsgemäßen PARPs nicht vorhanden. Vermutlich vermitteln positiv-geladene Aminosäuren im N-terminalen Bereich der erfindungsgemäßen PARPs diese Eigenschaften. Da die "aktivierte DNA" (d.h. zum Beispiel DNA, die limitiert mit DNaseI behandelt wurde) eine Vielzahl von Defekten aufweist (Einzelstrangbrüche, Einzelstranglücken, Einzelstrangüberhänge, Doppelstrangbrüche etc.) ist es möglich, daß PARP1 und die erfindungsgemäßen PARPs zwar durch dieselbe "aktivierte DNA" aktiviert werden, jedoch durch eine andere Subpopulation von Defekten (z. B. Einzelstranglücken statt Einzelstrangbrüche).
Die funktionale $NAD^+$-Bindungsdomäne (d.h. katalytische Domäne) bindet das Substrat für die Synthese von Poly-(ADP-ribose). In Übereinstimmung mit bekannten PARPs ist insbesondere das Sequenzmotiv $GX^1X^2X^3GRG$, worin G für Glycin steht, K für Lysin steht und $X^1$, $X^2$ und $X^3$ unanbhängig voneinander für eine beliebige Aminosäure stehen, zu finden. Wie jedoch ein Vergleich der Aminosäuresequenzen der $NAD^+$-Bindungsdomänen erfindungsgemäßer PARP-Moleküle mit bisher bekanntem humanem PARP1 überraschenderweise zeigt, weichen die erfindungsgemäßen Sequenzen von der bekannten Sequenz für die $NAD^+$-Bindungsdomäne deutlich ab.
Einer erfindungsgemäß bevorzugten Gruppe von PARP-Molekülen ist folgendes allgemeines Sequenzmotiv in der katalytischen Domäne vorzugsweise gemeinsam:

$PX_n(S/T)GX_3GKGIYFA$ (SEQ ID NO:11), insbesondere
$(S/T)XGLR(I/V)XPX_n(S/T)GX_3GKGIYFA$ (SEQ ID NO:12), vorzugsweise
$LLWHG(S/T)X_7IL(S/T)XGLR(I/V)XPX_n(S/T)GX_3GKGIYFAX_3SKSAXY$ (SEQ ID NO:13)

worin (S/T) die alternative Besetzung dieser Sequenzposition mit S oder T beschreibt, (I/V) die alternative Besetzung dieser Sequenzposition mit I oder V beschreibt und n für einen ganzzahligen Wert von 1 bis 5 steht und die Reste X unabhängig voneinander für eine beliebige Aminosäure stehen. Letztes Motiv wird auch als "PARP-Signature" Motiv bezeichnet.
Die Automodifikationsdomäne ist in den erfindungsgemäßen PARPs vorzugsweise ebenfalls ausgebildet. Sie kann im Bereich von etwa 100 bis 200 Aminosäuren vor dem N-terminalen Ende der $NAD^+$-Bindungsdomäne liegen.
Erfindungsgemäße PARP-Homologe können außerdem N-terminal zur $NAD^+$-Bindungsdomäne (d.h. etwa 30 bis etwa 80 Aminosäuren näher am N-Terminus) ein Leucin-Zipper-artiges Sequenzmotiv der allgemeinen Formel

$$(L/V)X_6LX_6LX_6L \text{ (SEQ ID NO:14)}$$

umfassen,
worin (L/V) für die alternative Besetzung dieser Sequenzposition mit L oder V steht und die Reste X unabhängig voneinander für eine beliebige Aminosäure stehen. Die erfindungsgemäß beobachteten Leucin-Zipper-Motive unterscheiden sich hinsichtlich ihrer Lage deutlich von den für PARP aus Drosophila beschriebenen. Leucin-Zipper können zu Homodimeren (zwei PARP-Moleküle) oder Heterodimeren (ein PARP-Molekül mit einem davon verschiedenen Bindungspartner) führen.

Die erfindungsgemäßen PARP-Homologen umfassen vorzugsweise außerdem N-terminal zum oben genannten Leucin-Zipper-artigen Sequenzmotiv, d.h. etwa 10 bis 250 Aminosäurereste näher am N-Terminus, wenigstens ein weiteres der folgenden Teilsequenz-Motive:

| | |
|---|---|
| $LX_9NX_2YX_2QLLX(D/E)X_bWGRVG$, | (Motiv 1; SEQ ID NO:15) |
| $AX_3FXKX_4KTXNXWX_sFX_3PXK$, | (Motiv 2; SEQ ID NO:16) |
| $QXL(I/L)X_2IX_9MX_{10}PLGKLX_3QIX_6L$, | (Motiv 3; SEQ ID NO:17) |
| $FYTXIPHXFGX_3PP$, | (Motiv 4; SEQ ID NO:18) |
| und | |
| $KX_3LX_2LXDIEXAX_2L$ | (Motiv 5; SEQ ID NO:19), |

worin (D/E) die alternative Besetzung dieser Sequenzposition mit D oder E beschreibt, (I/L) die alternative Besetzung dieser Sequenzposition mit I oder L beschreibt, b für den ganzzahligen Wert 10 oder 11 steht und die Reste X unabhängig voneinander für eine beliebige Aminosäure stehen. Am meisten bevorzugt sind diese Motive 1 bis 5 in der genannten Reihenfolge gleichzeitig vorhanden, wobei Motiv 1 dem N-Terminus am nächsten liegt.

Auf das oben genannte PARP-Signature Motiv folgt in den erfindungsgemäßen Proteinen wenigstens ein weiteres der folgenden Motive:

| | |
|---|---|
| $GX_3LXEVALG$ | (Motiv 6; SEQ ID NO:20) |
| $GX_2SX_4GX_3PX_aLXGX_2V$ | (Motiv 7; SEQ ID NO:21) und |
| $E(Y/F)X_2YX_3QX_4YLL$ | (Motiv 8; SEQ ID NO:22) |

worin (Y/F) die alternative Besetzung dieser Sequenzposition mit Y oder F beschreibt, a gleich 7 bis 9 und X jeweils für eine beliebige Aminosäure steht. Am bevorzugtesten sind die drei C-terminalen Motive gleichzeitig und in der genannten Reihenfolge ausgebildet, wobei Motiv 8 dem C-Terminus am nächsten liegt.

Ein bevorzugter Aufbau einer erfindungsgemäßen PARP-Struktur kann schematisch wie folgt beschrieben werden:

Motive 1 bis 5/PARP-Signature/Motive 6 bis 8 oder Motive 1 bis 5/Leucin-Zipper/PARP-Signature/Motive 6 bis 8

wobei zwischen den Einzelmotiven weiterer Aminosäurereste, wie z.B. bis zu 40, und am N-Terminus und/oder am C-Terminus weitere Aminosäurereste, wie z.B. bis zu 80, angeordnet sein können.

Erfindungsgemäß besonders bevorzugte PARP-Homologe sind die Proteine humanPARP2, humanPARP3, mausPARP3 und die funktionalen Äquivalente davon. Das als humanPARP2 bezeichnete Protein umfaßt 570 Aminosäuren (vgl. SEQ ID NO:2). Das als humanPARP3 bezeichnete Protein existiert möglicherweise in zwei Formen. Typ 1 umfaßt dabei 533 Aminosäuren (SEQ ID NO:4) und Typ 2 umfaßt 540 Aminosäuren (SEQ ID NO:6). Die Formen können sich durch unterschiedliche Initiation der Translation ergeben. Das als mausPARP3 bezeichnete Protein existiert in zwei Formen, die sich durch eine Deletion von 5 Aminosäuren (15 bp) voneinander unterscheiden. Typ 1 umfaßt dabei 533 Aminosäuren (SEQ ID NO:8), Typ 2 umfaßt 528 Aminosäuren (SEQ ID NO:10). Die erfindungsgemäßen PARP-Homologen unterscheiden sich in ihren Sequenzen deutlich von dem aus Arabidopsis thaliana (a.a.O.) bekannten PARP-Protein. So weisen PARP2 und PARP3 z. B. nicht die Pflanzen-PARP charakteristische Peptidsequenz AAVLDQWIPD, entsprechend den Aminosäureresten 143 bis 152 des Arabidopsis-Proteins, auf.

Ein weiterer Gegenstand der Erfindung betrifft Bindungspartner für die erfindungsgemäßen PARP-Homologen. Diese Bindungspartner sind vorzugsweise ausgewählt unter

a) Antikörpern und Fragmenten, wie z.B. Fv, Fab, $F(ab)'_2$, davon
b) proteinartigen Verbindungen, welche, z.B. über die obige Leucin-Zipper-Region oder einen anderen Sequenzabschnitt, mit PARP wechselwirken, und

c) niedermolekularen Effektoren, welche eine biologische PARP-Funktion, wie z.B die katalytische PARP-Aktivität, d.h. die $NAD^+$-verbrauchende ADP-Ribosylierung, oder die Bindung an ein Aktivatorprotein oder an DNA modulieren.

Ein weiterer Gegenstand der Erfindung betrifft Nukleinsäuren, umfassend

a) eine, für wenigstens ein erfindungsgemäßes PARP-Homologes kodierende, Nukleotidsequenz oder die komplementäre Nukleotidsequenz davon;
b) eine Nukleotidsequenz, die, vorzugsweise unter stringenten Bedingungen mit einer Sequenz gemäß a) hybridisiert; oder
c) Nukleotidsequenzen, die durch Entartung (Degeneration) des genetischen Codes von den in a) und b) definierten Nukleotidsequenzen abgeleitet sind.

Insbesondere umfassen erfindungsgemäß geeignete Nukleinsäuren wenigstens eine der Teilsequenzen welche für die oben genannten Aminosäuresequenzmotive kodieren.

Erfindungsgemäß bevorzugte Nukleinsäuren umfassen Nukleotidsequenzen gemäß SEQ ID NO: 1 und 3, und insbesondere für erfindungsgemäße PARP-Homologe charakteristische Teilsequenzen daraus, wie z.B. Nukleotidsequenzen, umfassend

a) die Nukleotide +3 bis + 1715 gemäß SEQ ID NO:1;
b) die Nukleotide +242 bis + 1843 gemäß SEQ ID NO:3;
c) die Nukleotide +221 bis + 1843 gemäß SEQ ID NO:5;
d) die Nukleotide +112 bis +1710 gemäß SEQ ID NO:7; oder
e) die Nukleotide + 1 bis + 1584 gemäß SEQ ID NO:9

oder Teilsequenzen von a), b), c), d) und e), welche für oben genannte charakteristische Aminosäuresequenzmotive der erfindungsgemäßen PARP-Homologen kodieren.

Ein weiterer Gegenstand der Erfindung umfaßt Expressionskassetten, welche unter genetischer Kontrolle regulativer Nukleotidsequenzen wenigstens eine der oben beschriebenen erfindungsgemäßen Nukleotidsequenzen enthalten. Diese sind zur Herstellung erfindungsgemäßer rekombinanter Vektoren, wie z.B. von viralen Vektoren oder Plasmiden brauchbar; welche wenigstens eine erfindungsgemäße Expressionskassette enthalten.

Erfindungsgemäße rekombinante Mikroorganismen sind mit wenigstens einem der oben genannten Vektoren transformiert.

Ein weiterer Gegenstand der Erfindung betrifft ein, homogen oder heterogen durchführbares, in vitro Nachweisverfahren für PARP-Inhibitoren, das dadurch gekennzeichnet, daß man

a) ein ungeträgertes oder geträgertes Poly-ADP-ribosylierbares Target mit einem Reaktionsgemisch inkubiert, umfassend

  a1) ein erfindungsgemäßes PARP-Homologes;
  a2) einen PARP-Aktivator; und
  a3) einen PARP-Inhibitor oder einen Analyten, in welchem man wenigstens einen PARP-Inhibitor vermutet;

b) die Poly-ADP-Ribosylierungsreaktion durchführt; und
c) die Poly-ADP-Ribosylierung des Target qualitativ oder quantitativ bestimmt.

Vorzugsweise wird das Nachweisverfahren so durch geführt, dass man das PARP-Homologe mit dem PARP-Aktivator und dem PARP-Inhibitor oder einen Analyten, in welchem man wenigstens einen PARP-Inhibitor vermutet, vorinkubiert, wie z.B. etwa 1 bis 30 Minuten, bevor man die Poly-ADP-Ribosylierungsreaktion durchführt.

Nach Aktivierung durch DNS mit Einzelstrangbrüchen (erfindungsgemäß bezeichnet als "aktivierte DNS") poly-ADP-ribosyliert PARP eine Vielzahl nukleärer Proteine in Gegenwart von NAD. Zu diesen Proteinen zählt zum einen PARP selber, aber auch Histone etc.

Das bei den Nachweisverfahren vorzugsweise verwendete Poly-ADP-ribosylierbare Target ist ein Histon-Protein in seiner nativen Form oder ein davon abgeleitetes Poly-ADP-ribosylierbares Äquivalent. Beispielhaft wurde eine Histonpräparation der Firma Sigma verwendet (SIGMA, Katalog-Nr. H-7755; Histone Typ II-AS aus Kalbsthymus, Luck, J. M., et al., J. Biol. Chem., 233, 1407 (1958), Satake K., et al., J. Biol. Chem, 235, 2801 (1960)). Im Prinzip können alle Arten von Proteinen oder Teile von diesen verwendet werden, die einer Poly-ADP-ribosylierung durch PARP zugänglich sind. Dies sind bevorzugterweise nukläre Proteine, z. B. Histone, DNA-Polymerase, Telomerase oder PARP selber.

Auch synthetische Peptide, die von den entsprechenden Proteinen abgeleitet sind, können als Target fungieren.

Es können im erfindungsgemäßen ELISA Histonmengen im Bereich von etwa 0,1 µg/well bis etwa 100 µg/well, vorzugsweise etwa 1 µg/well bis etwa 10 µg/well, verwendet werden. Die PARP-Enzymmengen liegen in einem Bereich von etwa 0,2 pMol/well bis etwa 2 nMol/well, vorzugsweise von etwa 2 pMol/well bis etwa 200 pMol/well, wobei der Reaktionsansatz jeweils 100 µl/well ausmacht. Reduzierungen auf kleinere Wells und entsprechend kleinere Reaktionsvolumina sind möglich.

Beim erfindungsgemäßen HTRF Assay werden identische PARP-Mengen eingesetzt, die Menge an Histon oder modifizierten Histonen liegt im Bereich von etwa 2 ng/well bis etwa 25 µg/well, vorzugsweise etwa 25 ng/well bis etwa 2,5 µg/well, wobei der Reaktionsansatz jeweils 50 µl/well ausmacht. Reduzierungen auf kleinere Wells und entsprechend kleinere Reaktionsvolumina sind möglich.

Der erfindungsgemäß verwendete PARP-Aktivator ist vorzugsweise aktivierte DNA.

Als Aktivator können diverse Typen geschädigter DNA fungieren. DNA-Schädigungen können durch Verdau mit DNasen oder anderen DNAmodifizierenden Enzymen (z. B. Restriktionsendonukleasen), durch Bestrahlung oder andere physikalische Methoden oder chemische Behandlung der DNA erzielt werden. Ferner ist es möglich, mittels synthetischer Oligonukleotide die Situation einer DNA-Schädigung gezielt zu simulieren. In den beispielhaft angegebenen Assays wurde aktivierte DNA aus Kalbsthymus eingesetzt (SIGMA, Produkt-Nr. D4522, CAS: 91080-16-9, hergestellt nach der Methode von Aposhian und Kornberg unter Verwendung von Kalbsthymus-DNA (SIGMA D-1501) und Deoxyribonuklease Typ I (D-4263). Aposhian H. V. und Kornberg A., J. Biol. Chem., 237, 519 (1962)). Verwendet wurde die aktivierte DNA in einem Konzentrationsbereich von 0,1 bis 1000 µg/ml, vorzugsweise von 1 bis 100 µg/ml im Reaktionsschritt.

Die Poly-ADP-Ribosylierungsreaktion wird in den erfindungsgemäßen Verfahren durch Zugabe von $NAD^+$ gestartet. Die Konzentrationen von NAD lagen in einem Bereich von etwa 0,1 µM bis etwa 10 mM, bevorzugt in einem Bereich von etwa 10 µM bis etwa 1 mM.

Gemäß der heterogen durchführbaren Variante obigen Verfahrens wird die Poly-ADP-Ribosylierung des geträgerten Target mit Anti-Poly-(ADP-ribose)-Antikörper bestimmt. Dazu trennt man das Reaktionsgemisch vom geträgerten Target ab, wäscht und inkubiert mit dem Antikörper. Dieser Antikörper kann selbst markiert sein. Alternativ verwendet man zum Nachweis von gebundenem Anti-Poly-(ADP-ribose)-Antikörper einen markierten sekundären Antikörper, oder ein entsprechendes markiertes Antikörperfragment. Geeignete Markierungen sind z.B. Radiomarkierung, Chromophor- oder Fluorophormarkierung, Biotinylierung, Chemilumineszenzmarkierung, Markierung mit paramagnetischem Metall, oder insbesondere Enzymmarkierungen, wie z.B. mit Meerrettich-Peroxidase. Entsprechende Nachweistechniken sind dem Fachmann allgemein bekannt.

Gemäß der homogen durchführbaren Variante obigen Verfahrens ist das nichtgeträgerte Target mit einem Akzeptor-Fluorophor markiert. Bevorzugt verwendet man dazu als Target biotinyliertes Histon, wobei das Akzeptor-Fluorophor über Avidin oder Streptavidin an die Biotingruppen des Histons gekoppelt ist. Als Akzeptor-Fluorophor sind insbesondere geeignet Phycobiliproteine (z. B. Phycocyanine, Phycoerythrine), z. B. R-Phycocyanin (R-PC), Allophycocyanin (APC), R-Phycoerythrin (R-PE), C-Phycocyanin (C-PC), B-Phycoerythrin (B-PE) oder ihre Kombinationen untereinander oder mit Fluoreszenzfarbstoffen, wie Cy5, Cy7 oder Texas Red (Tandem system) (Thammapalerd, N. et al., Southeast Asian Journal of Tropical Medicine & Public Health, 27(2): 297-303 (1996); Kronick, M. N. et al., Clinical Chemistry, 29(9), 1582-1586 (1986); Hicks, J. M., Human Pathology, 15(2), 112-116 (1984)). Bei dem in den Beispielen verwendeten Farbstoff XL665 handelt es sich um ein quervernetztes Allophycocyanin (Glazer, A. N., Rev. Microbiol., 36, 173-198 (1982); Kronick, M. N., J. Imm. Meth., 92, 1-13 (1986); MacColl, R. et al., Phycobiliproteins, CRC Press, Inc., Boca Raton, Florida (1987); MacColl, R. et al., Arch. Biochem. Biophys., 208(1), 42-48 (1981)).

Außerdem ist bevorzugt, bei dem homogenen Verfahren die Poly-ADP-Ribosylierung des nicht geträgerten Target mit Anti-Poly-(ADP-ribose)-Antikörper zu bestimmen, der mit einem Donor-Fluorophor markiert ist, das zur Energieübertragung auf das Akzeptor-Fluorophor befähigt ist, wenn Donor und Akzeptor durch Bindung des markierten Antikörpers an das Poly-ADP-ribosylierte Histon in räumliche Nähe gelangen. Vorzugsweise verwendte man ein ein Europium-Kryptat als Donor-Fluorophor für den Anti-Poly-(ADP-ribose)-Antikörper.

Neben dem verwendeten Europium-Kryptat können auch weitere Verbindungen als potentielle Donor-Moleküle auftreten. Hierbei kann zum einen der Kryptatkäfig modifiziert werden. Auch Austausch des Europiums gegen andere Seltenerdmetalle, wie z. B. Terbium, ist denkbar. Entscheidend ist eine lange Lebensdauer der Fluoreszenz, die die Zeitverzögerung garantiert (Lopez, E. et al., Clin. Chem. 39/2, 196-201 (1993); US-Patent 5,534,622).

Die oben beschriebenen Nachweisverfahren basieren auf dem Prinzip, daß die Aktivität von PARP mit der Menge der an den Histonen gebildeten ADP-ribose-Polymeren korreliert. Der hier beschriebene Assay ermöglicht die Quantifizierung der ADP-ribose Polymere mittels spezifischer Antikörper in Form eines ELISA- und eines HTRF-(homogenous time-resolved fluorescence; homogene zeit-aufgelöste Fluoreszenz) Assays. Konkrete Ausführungsformen dieser beiden Tests sind in den folgenden Ausführungsbeispielen näher beschrieben.

Das entwickelte HTRF (Homogenous Time-Resolved Fluorescence)-Testsystem mißt die Bildung von Poly-(ADP-Ribose) an Histonen mittels spezifischer Antikörper. Im Unterschied zum ELISA wird dieser Test in homogener Phase ohne Separations- und Waschschritte durchgeführt. Dies ermöglicht einen höheren Probendurchsatz und eine geringere Fehleranfälligkeit. HTRF basiert auf dem "Fluoresenz Resonanz Energie Transfer" (FRET) zwischen zwei Fluorophoren. In einem FRET Assay kann ein angeregtes Donor-Fluorophor seine Energie auf ein Akzeptor-Fluorophor übertragen, wenn sich die beiden in einer räumlichen Nähe befinden. In der HTRF-Technologie ist das Donor-Fluorophor ein Europium-Kryptat [(Eu)K] und der Akzeptor ist XL665, ein stabilisiertes Allophycocyanin. Das Europium-Kryptat basiert auf Arbeiten von Jean Marie Lehn (Strasbourg) (Lopez, E. et al., Clin. Chem. 39/2, 196-201 (1993); US-Patent 5,534,622).

In einem homogenen Assay sind alle Komponenten auch während der Messung anwesend. Während dies Vorteile bei der Assaydurchführung bringt (Schnelligkeit, Aufwand), müssen Störungen durch Assaykomponenten (Eigenfluoreszenz, Quenching durch Farbstoffe etc.) ausgeschlossen werden. HTRF schließt diese Störungen durch eine zeitverzögerte Messung bei zwei Wellenlängen (665nm, 620nm) aus. Die HTRF hat eine sehr lange Abklingzeit und kann daher zeitverzögert gemessen werden. Jegliche interferierende, kurzlebige Hintergrundfluoreszenz (z. B. durch Assaykomponenten oder Inhibitoren der Substanzbank) stört hier nicht mehr. Darüberhinaus wird permanent bei zwei Wellenlängen gemessen, um "Quench-Effekte" farbiger Substanzen zu kompensieren. HTRF Assays sind z.B. im 96-or 384-well Mikrotiterplattenformat realisierbar und werden mit einem "Discovery HTRF Microplate Analyzer" (Canberra Packard) ausgewertet.

Erfindungsgemäß werden außerdem die folgenden in vitro-Screeningverfahren auf Bindungspartner für PARP, insbesondere für ein erfindungsgemäßes PARP-Homologes, bereitgestellt.

Eine erste Variante wird so durchgeführt, daß man

a1) wenigstens ein PARP-Homologes an einem Träger immobilisiert;
b1) das immobilisierte PARP-Homologe mit einem Analyten in Kontakt bringt, in welchem man wenigstens einen Bindungspartner vermutet; und
c1) an das immobilisierte PARP-Homologe gebundene Bestandteile des Analyten, gegebenenfalls nach einer Inkubationsphase, bestimmt.

Gemäß einer zweiten Variante wird

a2) ein Analyt, welcher wenigstens einen möglichen Bindungspartner für das PARP-Homologe enthält, an einem Träger immobilisiert;
b2) der immobilisierte Analyt mit wenigsten einem PARP-Homologen in Kontakt gebracht, für welches man einen Bindungspartner sucht; und
c3) der immobilisierte Analyt wird, gegebenenfalls nach einer Inkubationsphase, auf die Bindung des PARP-Homologen untersucht.

Gegenstand der Erfindung ist auch ein Verfahren zur qualitativen oder quantitativen Bestimmung einer PARP-Homologe kodierenden Nukleinsäure, gekennzeichnet durch

a) Inkubation einer biologischen Probe mit einer definierten Menge einer exogenen erfindungsgemäßen Nukleinsäure (z.B. mit einer Länge von etwa 20 bis 500 Basen oder länger), Hybridisierung, vorzugsweise unter stringenten Bedingungen, Bestimmung der hybridisierenden Nukleinsäuren und gegebenenfalls Vergleich mit einem Standard; oder

b) Inkubation einer biologischen Probe mit einer definierten Menge von Oligonucleotid-Primerpaaren mit Spezifität für ein PARP-Homologes kodierende Nukleinsäure, Amplifizierung der Nukleinsäure, Bestimmung des Amplifikationsprodukts und gegebenenfalls Vergleich mit einem Standard.

Ein weiterer Gegenstand der Erfindung ist ein Verfahren zur qualitativen oder quantitativen Bestimmung eines erfindungsgemäßen PARP-Homologen, gekennzeichnet durch

a) Inkubation einer biologischen Probe mit wenigstens einem für ein PARP-Homologes spezifischen Bindungspartner,
b) Nachweis des Bindungspartner/PARP-Komplexes und gegebenenfalls
c) Vergleich des Ergebnisses mit einem Standard.

Vorzugsweise ist hierbei der Bindungspartner ein Anti-PARP-Antikörper oder ein bindendes Fragment davon, der gegebenenfalls eine detektierbare Markierung trägt.

Die erfindungsgemäßen Bestimmungsverfahren für PARP, insbesondere für PARP-Homologe und für die kodierenden Nukleinsäuresequenzen davon eignen sich vorteilhafterweise zur Diagnostizierung von Sepsis- oder Ischämie-bedingten Gewebeschädigungen, insbesondere von Schlaganfällen, Myokard-Infarkten, Diabetes oder septischen Schocks.

Weiterhin umfaßt die Erfindung ein Verfahren zur Bestimmung der Wirksamkeit vom PARP-Effektoren, das dadurch gekennzeichnet ist, daß man

a) ein erfindungsgemäßes PARP-Homologes mit einem Analyten inkubiert, welcher einen Effektor einer physiologischen oder pathologischen PARP-Aktivität enthält; den Effektor gegebenenfalls wieder abtrennt; und
b) die Aktivität des PARP-Homologen, gegebenenfalls nach Zugabe von Substraten oder Cosubstraten, bestimmt.

Ein weiterer Gegenstand der Erfindung betrifft gentherapeutisches Mittel, die in einem gentherapeutisch akzeptablen Träger ein Nukleinsäurekonstrukt enthalten, das

a) eine Antisense-Nukleinsäure gegen eine kodierende Nukleinsäure gemäß der Erfindung; oder
b) ein Ribozym gegen eine nicht-kodierende erfindungsgemäße Nukleinsäure umfasst; oder
c) für einen spezifischen PARP-Inhibitor kodiert.

Weiterhin betrifft die Erfindung pharmazeutische Mittel, enthaltend in einem pharmazeutisch akzeptablen Träger wenigstens ein erfindungsgemäßes PARP-Protein, wenigstens einen erfindungsgemä-βen PARP-Bindungspartner oder wenigstens eine erfindungsgemäße kodierende Nukleotidsequenz.

Schließlich betrifft die Erfindung die Verwendung von Bindungspartnern eines PARP-Homologen zur Diagnose oder Therapie von Krankheitszuständen, an deren Entstehung und/oder Verlauf wenistens ein PARP-Protein, insbesondere ein erfindungsgemäßes PARP-Homologes, oder ein davon abgeleitetes Polypeptid beteiligt ist. Der verwendete Bindungspartner kann beispielsweise ein niedermolekularer Bindungspartner sein, dessen Molekulargewicht z. B. kleiner als etwa 2000 Dalton oder kleiner als etwa 1000 Dalton sein kann.

Gegenstand der Erfindung ist außerdem die Verwendung von PARP-Bindungspartnern zur Diagnose oder Therapie von Krankheitszuständen, die durch eine Energiedefizienz vermittelt werden. Eine Energiedefizienz im Sinne vorliegender Erfindung ist insbesondere eine zelluläre Energiedefizienz, welche bei dem erkrankten Patienten systemisch oder in einzelnen Körperbereichen, Oganen oder Organbereichen, oder Geweben oder Gewebebereichen zu beobachten ist. Diese ist durch eine über den physiologischen Schwankungsbereich des NAD- und/oder ATP-Spiegels nach oben oder unter hinausgehende NAD- und/oder ATP-Depletion gekennzeichnet, welche vorzugsweise durch ein Protein mit PARP-Aktivität, insbesondere ein erfindungsgemäßes PARP-Homologes, oder ein davon abgeleitetes Polypeptid, vermittelt wird.

"Ernergiedefizienz vermittelte Erkrankungen" im Sinne der Erfindung umfassen ferner solche bei denen eine Gewebeschädigung auf Zelltod infolge von Nekrose oder Apoptose zurückzuführen ist. Die erfindungsgemäßen Methoden sind geeignet zur Behandlung und Vorbeugung von Gewebeschädigungen infolge einer Zellschädigung durch Apoptose oder Nekrose; Schädigungen des Nervengewebes durch Ischämien und/oder Reperfusion; neurologischen Erkrankungen; neurodegenrativen Erkrankungen; vaskulärem Schlaganfall; zur Behandlung und Vorbeugung kardiovaskulärer Erkrankungen; zur Behandlung anderer Erkrankungen oder Zustände, wie zum Beispiel altersbedingter Makuladegeneration, AIDS oder anderen Immunschwächeerkrankungen; Arthritis; Atherosklerosis; Kachexie; Krebs; degenerativen Erkrankungen der Skelettmuskulatur; Diabetes; Schädeltrauma; entzündlichen Erkrankungen des Magen/Darmtraktes, wie zum Beispiel Crohnsche Erkrankung; Muskeldystrophie; Osteoarthritis; Osteoporose; chronischen und/oder akuten Schmerzen; Nierenversagen; Retinaischämie; septischem Schock (wie zum Beispiel Endotoxin-Schock); Alterung der Haut oder Alterung allgemein; allgemeine Alterungserscheinungen. Die erfindungsgemäßen Methoden sind außerdem einsetzbar zur Verlängerung der Lebenszeit und der proliferativen Kapazität von Körperzellen und zur Sensitisierung von Tumorzellen bei einer Bestrahlungstherapie.

Gegenstand der Erfindung ist insbesondere die Verwendung eines PARP-Bindungspartners gemäß obiger Definition zur Diagnose oder Therapie (akut oder prophylaktisch) von Energiedefizienz-vermittelten Krankheitszuständen, ausgewählt unter neurodegenerativen Erkrankungen, oder Sepsis- oder Ischämie-bedingten Gewebeschädigungen, insbesondere von neurotoxischen Störungen, Schlaganfällen, Myokard-Infarkten, Schädigungen während oder nach der Infarktlyse (z. B. mit TPA, Reteplase oder mechanisch mit Laser oder Rotablator) und von Mikroinfarkten während und nach Herzklappenersatz, Aneurismenresektionen und Herztransplantationen, Kopf- und Rückenmarkstraumen, Infarkten der Niere (akutes Nierenversagen, akute Niereninsuffizienz oder Schädigungen während und nach einer Nierentransplantation), Infarkten der Leber (Leberversagen, Schädigungen während oder nach einer Lebertransplantation), Schädigungen der Skelettmuskulatur, peripheren Neuropathien, AIDS Demenz, septischen Schocks, Diabetes, neurodegenerativen Erkrankungen, die nach Ischämie, Trauma (Schädel-Hirn-Trauma), Massenblutung, Subarachnoidal-Blutungen und Schlaganfall auftreten, sowie neurodegenerativen Erkrankungen, wie Alzheimerkrankheit, multipler Infarkt-Dementia, Huntington-Erkrankung, Parkinsonscher Krankheit, amyotropher lateraler Sklerose, Epilepsie, insbesondere von generalisierten epileptischen Anfällen, wie z. B. Petit mal, und tonisch-clonischen Anfällen und partiell epileptischen Anfällen, wie Temporal Lobe, und komplex-partiellen Anfällen, Nierenversagen, darüber hinaus bei der Chemotherapie von Tumoren und Verhinderung von Metastasierung sowie zur Behandlung von Entzündungen und rheumatischen Erkrankungen, z. B. der rheumatischen Arthritis; ferner bei der Behandlung einer Revaskularisation kritisch verengter Koronararterien und kritisch verengter peripherer Arterien, z. B. Beinarterien.

"Ischämie" im Sinne der Erfindung umfaßt eine lokalisierte Sauerstoffunterversorgung eines Gewebes, bedingt durch Blockierung des arteriellen Blutflusses. Globale Ischämie tritt auf, wenn der Blutfluß zum gesamten Gehirn für eine limitierte Zeit unterbrochen wird. Dies kann z.B. durch Herzstillstand bedingt sein. Fokale Ischämie tritt auf, wenn ein Teil des Gehirns von seiner normalen Blutzufuhr abgeschnitten wird. Fokale Ischämie kann durch thromboembolischen Verschluß eines Blutgefäßes, durch Hirntrauma, Ödeme oder Hirntumor verursacht werden. Selbst vorübergehende Ischämien können zu weitreichenden neuronalen Schäden führen. Auch wenn Schäden an "Nervengewebe" Tage oder Wochen nach dem Beginn der Ischämie auftreten können, treten manche permanente Schäden (z.B. nekrotischer Zelltod) in den ersten Minuten nach dem Unterbrechen der Blutzufuhr auf. Diese Schäden sind zum Beispiel bedingt durch Glutamat-Neurotoxizität und folgen einer sekundären Reperfusion, wie z.B. Freisetzung von Radikalen (z.B. Sauerstoff-Radikalen, NO-Radikalen). Ischämien können ebenso in anderen Organen und Geweben wie zum Beispiel im Herzen (Herzinfarkt und anderen kardiovaskulären Erkrankungen bedingt durch Verschluß der Koronararterien) oder im Auge (Ischämie der Retina) auftreten.

Gegenstand der Erfindung ist außerdem die Verwendung einer effektiven therapeutischen Menge eines PARP-Bindungspartners zur Beeinflussung neuronaler Aktivität. "Neuronale Aktivität" im Sinne der Erfindung kann in einer Stimulation geschädigter Neuronen, Förderung der neuronalen Regeneration oder Behandlung von neuronalen Erkrankungszuständen bestehen.

"Neuronale Schäden" im Sinne der Erfindung umfassen jede Art von Schädigung des "Nervengewebes" und jede körperliche oder geistige Beeinträchtigung oder Tod infolge dieser Schädigung. Die Ursache der Schädigung kann zum Beispiel metabolischer, toxischer, chemischer, thermischer Art sein und schließt beispielhaft Ischämien, Hypoxien, Trauma, cerebrovaskuläre Schäden, Operationen, Druck, Hemorrhagien, Bestrahlungen, Vasospasmen, neurodegenerative Erkrankungen, Infektionen, Epilepsie, Wahrnehmungsstörungen, Störungen im Glutamatstoffwechsel und die durch sie bedingten sekundären Effekte ein.

"Nervengewebe" im Sinne der Erfindung umfaßt die verschiedenen Komponenten, die das Nervensystem bilden, ausgewählt unter anderem aus Neuronen, Glia-Zellen, Astrozyten, Schwannschen Zellen, dem Gefäßsystem innerhalb und zur Versorgung, dem ZNS, Gehirn, Stammhirn, Rückenmark, peripheres Nervensystem etc.

"Neuroprotektiv" im Sinne der Erfindung umfaßt die Reduktion, den Stop, die Verlangsamung oder die Verbesserung neuronalen Schäden und den Schutz, die Wiederbelebung, die Regeneration von Nervengewebe, das einem neuronalen Schaden ausgesetzt war.

Eine "Prävention von neurodegenerativen Erkrankungen" schließt die Möglichkeit zur Verhinderung, Verlangsamung und Verbesserung von neurodegenerativen Erkrankungen bei Personen ein, bei denen eine solche Erkrankung diagnostiziert wurde oder die zu entsprechenden Risikogruppen für diese neurodegenerativen Erkrankungen zählen. Ebenso ist die Behandlungen von Personen gemeint, die bereits an Symptomen dieser Erkrankungen leiden.

"Behandlung" im Sinne der Erfindung umfaßt

(i) Verhinderung einer Erkrankung, einer Störung oder eines Zustandes in Personen mit einer Prädisposition zu diesen;

(ii) Verhinderung einer Erkrankung, einer Störung oder eines Zustandes durch Verlangsamung ihres Fortschritts; und

(iii) Verbesserung einer Erkrankung, einer Störung oder eines Zustandes.

Bespiele für "neurologische Erkrankungen", die durch die erfindungsgemäßen Methoden behandelt werden können, sind Neuralgien (tigeminal, glossopharyngeal), Myasthenia gravis, Muskeldystrophien, Amyotrophe Laterale Sklerose (ALS), progressive Muskelatrophie, periphere Neuropathien bedingt durch Vergiftungen (z.B. Bleivergiftung), Guillain-Barré-Syndrom, Huntington-Krankeit, Alzheimersche Krankheit, Parkinsonsche Krankheit, oder Plexus-Erkrankungen. Bevorzugt sind die erfindungsgemäßen Methoden geeignet zur Behandlung von neurologischen Erkrankungen, ausgewählt unter peripheren Neuropathien, bedingt durch physische Verletzung oder Erkrankung; Schädeltrauma, wie zum Beispiel traumatische Hirnverletzung; physische Schädigung des Rückenmarks; Schlaganfall einhergehend mit Hirnschädigung, wie vaskulärer Schlaganfall in Verbindung mit Hypoxie und Hirnschädigung, und cerebrale Reperfusionschäden; demyelinierenden Erkrankungen (Myelopathien, Alzheimersche Erkrankung, Parkisonsche Erkrankung, Huntington-Krankheit, Amyotrophe Laterale Sklerose).

Die erfindungsgemäßen Methoden können ferner zur Behandlung kardiovaskulärer Erkrankungen verwendet werden. "Kardiovaskuläre Erkrankungen" im Sinne der Erfindung umfassen solche, die Ischämien verursachen oder durch Ischämien oder Ischämie/Reperfusion des Herzens verursacht werden. Beispiele sind Koronargefäßerkrankungen (zum Beispiel Atherosklerose), Angina pectoris, Herzinfarkt, kardiovaskuläre Schäden durch Herzstillstand oder Bypass-Operation.

Die erfindungsgemäßen Methoden können zur Behandlung von Krebs oder zur Sensitisierung von Krebszellen bei Bestrahlungstherapie verwendet werden. Der Begriff "Krebs" ist im weitesten Sinne zu verstehen. Modulatoren der erfindungsgemäßen Proteine können als "Anti-Krebs Therapeutika" verwendet werden. Beispielhaft können die Methoden zur Behandlung von Krebsarten oder Tumorzellen, wie ACTH-produziertenden Tumoren, akuter lymphatischer oder lymphoblastischer Leukämie; akuter oder chronischer lymphozytärer Leukämie; akuter nicht-lymphozytärer Leukämie; Blasenkrebs, Hirntumoren; Brustkrebs; Zervikalkarzinom; chronischer myelozytärer Leukämie; Darmkrebs; T-Zonen-Lymphom; Endometriose; Speiseröhrenkrebs; Gallenblasenkrebs; Ewing Sarkom; Kopf- und Nackenkrebs; Zungenkrebs; Hodgkin Lymphom; Kaposi Sarkom; Nierenkrebs; Leberkrebs; Lungenkrebs; Mesotheliom; multiplem Myelom; Neuroblastom; non-Hodgkin Lymphom; Osteosarkom; Ovarialkarzinom; Gliablastom; Mammakarzinom; Cervikalkarzinom; Prostatakrebs; Bauchspeicheldrüsenkrebs; Peniskrebs; Retinoblastom; Hautkrebs; Magenkrebs, Schilddrüsenkrebs; Uteruskarzinom; Vaginalkarzinom; Wilm's Tumor; oder Trophoblastom verwendet werden.

"Radiosensitizer" oder "Bestrahlungs-Sensitisierer" im Sinne der Erfindung betreffen Moleküle, die im Organismus die Sensitivität der Zellen gegenüber einer Bestrahlung mit elektromagnetischer Strahlung (zum Beispiel Röntgenstrahlung) erhöhen oder diese Bestrahlungsbehandlung beschleunigen. Bestrahlungs-Sensitisierer erhöhen die Empfindlichkeit von Krebszellen gegen die toxischen Effekte der elektromagnetischen Strahlung. In der Literatur sind unter anderem Mitomycin C, 5-Bromdeoxyuridin und Metronidazol bekannt. Anwendbar ist Strahlung mit Wellenlängen im Bereich von $10^{-20}$ bis 10 Metern, vorzugsweise gamma-Strahlung ($10^{-20}$ bis $10^{-13}$ m), Röntgenstrahlung ($10^{-11}$ bis $10^{-9}$ m), ultraviolette Strahlung (10 nm bis 400 nm), sichtbares Licht (400 nm bis 700 nm), Infrarotstrahlung (700 nm bis 1 mm) und Mikrowellenbestrahlung (1 mm bis 30 cm).

Erkrankungen die durch eine solche Therapie behandelt werden können, sind vor allem neoplastische Erkrankungen, gutartige oder bösartige Tumoren und Krebs. Die Behandlung anderer Erkrankungen unter Verwendung von elektromagnetischer Strahlung ist ebenfalls möglich.

Die vorliegende Erfindung wird nun unter Bezugnahme auf die beiliegenden Figuren näher beschrieben. Dabei zeigt:

Figur 1 ein Sequenz-Alignment von menschlichem PARP (humanPARP1) und zwei erfindungsgemäß bevorzugte PARPs (humanPARP2, humanPARP3, murinPARP3). Sequenzübereinstimmungen zwischen humanPARP1 und humanPARP2, humanPARP3 bzw. murinPARP3 sind umrahmt dargestellt. Die Majoritätssequenz ist über dem Alignment angegeben. Die Zink-Finger Motive von humanPARP1 befinden sich in den Sequenzabschnitten entsprechend den Aminosäureresten 21 bis 56 und 125 bis 162;

Figur 2 Northern Blots mit unterschiedlichen menschlichen Geweben zur Veranschaulichung der Gewebeverteilung des erfindungsgemäßen PARP2 und PARP3-Moleküle. Bahn 1: Gehirn; Bahn 2: Herz; Bahn 3: Skelettmuskel; Bahn 4: Dickdarm; Bahn 5: Thymus; Bahn 6: Milz; Bahn 7: Niere; Bahn 8: Leber; Bahn 9: Dünndarm; Bahn 10: Plazenta; Bahn 11: Lunge; Bahn 12: Periphere Blutleukozyten; die jeweilige Lage der Größenstandards (kb) ist angegeben.

Figur 3 einen Northern Blot mit weiteren unterschiedlichen menschlichen Geweben zur Veranschaulichung der Gewebeverteilung des erfindungsgemäßen PARP3-Moleküls. Bahn 1: Herz; Bahn 2: Gehirn; Bahn 3: Plazenta; Bahn 4: Lunge; Bahn 5: Leber; Bahn 6: Skelettmuskel; Bahn 7: Niere; Bahn 8: Pankreas; die jeweilige Lage der Größenstandards (kb) ist angegeben.

Figur 4 einen Western Blot mit unterschiedlichen menschlichen Geweben zur Veranschaulichung der Gewebeverteilung des erfin-dungsgemäßen PARP3-Moleküls auf Protein Ebene. Bahn 1: Herz; Bahn 2: Lunge; Bahn 3: Leber; Bahn 4: Milz; Bahn 5: Niere; Bahn 6: Dickdarm; Bahn 7: Muskel; Bahn 8: Hirn; die jeweilige Lage der Grö-ßenstandards (kD) ist angegeben

Figur 5 einen Western Blot mit unterschiedlichen menschlichen Geweben zur Veranschaulichung der Gewebeverteilung des erfin-dungsgemäßen PARP3-Moleküls. Bahn 1: Frontaler Cortex; Bahn 2: Posterior Cortex; Bahn 3: Cerebellum; Bahn 4: Hippocampus; Bahn 5: Olfactory Bulb; Bahn 6: Striatum; Bahn 7: Thalamus; Bahn 8: Midbrain; Bahn 9: Entorhinal Cortex; Bahn 10: Pons; Bahn 11: Medulla; Bahn 12: Rückenmark.

Figur 6 eine schematische Darstellung des PARP-Assays (ELISA)

Figur 7 eine schematische Darstellung des PARP-Assays (HTRF)

Weitere bevorzugte Ausführungsformen der Erfindung sind in den folgenden Abschnitten beschrieben.

PARP-Homologe und funktionale Äquivalente

Soweit keine anderen Angaben gemacht werden, werden im Rahmen der vorliegenden Beschreibung Aminosäuresequenzen beginnend mit dem N-Terminus angegeben. Wird der Ein-Buchstaben-Code für Aminosäuren verwendet, so steht G für Glycin, A für Alanin, V für Valin, L für Leucin, I für Isoleucin, S für Serin, T für Threonin, D für Asparaginsäure, N für Asparagin, E für Glutaminsäure, Q für Glutamin, W für Tryptophan, H für Histidin, R für Arginin, P für Prolin, K für Lysin, Y für Tyrosin, F für Phenylalanin, C für Cystein und M für Methionin.

Die vorliegenden Erfindung ist nicht auf die oben konkret beschriebenen PARP-Homologen beschränkt. Vielmehr werden auch solche Homologen erfaßt, welche funktionale Äquivalente davon darstellen. Funktionale Äquivalente umfassen sowohl natürliche, wie z.B. Spezies-spezifische oder Organ-spezifische, als auch künstlich erzeugte Varianten der hierin konkret beschriebenen Proteine. Erfindungsgemäße funktionale Äquivalente unterscheiden sich durch Addition, Substitution, Inversion, Insertion und/oder Deletion von einem oder mehreren Aminosäureresten von humanPARP2 (SEQ ID NO:2), humanPARP3 (SEQ ID NO: 4 und 6) und mausPARP3 (SEQ ID NO: 8 und 10), wobei wenigstens noch die NAD-Bindungsfunktion des Proteins, vermittelt durch eine funktionale katalytische C-terminale Domäne, erhalten bleibt. Ebenso sollte vorzugsweise die Poly(ADP-ribose)-erzeugende katalytische Aktivität erhalten bleiben. Funktionale Äquivalente umfassen gegebenenfalls auch solche Varianten, in denen die Leucin-Zipper-ähnliche Region im wesentlichen erhalten bleibt.

Dabei können beispielsweise, ausgehend von der Sequenz für humanPARP2 oder humanPARP3 bestimmte Aminosäuren durch solche mit ähnlichen physikochemischen Eigenschaften (Raumerfüllung, Basizität, Hydrophobizität etc.) ersetzt werden. Beispielsweise können Argininreste gegen Lysinreste, Valinreste gegen Isoleucinreste oder Asparaginsäurereste gegen Glutaminsäurereste ausgetauscht werden. Es können aber auch ein oder mehrere Aminosäuren in ihrer Reihenfolge vertauscht, hinzugefügt oder entfernt werden, oder es können mehrere dieser Maßnahmen miteinander kombiniert werden. Die derart gegenüber der humanPARP2- oder humanPARP3-Sequenz veränderten Proteine besitzen wenigstens 60 %, bevorzugt wenigstens 75 %, ganz besonders bevorzugt wenigstens 85 % Homologie zur Ausgangssequenz, berechnet nach dem Algorithmus von Pearson und Lipman, Proc. Natl. Acad. Sci (USA) 85(8), 1988, 2444-2448.

Folgende Homologien wurden auf Aminosäureebene bzw. DNA-Ebene zwischen humanPARP1, 2 und 3 bestimmt (FastA-Programm, Pearson und Lipman, a.a.O.):

Aminosäure-Homologien:

| | Prozent Identität | Prozent Identität in PARP-Signature |
|---|---|---|
| PARP1/PARP2 | 41,97% (517) | 86% (50) |
| PARP1/PARP3 | 33,81% (565) | 53,1% (49) |
| PARP2/PARP3 | 35,20% (537) | 53,1% (49) |

Zahlen in Klammern geben die Anzahl der überlappenden Aminosäuren an.

DNA-Homologien:

| | Prozent Identität im ORF | Prozent Identität in PARP-Signature |
|---|---|---|
| PARP1/PARP2 | 60,81% (467) | 77,85% (149) |
| PARP1/PARP3 | 58,81% (420) | 59,02% (61) |
| PARP2/PARP3 | 60,22% (269) | 86,36% (22) |

Zahlen in Klammern geben die Anzahl der überlappenden Nukleotide an.

Die erfindungsgemäßen Polypeptide lassen sich aufgrund der hohen Ähnlichkeit im Bereich der katalytischen Domäne als homologe Poly(ADP-ribose)polymerasen klassifizieren.

Erfindungswesentlich ist außerdem, daß die neuen PARP-Homologen keine herkömmlichen Zink-Finger Motive aufweisen. Das bedeutet, daß diese Enzyme nicht notwendigerweise oder in einer von PARP1 verschiedenen Weise in die DNA-Reparatur involviert sind, wohl aber noch ihren pathologischen Mechanism ($NAD^+$-Verbrauch und somit Energieverbrauch durch ATP-Konsum) ausüben können. Die starke Proteinexpression vor allem von PARP3, die im Western-Blot zu beobachten ist, läßt eine bedeutende Rolle im NAD-Verbrauch vermuten. Dies ist von besonderer Bedeutung für die Wirkstoffentwicklung. Potentielle neue Inhibitoren gegen die erfindungsgemäßen Polymerasen können also die pathologischen Funktionen hemmen, ohne negative Effekte auf die gewünschten physiologischen Eigenschaften zu haben. Mit Inhibitoren gegen die bislang bekannte PARPs war dies nicht möglich, da auch immer die DNA-Reparaturfunktion mitinhibiert wurde. Die potentiell mutagene Wirkung bekannter PARP-Inhibitoren ist somit leicht verständlich. Ferner ist es denkbar, PARP-Inhibitoren so zu gestalten, daß sie mit hoher Affinität alle PARP-Homologen effektiv inhibieren. In diesem Fall ist gegebenenfalls eine potenzierte Wirkung denkbar.

Das erfindungsgemäß bevorzugte PARP-Homologe gemäß SEQ ID NO:2 (human PARP2) läßt sich vorteilhafterweise aus dem menschlichen Hirn, Herz, skelettmuskel, Niere und Leber isolieren. In anderen Geweben oder Organen ist humanPARP2 deutlich schwächer exprimiert.

Das erfindungsgemäß bevorzugte PARP-Homologe gemäß SEQ ID NO: 4 und 6 (humanPARP3) läßt sich vorteilhafterweise aus dem menschlichen Hirn (hier bevorzugt aus dem Hippocampus), Herz, Skelettmuskel, Leber oder Niere isolieren. In anderen Geweben oder Organen, wie Muskel oder Leber, ist humanPARP3 deutlich schwächer exprimiert.

Der mit der Proteinisolierung vertraute Fachmann wird zur Gewinnung erfindungsgemäßer natürlicher PARPs aus Geweben oder erfindungsgemäßer rekombinant hergestellter PARPs aus Zellkulturen die dazu jeweils am geeignetste Kombination von präparativen Verfahrensmaßnahmen ergreifen. Geeignete präparative Standardmethoden sind zum Beispiel beschrieben in Cooper, T.G., Biochemische Arbeitsmethoden, Verlag Walter de Gruyter, Berlin, New York oder in Scopes, R. Protein Purification, Springer Verlag, New York, Heidelberg, Berlin.

Gegenstand der Erfindung sind außerdem PARP2- und PARP3-Homologe, welche zwar aus anderen eukaryotischen Spezies, d.h. Evertebraten (Invertebraten) oder Vertebraten, insbesondere anderen Säugern, wie z.B. Maus, Ratte, Katze, Hund, Schwein, Schaf, Rind, Pferd, oder Affe oder aus anderen Organen, wie z.B. Myokard, isolierbar sind, aber die wesentlichen, von den erfindungsgemäßen PARPs vorgegebenen strukturellen und funktionellen Eigenschaften besitzen.

Insbesondere das aus menschlichem Hirn isolierbare humanPARP2 und dessen funktionale Äquivalente sind bevorzugte Agenzien für die Entwicklung von Inhibitoren gegen neurodegenerative Erkrankungen, wie z. B. Schlaganfall. Es kann nämlich angenommen werden, daß die Wirkstoffentwicklung, basierend auf PARP2 als Indikator, die Entwicklung von Inhibitoren ermöglicht, welche für die Anwendung an menschlichem Gehirn optimiert sind. Es ist jedoch nicht auszuschließen, daß auf Basis von PARP2 entwickelte Inhibitoren auch zur Therapierung PARP-vermittelter pathologischer Zustände anderer Organe einsetzbar sind (vgl. Gewebeverteilung der erfindungsgemäßen Proteine).

Ähnlich wie PARP1 werden auch PARP2 und vermutlich PARP3 durch geschädigte DNA, wenn auch durch einen vermutlich anderen Mechanismus aktiviert. Eine Bedeutung in der DNA-Reparatur ist denkbar. Die Blockade der erfindungsgemäßen PARPs würde auch in Indikationen, wie Krebs, von Nutzen sein (z.B. in der Radiosensitisierung von Tumorpatienten).

Eine weitere wesentliche biologische Eigenschaft von erfindungsgemäßen PARPs und deren funktionalen Äquivalenten ist in deren Befähigung zur Bindung eines Interaktionspartners zu sehen. Im Unterschied zur bislang bekannten PARPs aus höheren Eukaryoten, wie insbesondere Säugern, verfügen humanPARP2 und 3 über potentielle sogenannte Leucin-Zipper-Motive. Dies ist ein typisches Motiv für Protein-Protein-Wechselwirkungen. Diese Motive erlauben möglicherweise eine Modulation der PARP-Aktivität durch einen Interaktionspartner. Somit liefert auch dieses zusätzliche Strukturelement einen möglichen Ansatzpunkt für die Entwicklung von PARP-Effektoren, wie z.B. Inhibitoren.

Ein weiterer Gegenstand der Erfindung sind somit Proteine, die mit PARP2 und/oder 3 wechselwirken, bevorzugt solche, die ihre Aktivierung oder Inaktivierung bewirken.

Ein weiterer Gegenstand der Erfindung sind auch Proteine, die noch die oben genannte Ligandenbindungsaktivität aufweisen und die ausgehend von den konkret offenbarten Aminosäuresequenzen durch gezielte Veränderungen herstellbar sind.

Ausgehend von der Peptidsequenz der erfindungsgemäßen Proteine können synthetische Peptide generiert werden, die einzeln oder in Kombination als Antigene für die Produktion von polyklonalen oder monoklonalen Antikörpern eingesetzt werden. Es ist auch möglich, das PARP-Protein oder Bruchstücke davon zur Generierung von Antikörpern einzusetzen. Gegenstand der Erfindung sind somit auch Peptidfragmente erfindungsgemäßer PARP-Proteine, welche charakteristische Teilsequenzen umfassen, insbesondere solche Oligo- oder Polypeptide, welche wenigstens eines der oben genannten Sequenzmotive umfassen. Solche Fragmente sind beispielsweise durch proteolytischen Verdau von PARP-Proteinen oder auf chemischem Weg durch Peptidsynthese erhältlich.

Neue PARP2- und PARP3-Bindungspartner

Unter Verwendung der oben beschriebenen spezifischen Assay-Systeme für Bindungspartner von PARP2 und PARP3 wurden aktive und vorzugsweise selektive Inhibitoren gegen die erfindungsgemäßen Proteine entwickelt. Diese Inhibitoren sind gegebenenfalls auch gegenüber PARP1 aktiv.

Erfindungsgemäß bereitgestellte Inhibitoren besitzt gegenüber PARP2 eine stark ausgeprägte inhibitorische

Aktivität. Die $K_i$-Werte können dabei weniger als etwa 1000 nM, wie z. B. weniger als etwa 700 nM, weniger als etwa 200 nM oder weniger als etwa 30 nM, wie z.B. etwa 1 bis 20 nM, betragen.

Erfindungsgemäß bereitgestellte Inhibitoren können außerdem eine überraschende Selektivität für PARP2 besitzen. Das Verhältnis $K_i$(PARP1) : $K_i$(PARP2) für solche erfindungsgemäße Inhibitoren ist z.B. größer als 3 oder größer als 5, wie z. B. größer als 10, oder größer als 20.

Beispielhaft ist zu nennen 4-(N-(4-Hydroxyphenyl)aminomethyl)-(2H)-dihydrophthalazin-1-on. Die Herstellung dieser und analoger Verbindungen erfolgt entsprechend den Anweisungen in Puodzhyunas et al., Pharm. Chem. J., 1973, 7, 566f., oder Mazkanowa et al., Zh. Obshch. Khim., 1958, 28, 2798f., oder Mohamed et al., Ind. J. Chem. B. 1994, 33, 769f., worauf hiermit ausdrücklich Bezug genommen wird.

Die oben genannte Verbindung besitzt gegenüber PARP2 einen $K_i$-Wert von 113 nM und wirkt gegenüber PARP2 etwa 8-fach selektiver als gegenüber PARP1.

Für PARP-Homologe kodierende Nukleinsäuren:

Wenn keine anderen Angaben gemacht werden so erfolgt im Rahmen der vorliegenden Beschreibung die Angabe der Nukleotidsequenzen von 5'- in 3'-Richtung.

Ein weiterer Gegenstand der Erfindung sind Nukleinsäuresequenzen, die für die oben genannten Proteine, insbesondere für solche mit der in SEQ ID NO: 2, 4, 6, 8 und 10 dargestellten Aminosäuresequenz, kodieren, ohne jedoch darauf beschränkt zu sein. Erfindungsgemäß brauchbare Nukleinsäuresequenzen umfassen auch Allelvarianten, die, wie oben für die Aminosäuresequenzen beschrieben, durch Deletion, Inversion, Insertion, Addition und/oder Substitution von Nukleotiden, vorzugsweise von Nucleotiden gemäß SEQ ID NO: 1, 3, 7 und 9, erhältlich sind, wobei die biologischen Eigenschaften bzw. die biologische Aktivität des korrespondierenden Genprodukts aber im wesentlichen erhalten bleibt. Brauchbare Nukleotidsequenzen erhält man beispielsweise durch Nukleotidsubstitutionen, welche stumme (ohne Veränderung der Aminosäuresequenz) oder konservative Aminosäure-Veränderungen (Austausch von Aminosäuren gleicher Größe, Ladung, Polarität oder Löslichkeit) bewirken.

Weiterhin umfassen erfindungsgemäße Nukleinsäuresequenzen auch funktionelle Äquivalente der Gene, wie eukaryontische Homologe beispielsweise aus Evertebraten, wie Caenorhabditis oder Drosophila, oder Vertebraten, vorzugsweise aus den oben beschriebenen Säugern. Bevorzugt sind Gene aus Vertebraten, die für ein Genprodukt kodieren, das die oben beschriebenen, erfindungswesentlichen Eigenschaften besitzt.

Die erfindungsgemäßen Nukleinsäuren können in herkömmlicher Weise auf verschiedenen Wegen erhalten werden:

Beispielsweise kann eine genomische oder eine cDNA-Bibliothek auf DNA durchmustert werden, die für ein PARP-Molekül oder einen Teil davon kodiert. Beispielsweise kann eine aus menschlichem Gehirn, Herz oder Niere gewonnene cDNA-Bibliothek mit einer geeigneten Sonde, wie z.B einem markierten Einzelstrang-DNA-Fragment durchmustert werden, die einer aus SEQ ID NO: 1 oder 3 ausgewählten Teilsequenz geeigneter Länge oder dazu komplementären Sequenz entspricht. Dazu können beispielsweise die in einen geeigneten Klonierungsvektor überführten DNA-Fragmente der Bibliothek nach Transformation in ein Bakterium auf Agarplatten ausplattiert werden. Die Klone können anschließend auf Nitrozellulose-Filter übertragen und nach Denaturierung der DNA mit der markierten Sonde hybridisiert werden. Positive Klone werden dann isoliert und charakterisiert.

Die für erfindungsgemäße PARP-Homologe oder Teilfragmente kodierende DNA kann auch ausgehend von den in vorliegender Anmeldung enthaltenen Sequenzinformationen chemisch synthetisiert werden. Beispielsweise können hierfür Oligonukleotide mit einer Länge von etwa 100 Basen in an sich bekannter Weise synthetisiert und sequentiell ligiert werden, indem man beispielsweise geeignete terminale Restriktionsschnittstellen vorsieht.

Die erfindungsgemäßen Nukleotidsequenzen sind auch mit Hilfe der Polymerase-Kettenreaktion (PCR) herstellbar. Dazu hybridisiert man eine Target-DNA, wie z.B. DNA aus einem geeigneten Full-Length-Klon, mit einem Paar synthetischer Oligonukleotid-Primer von etwa 15 Basen Länge, welche an den gegenüberliegenden Enden der Target-DNA binden. Anschließend wird der dazwischenliegende Sequenzabschnitt mit DNA-Polymerase aufgefüllt. Die mehrfache Wiederholung dieses Cyclus erlaubt eine Amplifizierung der Target-DNA (vgl. White et al.(1989), Trends Genet. 5, 185)

Weiterhin sind unter den erfindungsgemäßen Nukleinsäuresequenzen auch verkürzte Sequenzen, Einzelstrang-DNA oder RNA der kodierenden und nichtkodierenden, komplementären DNA-Sequenzen, mRNA-Sequenzen und davon abgeleitete cDNAs zu verstehen.

Die Erfindung umfaßt weiterhin die mit obigen Sequenzen unter stringenten Bedingungen hybridisierenden Nukleotidsequenzen. Stringente Hybridisierungsbedingungen im Sinne der vorliegenden Erfindung sind gegeben, wenn die hybridisierenden Sequenzen eine Homologie von etwa 70 bis 100%, wie z.B etwa 80 bis 100% oder 90 bis 100%, aufweisen (vorzugsweise in einem Aminosäureabschnitt von mindestens etwa 40, wie z.B. etwa 50, 100, 150, 200, 400 oder 500 Aminosäuren).

Stringente Bedingungen für das Screening von DNA, insbesondere cDNA-Banken, sind z.B gegeben, wenn man bei einer Temperatur von etwa 60°C mit 0,1X SSC-Puffer (20X SSC-Puffer = 3M NaCl, 0,3M Natriumcitrat, pH 7,0) und 0,1% SDS den Hybridisierungsansatz wäscht.

Northern-Blot-Analysen werden unter stringenten Bedingungen beispielseweise bei einer Temperatur von etwa 65 °C mit 0,1X SSC, 0,1% SDS gewaschen.

Nukleinsäurederivate und Expressionskonstrukte:

Unter den Nukleinsäuresequenzen sind auch Derivate, wie beispielsweise Promotorvarianten oder alternative Spleißvarianten, zu verstehen. Die Promotoren, die den erfindungsgemäßen Nukleotidsequenzen unter operativer Verknüpfung vorgeschalten sind, können dabei durch Nukleotidaddition(en) oder -substitution(en), Inversion(en), Insertion (en) und/oder Deletion(en) verändert sein, ohne daß aber die Funktionalität bzw. Wirksamkeit der Promotoren beeinträchtigt wird. Des weiteren können die Promotoren durch Veränderung ihrer Sequenz in ihrer Wirksamkeit erhöht oder komplett durch wirksamere Promotoren auch artfremder Organismen ausgetauscht werden. Oben beschriebene Promotorvarianten werden zur Herstellung von erfindungsgemäßen Expressionskassetten herangezogen.

Als konkrete Beispiele für humanPARP2-Spleißvarianten sind zu nennen:

Variante humanPARP2a: Deletion der Basenpaare 766 bis 904 (vgl. SEQ ID NO:1). Dies führt zu einem Frame-Shift mit einem neuen Stop-Codon ("TAA" gemäß Nucleotiden 922 bis 924 in SEQ ID NO:1). Variante humanPARP2b: Insertion von
5'- gta tgc cag gaa ggt cat ggg cca gca aaa ggg tct ctg -3'
nach Nukleotid 204 (SEQ ID NO:1). Dies verlängert die Aminosäuresequenz um den Einschub: GMPGRSWASKRVS

Unter Nukleinsäurederivaten sind auch Varianten zu verstehen, deren Nukleotidsequenzen im Bereich von -1 bis -1000 vor dem Startkodon so verändert wurden, daß die Genexpression und/oder die Proteinexpression erhöht wird.

Neben der oben beschriebenen Nukleotidsequenz umfassen erfindungsgemäß brauchbare Nukleinsäurekonstrukte in funktioneller, operativer Verknüpfung einer oder mehrerer weiterer regulativer Sequenzen, wie Promotoren, Amplifikationssignale, Enhancer, Polyadenylierungssequenzen, Replikationsursprünge, Reportergene, selektierbare Markergene und dergleichen. Diese Verknüpfung kann je nach gewünschter Anwendung zu einer Erhöhung oder Erniedrigung der Genexpression führen.

Zusätzlich zu den neuen Regulationssequenzen kann die natürliche Regulationssequenz vor den eigentlichen Strukturgenen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt, es werden keine zusätzlichen Regulationssignale vor die Strukturgene insertiert und der natürliche Promotor mit seiner Regulation wird nicht entfernt. Stattdessen wird die natürliche Regulationssequenz so mutiert, daß keine Regulation mehr erfolgt und die Genexpression gesteigert oder verringert wird. Auch am 3'-Ende der Nukleinsäuresequenzen können zusätzliche vorteilhafte regulatorische Elemente insertiert werden. Die Nukleinsäuresequenzen können in einer oder mehreren Kopien im Genkonstrukt enthalten sein.

Vorteilhafte Regulationssequenzen für das erfindungsgemäße Expressionsverfahren sind beispielsweise in Promotoren wie cos-, tac-, trp-, tet-, trp-tet-, lpp-, lac-, lpp-lac-, laclq-, T7-, T5-, T3-, gal-, trc-, ara-, SP6-, 1-PR- oder im 1-PL-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefepromotoren ADC1, MFa, AC, P-60, CYCl, GAPDH oder in den Pflanzenpromotoren CaMV/35S, SSU, OCS, lib4, usp, STLS1, B33, nos oder im Ubiquitin- oder Phaseolin-Promotor enthalten.

Prinzipiell können alle natürlichen Promotoren mit ihren Regulationssequenzen verwendet werden. Darüberhinaus können auch synthetische Promotoren vorteilhaft verwendet werden.

Die genannten regulatorischen Sequenzen sollen die gezielte Expression der Nukleinsäuresequenzen und die Proteinexpression ermöglichen. Dies kann beispielsweise je nach Wirtsorganismus bedeuten, daß das Gen erst nach Induktion exprimiert oder überexprimiert wird, oder daß es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Unter "Enhancer" sind beispielsweise DNA-Sequenzen zu verstehen, die über eine verbesserte Wechselwirkung zwischen RNA-Polymerase und DNA eine erhöhte Expression bewirken.

Das rekombinante Nukleinsäurekonstrukt bzw. Genkonstrukt wird zur Expression in einem geeigneten nichtmenschlichen Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren wie beispielsweise Phagen, Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Expression der Konstrukte:

Vorteilhafterweise werden die oben beschriebenen erfindungsgemä-ßen rekombinanten Konstrukte in ein geeignetes Wirtssystem eingebracht und exprimiert. Dabei werden vorzugsweise dem Fachmann bekannte geläufige Klonierungs- und Transfektionsmethoden verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, beschrieben.

Als Wirtsorganismen sind prinzipiell alle nicht-menschlichen Organismen geeignet, die eine Expression der erfindungsgemäßen Nukleinsäuren, ihrer Allelvarianten, ihrer funktionellen Äquivalente oder Derivate oder des rekombinanten Nukleinsäurekonstrukts ermöglichen. Unter Wirtsorganismen sind beispielsweise Bakterien, Pilze, Hefen, pflanzliche oder tierische Zellen zu verstehen. Bevorzugte Organismen sind Bakterien, wie solche der Gattungen Escherichia, wie z.B. Escherichia coli, Streptomyces, Bacillus oder Pseudomonas, eukaryotische Mikroorganismen, wie Saccharomyces cerevisiae, Aspergillus, höhere eukaryotische Zellen aus Tieren oder Pflanzen, beispielsweise Sf9 oder CHO-Zellen.

Gewünschtenfalls kann das Genprodukt auch in transgenen Organismen wie transgenen nicht-menschlichem Tieren, wie insbesondere Mäusen, Schafen oder transgenen Pflanzen zur Expression gebracht werden. Bei den transgenen nicht-menschlichen Organismen kann es sich auch um sogenannte Knock-Out Tiere oder Pflanzen handeln, in denen das korrespondierende endogene Gen ausgeschaltet wurde, wie z.B. durch Mutation oder partielle oder vollständige Deletion.

Die Kombination aus den nicht-menschlichen Wirtsorganismen und den zu den Organismen passenden Vektoren, wie Plasmide, Viren oder Phagen, wie beispielsweise Plasmide mit dem RNA-Polymerase/Promoter System, die Phagen $\lambda$, $\mu$ oder andere temperente Phagen oder Transposons und/oder weiteren vorteilhaften regulatorischen Sequenzen bildet ein Expressionssystem. Bevorzugt sind unter dem Begriff Expressionssysteme beispielsweise die Kombination aus Säugetierzellen, wie CHO-Zellen, und Vektoren, wie pcDNA3neo-Vektor, die für Säugetierzellen geeignet sind, zu verstehen.

Wie oben beschrieben, kann das Genprodukt vorteilhaft auch in transgenen nicht-menschlichem Tieren, z.B. Mäusen, Schafen oder transgenen Pflanzen zur Expression gebracht werden. Ebenso ist es möglich, zellfreie Translationssysteme mit der von der Nukleinsäure abgeleiteten RNA zu programmieren.

Darüberhinaus kann das Genprodukt auch in Form therapeutisch oder diagnostisch geeigneter Fragmente exprimiert werden. Zur Isolierung des rekombinanten Proteins können vorteilhaft Vektorsysteme oder Oligonukleotide verwendet werden, die die cDNA um bestimmte Nukleotidsequenzen verlängern und damit für veränderte Polypeptide kodieren, die einer einfacheren Reinigung dienen. Derartige geeignte Modifikationen sind beispielsweise als Anker fungierende sogenannte "Tags", wie z.B. die als Hexa-Histidin-Anker bekannte Modifikation, oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Herstellung von Antikörpern:

Die Herstellung von Anti-PARP2-Antikörpern erfolgt in einer dem Fachmann geläufigen Weise. Mit Antikörpern sind sowohl polyklonale, monoklonale, humane oder humanisierte Antikörper oder Fragmente davon, single chain Antikörper oder auch synthetische Antikörper gemeint, ebenso wie Antikörperfragmente, wie Fv, Fab und $F(ab)'_2$. Geeignete Herstellungsverfahren sind z.B. beschrieben in Campbell, A.M., Monoclonal Antibody Technology, (1987) Elsevier Verlag, Amsterdam, New York, Oxford und in Breitling, F. und Dübel, S., Rekombinante Antikörper (1997), Spektrum Akademischer Verlag, Heidelberg.

Weitere Anwendung der kodierenden Sequenz:

Die vorliegende cDNA bietet außerdem die Voraussetzung, die genomische Sequenz der neuen PARP-Gene zu klonieren. Darunter fallen auch die dazugehörigen regulatorischen oder Promotorsequenzen, die beispielsweise durch Sequenzierung des 5'-stromaufwärts gelegenen Bereiches der erfindungsgemäßen cDNA zugänglich sind oder in den Introns der Gene zu finden sind. Die Sequenzinformation der cDNA ist auch die Grundlage für die Herstellung von Antisense-Molekülen oder Ribozymen mit Hilfe bekannter Methoden (vgl. Jones, J.T. und Sallenger, B.A. (1997) Nat. Biotechnol. 15, 902; Nellen, W. und Lichtenstein, C. (1993) TIBS, 18, 419). Die genomische DNA kann ebenfalls zur Herstellung der oben beschriebenen Genkonstrukte verwendet werden.

Eine weitere Möglichkeit des Einsatzes der Nukleotidsequenz oder Teilen davon ist die Erzeugung transgener nicht-menschlichen Tiere. Transgene Überexpression oder genetischer Knockout der Sequenzinformation in geeigneten Tiermodellen kann wertvolle weitere Informationen über die (Patho-)Physiologie der neuen Gene liefern.

Therapeutische Anwendungen:

In Situationen, in denen ein Mangel an einem erfindungsgemäßen Protein herrscht, können mehrere Methoden zur Substituierung eingesetzt werden. Zum einen kann das Protein, natürlich oder rekombinant direkt, oder gentherapeutisch in Form seiner kodierenden Nukleinsäure (DNA oder RNA) appliziert werden. Dazu können beliebige Vektoren beispielsweise sowohl virale, als auch nichtvirale Vehikel zum Einsatz kommen. Geeignete Methoden werden beispielsweise beschrieben von Strauss und Barranger in Concepts in Gene Therapy (1997), Walter de Gruyter, Hrsg. Eine weitere Alternative bietet die Stimulation des endogenen, körpereigenen Genes durch geeignete Mittel.

Auch der turn-over oder die Inaktivierung erfindungsgemäßer PARPs, z.B. durch Proteasen, können blockiert werden. Schließlich können Inhibitoren oder Agonisten erfindungsgemäßer PARPs zum Einsatz gelangen.

In Situationen, in denen überschüssiges PARP oder überaktiviertes PARP vorliegt, können unterschiedliche Typen von Inhibitoren eingesetzt werden. Diese Inhibition kann sowohl durch Antisense-Moleküle, Ribozyme, Oligonukleotide oder Antikörper, als auch durch niedermolekulare Verbindungen erreicht werden.

Die erfindungsgemäßen wirkstoffe, d.h. PARP-Proteine, Nukleinsäuren und PARP-Bindungspartner, wie z.B. Antikörper oder Modulatoren, können entweder als einzelne therapeutische Wirkstoffe oder als Mischungen mit anderen therapeutischen Wirkstoffen verabreicht werden. Sie können als solche verabreicht werden, im Allgemeinen werden sie jedoch in Form pharmazeutischer Mittel verabreicht, d.h. als Mischungen des oder der wirkstoffe mit wenigstens einem geeigneten pharmazeutischen Träger oder Verdünnungsmittel. Die Wirkstoffe oder Mittel können auf jedem für den jeweiligen therapeutischen Zweck geeigneten Weg, z.B. oral oder parenteral, verabreicht werden.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienten enthalten, wie Bindemittel (z.B. Sirup, Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talcum, Polyethylenglykol oder Siliciumdioxid), disintegrierende Mittel (z.B. Stärke) oder Netzmittel (z.B. Natriumlaurylsulfat). Orale flüssige Präparate können in Form wässriger oder öliger Suspensionen, Lösungen, Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren, enthalten. Für die parenterale Verabreichung kann man Lösungen oder Suspensionen mit üblichen pharmazeutischen Trägern einsetzen. Die parenterale Applikation erfindungsgemäßer wirkstoffe erfolgt vorteilhafterweise unter Verwendung eines parenteral, insbesondere intravenös verabreichbaren, flüssigen pharmazeutischen Mittels. Dieses enthält vorzugsweise in einem für diesen Zweck geeigneten, pharmazeutisch akzeptablen Träger, eine wirksame Menge wenigstens eines wirkstoffs, vorzugsweise in gelöster Form. Beispiele hierfür geeigneter pharmazeutische Träger sind insbesondere wäßrige Lösungen, wie z.B. physiologische Kochsalzlösung, Phosphat-gepufferte Kochsalzlösung, Ringerlösung, laktierte Ringerlösung und dergleichen. Außerdem kann das Mittel weitere Zusätze, wie Antioxidantien, chelatbildende Mittel oder antimikrobielle Mittel, enthalten.

Die jeweilige Wahl der Dosierung der erfindungsgemäßen Wirkstoffe und der jeweilige Dosierungsplan obliegen der Entscheidung des behandelnden Arztes. Dieser wird in Abhängigkeit vom gewählten Verabreichungsweg, von der Wirksamkeit des jeweiligen Medikaments, von der Art und Schwere der zu behandelnden Erkrankung, von dem Befinden des Patienten und dessen Ansprechen auf die Therapie einen geeignete Dosis und einen entsprechenden Dosierungsplan auswählen. So können z.B. die erfindungsgemäßen pharmakologisch wirksamen Substanzen an ein Säugetier (Mensch und Tier) in Dosen von etwa 0,5 mg bis etwa 100 mg pro kg Körpergewicht pro Tag verabreicht werden. Sie können in einer Einzeldosis oder in mehreren Dosen verabreicht werden.

Nicht-therapeutische Anwendungen:

Weiterhin können die erfindungsgemäßen Nukleinsäuren, wie z.B. cDNA, die genomische DNA, der Promotor, als auch das Polypeptid, sowie Teilfragmente davon in rekombinanter oder nichtrekombinanter Form zur Ausarbeitung von verschiedenen Testsystemen verwendet werden.

Beispielsweise kann ein Testsystem etabliert werden, das geeignet ist, die Aktivität des Promotors oder des Proteins in Anwesenheit einer Testsubstanz zu messen. Bevorzugt handelt es sich dabei um einfache, z.B. colorimetrische, luminometrische, fluorimetrische, immunologische oder radioaktive, meßmethoden, die die schnelle Meßbarkeit, vorzugsweise einer Vielzahl von Testsubstanzen erlauben. Derartige Tests eignen sich vorteilhaft für ein sogenanntes High-Throughput-Screening. Diese Testsysteme erlauben eine Bewertung von Testsubstanzen in Bezug auf deren Bindung an oder deren Agonisierung, Antagonisierung oder Inhibition von erfindungsgemäßen Proteinen.

Die Bestimmung von Menge, Aktivität und Verteilung der erfindungsgemäßen Proteine oder ihrer zugrundeliegenden mRNA im menschlichen Körper kann zur Diagnose, zur Bestimmung der Prädisposition und zum Monitoring bei bestimmten Erkrankungen dienen. Desgleichen kann die Sequenz der cDNA sowie der genomischen Sequenz zu Aussagen über genetische Ursachen und Prädispositionen bestimmter Erkrankungen herangezogen werden. Dazu können sowohl DNA/RNA-Sonden als auch Antikörper verschiedenster Art benutzt werden. Weiterhin können die erfindungsgemäßen Nukleotidsequenzen oder Teile davon in Form geeigneter Sonden zur Aufdeckung von Punktmutationen, Deletionen oder Insertionen dienen.

Weiterhin können die erfindungsgemäßen Proteine benutzt werden, um ihre natürlichen Liganden oder Interaktionspartner zu bestimmen und zu isolieren. Außerdem können die erfindungsgemäßen Proteine benutzt werden, um künstliche oder synthetische Liganden zu bestimmen und zu isolieren. Dazu kann man das rekombinant hergestellte oder gereinigte natürliche Protein derart derivatisieren, daß es Modifikationen trägt, die eine Verknüpfung mit Trägermaterialien erlauben. Derart gebundenen Proteine können mit unterschiedlichen Analyten, wie z.B. Proteinextrakten oder Peptidbibliotheken oder anderen Quellen für Liganden, inkubiert werden. Spezifisch gebundene Peptide, Proteine oder niedermolekulare, nichtproteinogene Substanzen können so isoliert und charakterisiert werden. Unter nichtproteinogenen Substanzen sind beispielsweise niedermolekulare, chemische Substanzen zu verstehen, die beispielsweise aus der klassischen wirkstoffsynthese oder aus sogenannten Substanzbibliotheken stammen können, die mit Hilfe der Kombinatorik synthetisiert worden sind.

Die verwendeten Proteinextrakte sind beispielsweise abgeleitet aus Homogenaten von Pflanzen oder Pflanzenteilen, Mikroorganismen, menschlichen oder tierischen Geweben oder Organen.

Liganden oder Interaktionspartner können ferner durch Verfahren, wie das Hefe "Two-Hybrid-System" identifiziert werden (Fields, S. und Song, O. (1989) Nature, 340, 245). Die dabei einsetzbaren Expressionsbanken sind beispielsweise ableitbar aus menschlichen Geweben, wie z.B. Gehirn, Herz, Niere usw.

Die erfindungsgemäßen Nukleinsäuresequenzen und die von ihnen kodierten Proteine können zur Entwicklung von Reagenzien, Agonisten und Antagonisten oder Inhibitoren zur Diagnose und Therapie von chronischen und akuten Erkrankungen, die mit der Expression oder Aktivierung einer der erfindungsgemäßen Proteinsequenzen, wie z.B. mit deren erhöhter oder erniedrigter Expression assoziiert sind, eingesetzt werden. Die entwickelten Reagenzien, Agonisten, Antagonisten oder Inhibitoren können anschließend zur Herstellung von pharmazeutischen Zubereitungen zur Behandlung oder Diagnose von Krankheiten verwendet. Dabei kann es sich beispielsweise um Erkrankungen des Gehirns, des peripheren Nervensystems, des Herz-Kreislaufsystems oder des Auges, von septischem Schock, der rheumatoiden Arthrits, Diabetes, akutes Nierenversagen, oder von Krebs handeln.

Die Relevanz der erfindungsgemäßen Proteine für die genannten Indikationen wurde mittels spezifischer Inhibitoren in relevanten Tiermodellen verifiziert.

Die Erfindung wird nun unter Bezugnahme auf die folgenden Ausführungsbeispiele näher erläutert.

Beispiel 1: Isolierung der PARP2- und PARP3-cDNA

Bei der Sequenzanalyse von cDNA-Klonen einer cDNA-Bibliothek aus menschlichem Gehirn (Human Brain 5'Stretch Plus cDNA Library, # HL3002a, Fa. Clontech) wurden die vorliegenden cDNA-Sequenzen erstmalig gefunden. Die Maus PARP3 Klone wurden aus einer "Lambda-Triplex mouse brain cDNA library" (Clontech Bst.-Nr. ML5004t) isoliert. Die Sequenzen dieser Klone sind in SEQ ID NO:1, 3, 7 und 9 beschrieben.

Beispiel 2: Expression von PARP2 bzw. PARP3 in menschlichen Geweben

Die Expression von humanPARP2 bzw. humanPARP3 wurde in zwölf verschiedenen menschlichen Geweben mittels Northern Blot-Analyse untersucht. Ein "Human Multiple Tissue Northern Blot (MTN™) der Firma Clontech (#7760-1 und #7780-1) wurde dazu mit einer RNA-Sonde hybridisiert. Die Sonde wurde durch in vitro Transkription der entsprechenden cDNA von humanem PARP2 bzw. humanem PARP3 in Anwesenheit Digoxigenin-markierter Nukleotide nach Vorschrift des Herstellers (BOEHRINGER MANNHEIM DIG Easy Hyb Best. Nr. 1603 558, DIG Easy Hyb Vorschrift für RNA:RNA Hybridisierung) hergestellt. In Abänderung des Protokolls wurde die Vorhybridisierung: 2x1h unter Zugabe von Heringssperma DNA (10mg/ml Hybridisierungslösung) durchgeführt. Die Hybridisierung erfolgte dann über Nacht unter Zugabe von Heringssperma DNA (10mg/ml Hybridisierungslösung). Die Detektion der Banden erfolgte unter Verwendung des CDP-Star Protokolls (BOEHRINGER MANNHEIM CDP-Star™ Best.
Nr. 1685 627).

Nach stringenter Waschung wurde das Transkript von PARP2 hauptsächlich im menschlichen Hirn, Herz, Skelettmuskel, Niere und Leber nachgewiesen. Die Größe des Transkriptes von ca. 1.9 kb entspricht der Länge der bestimmten cDNA (1.85kb) (vgl. Figur 2(A)).

In anderen Geweben oder Organen ist humanPARP2 deutlich schwächer exprimiert.

Nach stringenter Waschung wurde die Expression des Transkripts von PARP3 hauptsächlich im Herzen, Gehirn, Niere, Skelettmuskel und Leber nachgewiesen. Die Expression in weiteren Geweben (Plazenta, Lunge, Bauchspeicheldrüse) ist deutlich schwächer (vgl. Figur 2(B)). Zu humanPARP3 gibt es mindestens 2 Transkripte, die vermutlich durch unterschiedliche Polyadenylierungsstellen oder alternatives Spleißen zu erklären sind. Deren Größe (ca. 2,2 kb bzw. 2,5 kb) entspricht der Länge der bestimmten cDNA (2.3kb). Gewaschen wurde 2 x 5 Minuten mit 0,2 x SSC/0,2 % SDS bei Raumtemperatur und dann 2 x 15 Minuten mit 0,1 x SSC/0,1 % SDS bei 65 °C (hergestellt aus 20X SSC: 3M NaCl, 0,3M Natriumcitrat, pH 7,0).

Beispiel 3: Herstellung von Antikörpern

spezifische Antikörper gegen die erfindungsgemäßen Proteine wurden hergestellt. Diese dienten u.a. der Analyse der Gewebeverteilung auf Proteinebene von PARP2 und PARP3 durch Immunoblot (Western-Blot) Analyse. Beispiele für die Herstellung solcher Antikörper sind im Folgenden angegeben.

Folgende Peptide wurden für die Antikörperherstellung synthetisch in der dem Fachmann geläufigen Weise hergestellt. Teilweise wurden den Sequenzen ein Cystein-Rest N-.oder C-terminal angehängt, um die Kopplung an KLH (key hole limpet hemocyanin) zu erleichtern.

| | |
|---|---|
| PARP-2: | $NH_2$-MAARRRRSTGGGRARALNES-$CO_2H$ (Aminosäuren 1-20; SEQ ID N0:23) |
| | $NH_2$-KTELQSPEHPLDQHYRNLHC-$CO_2H$ (Aminosäuren 335-353; SEQ ID NO:24)) |
| PARP-3: | $NH_2$-CKGRQAGREEDPFRSTAEALK-$CO_2H$ (Aminosäuren 25-44; SEQ ID NO:25) |
| | $NH_2$-CKQQIARGFEALEALEEALK-$CO_2H$ (Aminosäuren 230-248; SEQ ID NO:26) |

Als repräsentatives Beispiel ist die Herstellung eines Anti-PARP3 Antikörpers erläutert.

Für das humane PARP3 wurden polyklonale Antikörper in Kaninchen unter Verwendung eines synthetischen Peptides mit der Peptid-Sequenz $H_2N$-KQQIARGFEALEALEEALK-$CO_2H$ (SEQ ID NO:27) generiert (Aminosäuren 230-248 der humanen PARP3 Proteinsequenz). Die entsprechende Maussequenz in diesem Bereich unterscheidet sich lediglich um eine Aminosäure ($H_2N$-KQQIARGFEALEALEEAMK-$CO_2H$; SEQ ID NO:28). N-Terminal wurde noch ein Cystein angehängt, um die Kopplung des Proteins an KLH zu ermöglichen.

Kaninchen wurden im Abstand von 7-14 Tagen insgesamt fünfmal mit dem KLH-Peptidkonjugat immunisiert. Das gewonnene Antiserum wurde gegen das Antigen affinitätsgereinigt. Die Isolierung der spezifischen IgG-Fraktion aus Serum erfolgte mittels der jeweiligen Peptide, die dazu zunächst in der dem Fachmann geläufigen Weise auf einer Affinitätssäule immobilisiert wurden. Auf diese Affinitätssäule wurde das jeweilige Antiserum gegeben, nichtspezifisch sorbierte Proteine wurden mit Puffer eluiert. Die spezifisch gebundene IgG-Fraktion wurde mit 0,2 M Glycin/HCl-Puffer pH 2,2 eluiert. Der pH-Wert wurde sofort mit einem 1M TRIS/HCl-Puffer pH 7,5 erhöht. Das Eluat mit der IgG-Fraktion wurde 1 : 1 (Volumen) mit gesättigter Ammoniumsulfatlösung versetzt und 30 min bei +4°C zur Komplettierung der Fällung inkubiert. Der entstandene Niederschlag wurde bei 10.000 g zentrifugiert, vom Überstand befreit und in möglichst wenig PBS/TBS gelöst. Die entstandene Lösung wurde anschließend gegen PBS/TBS im Verhältnis 1 : 100 (Volumen) dialysiert. Die Antikörper wurden auf eine Konzentration von ca. 100 $\mu$g IgG/ml eingestellt. Die so aufgereinigten PARP3 Antikörper hatten eine hohe Spezifität gegenüber PARP3. Während mausPARP3 gut erkannt wurde, war keine Kreuzreaktion mit PARP1 oder PARP2 zu beobachten.

Beispiel 4: Analyse der Gewebeverteilung mittels Immunoblots (Western Blot)

Die Gewebeverteilung auf Proteinebene wurde für PARP2 und PARP3 ferner durch Immunoblot (Western-Blot) Analyse untersucht.

Aufbereitung der Maus-Gewebe für Proteingele:

Gewebe oder Zellen wurden mittels Potter oder Ultra-Turrax homogenisiert. Dazu wurden 0.5g Gewebe (oder Zellen) in 5ml Puffer (10 mM Tris-HCl pH7.5, 1 mM EDTA, 6 mM $MgCl_2$), eine Tablette Proteasen Inhibitoren-Cocktail (Boehringer Mannheim, Best.Nr.: 1836153) und Benzonase (Reinheitsgrad I, MERCK) 30 min. bei 37°C inkubiert. Es wurden Gewebeproben aus der Maus für Herz, Lunge, Leber, Milz, Niere, Darm, Muskel, Gehirn und für humane embryonale Nierenzellen (HEK293, human embryonal kidney) hergestellt.

Protein-Gele:

Für Protein-Gele wurde das NuPAGE-System der Firma NOVEX gemäß Vorschrift verwendet. Zum Einsatz kamen Polyacrylamidgele (NuPAGE 4-12% BisTris, NOVEX NP 0321), Laufpuffer (MES-Running Buffer, NOVEX NP 0002), Anti-Oxidant (NOVEX NP 0005), Protein-Größenstandard (Multi Mark Multi Colored Standard, NOVEX LC 5725), Probenpuffer (NuPAGE LDS Sample Buffer (4X), NOVEX NP 0007). Die Laufzeit der Gele betrug 45 Minuten bei einer Spannung von 200V.

Western Blot:

Western Blots wurden mit dem System der Firma NOVEX nach Vorschrift durchgeführt. Verwendet wurde eine Nitrocellulose-Membran (Nitrocellulose Pore size 45 μm, NOVEX LC 2001). Die Transferzeit betrug 1 Stunde bei einer Stromstärke von 200mA. Der Transferpuffer bestand aus 50 ml Transferpuffer Konzentrat (NOVEX NP 0006), 1 ml Anti-Oxidant (NOVEX NP 0002), 100 ml Methanol p.a. und 849 ml $H_2O$ bidest.

Neben den so hergestellten Blots wurden zudem vorgefertigte Blots, z.B. der Firma Chemicon (Mouse Brain Blot, Chemicon, Katalog Nr.: NS 106 mit den Geweben 1. Frontal Cortex, 2. Posterior Cortex, 3. Cerebellum, 4. Hippocampus, 5. Olfactory bulb, 6. Striatum, 7. Thalamus, 8. Mittelhirn, 9. Entorhinal Cortex, 10. Pons, 11. Medulla, 12. Rückenmark) verwendet.

Antikörperreaktion gegen PARP3:

Die Western-Blots wurden mindestens 2 Stunden in TBST (TBS + 0,3 % Tween 20) mit 5% Trockenmilchpulver blockiert (TBS: 100 mM Tris pH 7,5, 200 mM NaCl). Die Antikörperreaktion mit dem primären Antikörper (1:1000 Verdünnung) erfolgte für mindestens 2 Stunden bei Raumtemperatur oder über Nacht bei 4 °C unter leichtem Schütteln (Überkopfschüttler) in TBST mit 5% Trockenmilchpulver (siehe oben). Anschließend wurde dreimal für 5 Minuten in TBST gewaschen. Die Inkubation mit dem sekundären Antikörper (anti-Rabbit IgG, Peroxidase gekoppelt, SIGMA A-6154, Verdünnung 1:2.000) erfolgte 1 Stunde in TBST mit 5% Trockenmilchpulver. Anschließend wurde wie oben dreimal je 5 Minuten gewaschen. Es folgte die Detektion basierend auf Chemilumineszenz mit dem SUPER BLAZE Kit (Pierce, Signal BLAZE Chemiluminescent Substrate 34095) nach Angabe der Herstellers. Verwendet wurde der "Lumi-Film" (Chemiluminescent Detection Film, Boehringer Best.Nr.: 1666916). Die Filme wurden ca. 2 min. entwickelt (Röntgenentwicklerkonzentrat, ADEFO-Chemie GmbH), gewässert, ca. 4 min. fixiert (Acidofix 85 g/l /AGFA), gewässert und abschließend getrocknet.

Beispiel 5: Herstellung der Enzyme

Humanes PARP1 wurde zum Vergleich rekombinant im Baculovirus-System in der dem Fachmann geläufigen Weise exprimiert und wie beschrieben (Shah et al., Analytical Biochemistry 1995, 227, 1-13) partiell aufgereinigt. Rinder PARP1 in einer 30-50%igen Reinheit (c= 0,22 mg/ml, spez. Aktivität 170 nmol ADP-ribose/min/mg Gesamtprotein bei 25 °C) wurde von BIOMOL (Best.-Nr. SE-165) bezogen. Humanes und Maus PARP2 und PARP3 wurden rekombinant im Baculovirus-System (Bac-to-Bac System, BRL LifeScience) exprimiert. Dazu wurden die entsprechenden cDNAs in den pFASTBAC-1-Vektor kloniert. Nach Herstellung von rekombinanter Baculovirus-DNA durch Rekombination in E. coli, erfolgte Transfektion von Insektenzellen (Sf9 oder High-Five) mit den entsprechenden rekombinanten Baculovirus-DNAs. Die Expression der entsprechenden Proteine wurde durch Western-Blot-Analyse verifiziert. Virenstämme wurden in der dem Fachmann geläufigen Weise amplifiziert. Größere Mengen rekombinanter Proteine wurden durch Infektion von 500 ml Insektenzellkultur (2 x $10^6$ Zellen/ml, mit Viren in einer MOI (multiplicity of infection; Verhältnis von Viren zu Zellen) von 5-10 infiziert und 3 bis 4 Tage inkubiert) hergestellt. Anschließend wurden die Insektenzellen durch Zentrifugation pelletiert und die Proteine aus dem Pellet aufgereinigt.

Die Aufreinigung erfolgte durch klassische, dem Fachmann geläufige Methoden der Proteinreinigung unter Detektion der Enzyme mit entsprechenden spezifischen Antikörpern. Teilweise wurden die Proteine auch über eine 3-Aminobenzamid/Affinitätssäule wie beschrieben (Burtscher et al., Anal Biochem 1986, 152:285-290) affinitätsgereinigt.

Die Reinheit betrug >90%.

Beispiel 6: Testsysteme für die Bestimmung der Aktivität PARP2 und PARP3 und der Inhibitorischen Wirkung von Effektoren auf PARP1, PARP2 und PARP3.

a) Herstellung von Antikörpern gegen Poly-(ADP-ribose)

Als Antigen zur Generierung von Anti-Poly-(ADP-ribose) Antikörpern kann Poly-(ADP-ribose) verwendet werden. Die Herstellung von Anti-Poly-(ADP-ribose) Antikörpern ist in der Literatur beschrieben. (Kanai Y et al. (1974) Biochem Biophys Res Comm 59:1, 300-306; Kawamaitsu H et al. (1984) Biochemistry 23, 3771-3777; Kanai Y et al. (1978) Immunology 34, 501-508).

Unter anderem wurden verwendet: Anti Poly-(ADP-ribose)-Antikörper (polyklonales Antiserum, Kaninchen), BIOMOL; Best.-Nr. SA-276. Anti Poly-(ADP-ribose)-Antikörper (monoklonal, Maus; Klon 10H; Hybridomaüberstand, affinitätsgereinigt).

Die Antiseren oder aus Hybridomakulturüberstand gewonnenen monoklonalen Antikörper wurden durch eine Protein-A-Affinitätschromatographie in der dem Fachmann geläufigen weise aufgereinigt.

b) ELISA

Materialien:

ELISA Farbreagenz: TMB-Fertigmix SIGMA T-8540

Eine 96-well Mikrotiterplatte (FALCON Micro-Test III™ Flexible Assay Plate, # 3912) wurde mit Histonen (SIGMA, H-7755) beschichtet. Histone wurden hierfür in Carbonatpuffer (0.05M $Na_2HCO_3$; pH 9.4) in einer Konzentration von 50 µg/ml gelöst. Die einzelnen Wells der Mikrotiterplatte wurden mindestens 2 Stunden bei Raumtemperatur oder über Nacht bei 4 °C mit je 150 µl dieser Histon-Lösung inkubiert. Anschließend werden die Wells durch Zugabe von 150 µl einer 1%igen BSA-Lösung (SIGMA, A-7888) in Carbonatpuffer für 2 Stunden bei Raumtemperatur blockiert. Es folgen drei Waschschritte mit Waschpuffer (0,05% Tween10 in 1x PBS; PBS (Phosphate buffered saline; Gibco, Best.-Nr. 10010): 0.21g/l $KH_2PO_4$, 9g/l NaCl, 0.726g/l $Na_2HPO_4 \cdot 7H_2O$, pH 7.4). Waschschritte wurden durchweg mit einem Mikrotiterplatten-Waschgerät durchgeführt (Mikrotiterplatten-Wäscher "Columbus", SLT-Labinstruments, Österreich).

Für die Enzymreaktion wurden eine Enzymreaktionslösung und eine Substratlösung jeweils als "Pre-Mix" benötigt. Die absolute Menge dieser Lösungen richtete sich nach der Anzahl der vorgesehenen Test-Wells.

Zusammensetzung der Enzymreaktionslösung pro Well:

- 4 µl PARP-Reaktionspuffer (1M Tris-HCl pH 8.0, 100mM $MgCl_2$, 10mM DTT)
- 20ng PARP1 (human oder bovin) oder 8ng PARP2 (Human oder Maus)
- 4 µl aktivierte DNA (1 mg/ml; SIGMA, D-4522)
- ad 40 µl $H_2O$

Zusammensetzung der Substrat-Lösung pro Well:

- 5 µl PARP-Reaktionspuffer (10x)
- 0.8 µl NAD-Lösung (10mM, SIGMA N-1511)
- 44 µl $H_2O$

Inhibitoren wurden in 1x PARP-Reaktionspuffer gelöst. DMSO, das gelegentlich zum Lösen von Inhibitoren in höheren Konzentrationen verwendet wurde, war bis zu einer Endkonzentration von 2% unproblematisch. Für die Enzymreaktion wurden 40 µl der Enzymreaktionslösung pro Well vorgelegt und mit 10 µl Inhibitor-Lösung für 10 Minuten inkubiert. Anschließend wurde die Enzymreaktion durch Zugabe von 50 µl Substrat-Lösung pro Well gestartet. Die Reaktion wurde 30 Minuten bei Raumtemperatur durchgeführt und anschließend durch dreimaliges Waschen mit Waschpuffer gestoppt.

Als primäre Antikörper wurden spezifische Anti-Poly-(ADP-ribose) Antikörper in einer 1:5000 Verdünnung eingesetzt. Die Verdünnung erfolgte in Antikörper-Puffer (1% BSA in PBS; 0.05% Tween20). Die Inkubationszeit für den primären Antikörper betrug eine Stunde bei Raumtemperatur. Nach anschließendem dreimaligem Waschen mit Waschpuffer erfolgte eine einstündige Inkubation bei Raumtemperatur mit dem sekundärem Antikörper (Anti-Maus-IgG, Fab-Fragmente, Peroxidase gekoppelt, Boehringer Mannheim , Best.-Nr. 1500.686; Anti-Rabbit-IgG, Peroxidase gekoppelt, SIGMA, Best.-Nr. A-6154) in einer 1:10000 Verdünnung in Antikörperpuffer. Nach dreimaligem Waschen mit Waschpuffer folgte die Farbreaktion unter Verwendung von 100 µl Farbreagenz (TMB-Fertigmix, SIGMA) pro Well für ca. 15 min. bei Raumtemperatur. Die Farbreaktion wurde durch Zugabe von 100 µl 2M $H_2SO_4$ gestoppt. Danach wurde sofort im ELISA-Platten-Lesegerät ("Easy Reader" EAR340AT, SLT-Labinstruments, Österreich) gemessen (450nm gegen 620nm). Das Messprinzip ist schematisch in Figur 6 dargestellt.

Für die Ermittlung des $K_i$-Wertes eines Inhibitors wurden verschiedene Konzentrationen zur Erstellung einer Dosis-Wirkungskurve herangezogen. Für eine bestimmte Inhibitorkonzentration werden 3fach-Werte erhoben. Arithmetische Mittelwerte werden mit Microsoft© Excel ermittelt. Die $IC_{50}$-Bestimmung erfolgt mit der Microcal© Origin Software (Vers. 5.0) ("Sigmoidal Fit"). Umrechnung der so berechneten $IC_{50}$-Werte auf $K_i$-Werte erfolgte durch Verwendung von "Eich-Inhibitoren". Die "Eich-Inhibitoren" wurden bei jeder Analyse mitgemessen. Der $K_i$-Werte der "Eich-Inhibitoren" wurde im gleichen Testsystem durch Dixon-Diagramm Analyse in der dem Fachmann geläufigen Weise ermittelt.

b) HTRF-(Homogenous time-resolved fluorescence) Assay

Beim erfindungsgemäßen HTRF-PARP-Assay werden Histone als Zielproteine der Modifikation durch PARP indirekt mit einem XL665-Fluorophor markiert. Der Anti-Poly-(ADP-ribose)-Antikörper wird direkt mit einem Europium-Kryptat markiert (Anti-PAR-Kryptat). Befindet sich das XL665-Fluorophor in einer unmittelbaren räumlichen Nähe, die durch eine Bindung an die Poly-(ADP-ribose) am Histon gewährleistet wird, dann ist eine Energieübertragung möglich. Die Emission bei 665 nm ist somit direkt proportional zu der Menge an gebundenem Antikörper, der wiederum der Poly-(ADP-ribose) Menge entspricht. Somit entspricht das gemessene Signal der PARP Aktivität. Das Meßprinzip ist schematisch in Figur 7 dargestellt. Die verwendeten Materialien sind, wenn nicht ausdrücklich angegeben, identisch mit denen im ELISA (s.o.) verwendeten.

Histone wurden in Hepes-Puffer (50mM, pH=7.5) zu 3mg/ml gelöst. Biotinylierung erfolgte mit Sulfo-NHS-LC-Biotin (Pierce, #21335T). Ein molares Verhältnis von 4 Biotin-Molekülen pro Histon wurde verwendet. Die Inkubationszeit betrug 90 Minuten (RT). Anschließend wurden die biotinylierten Histone über eine G25 SF HR10/10 Säule (Pharmacia, 17-0591-01) in Hepes Puffer (50mM, pH=7.0) aufgereinigt, um überschüssiges Biotinylierungsreagenz zu entfernen. Der Anti-Poly-(ADP-ribose)-Antikörper wurde mittels bifunktionaler Kopplungsreagenzien mit Europium-Kryptat markiert (Lopez, E. et al., Clin. Chem. 39(2), 196-201 (1993); US-Patent 5,534,622). Die Reinigung erfolgte auf einer G25SF HR10/30 Säule.

Ein molares Verhältnis von 3.1 Kryptaten pro Antikörper wurde erzielt. Die Ausbeute betrug 25%. Die Konjugate wurden in Gegenwart von 0.1% BSA in Phosphatpuffer (0.1M, pH=7) bei -80°C gelagert.

Für die Enzymreaktion wurden pro Well zusammenpipettiert:

- 10 µl PARP-Lösung in PARP-HTRF-Reaktionspuffer (50mM Tris-HCl pH 8.0, 10mM MgCl2, 1mM DTT) mit 20ng PARP1 (human oder bovin) oder 8ng PARP2 (Human oder Maus)
- 10 µl aktivierte DNA in PARP-HTRF-Reaktionspuffer (50µg/ml)
- 10 µl biotinylierte Histone in PARP-HTRF-Reaktionspuffer (1.25µM)
- 10 µl Inhibitor in PARP-HTRF-Reaktionspuffer

Diese Reagenzien wurden 2 Minuten vorinkubiert, bevor die Reaktion durch Zugabe von

- 10 µl NAD-Lösung in PARP-HTRF-Reaktionspuffer (41 µM/ml) gestartet wurde. Die Reaktionszeit betrug 30 Minuten bei Raumtemperatur.
  Anschließend wurde die Reaktion durch Zugabe von
- 10 µl PARP-Inhibitor (25 µM, $K_i$=10nM) in "Revelation"-Puffer (100mM Tris-HCl pH 7.2, 0.2M KF, 0.05% BSA)

gestoppt.

Danach wurden zugegeben:

- 10 µl EDTA-Lösung (SIGMA, E-7889, 0.5M in $H_2O$)
- 100 µl Sa-XL665 (Packard Instruments) in "Revelation"-Puffer (15-31.25nM)
- 50 µl Anti-PAR-Kryptat in "Revelation"-Puffer (1.6-3.3nM).

Nach 30 Minuten (bis 4 Stunden) konnte dann gemessen werden. Die Messung erfolgte auf einem "Discovery HTRF Microplate Analyzer" (Canberra Packard Instruments). Die Berechnung der $K_i$-Werte erfolgte wie beim ELISA beschrieben.

Beispiel 7: Testsysteme zur Bestimmung der therapeutischen Wirksamkeit von PARP-Inhibitoren

Neue PARP-Inhibitoren können in relevanten pharmakologischen Modellen auf ihre therapeutische Wirksamkeit überprüft werden. Beispiele für einige geeignete Modelle sind dazu in Tabelle 1 aufgeführt.

| Krankheit | Modell | Literatur |
|---|---|---|
| Neurodegenerative Erkrankungen (Schlaganfall, Parkinson etc.) | NMDA-Exzitotoxizität in der Maus oder Ratte | Beschreibug siehe unten |
| Schlaganfall | Permanente MCAO("middle cerebral arterial occlusion") | Tokime, T. et al., J. Cereb. Blood Flow Metab., 18(9): 991-7, 1998. Guegan, C., Brain Research. Molecular Brain Research, 55(1): 133-40, 1998. |
| | Transiente, fokale MCAO in Ratte oder Maus | Eliasson MJL et al., Nat Med 1997, 3:1089-1095. Endres, M et al., J Cereb Blood Flow Metab 1997, 17:1143-1151. Takahashi K et al., J Cereb Blood Flow Metab 1997, 17:1137-1142. |
| Parkinsonsche Krankheit | MPTP (1-Methyl-4-phenyl-1,2,3,6-tetrahydro-pyridin) Toxizität in der Maus/Ratte | Cosi C, et al., Brain Res., 1998 809 (1):58-67. Cosi C, et al., Brain Res., 1996 729 (2):264-9. |
| Herzinfarkt | Koronargefäß-Okklusion an Ratte, Schwein oder Kaninchen | Richard V, et al., Br. J. Pharmacol 1994, 113, 869-876. Thiemermann C, et al., Proc Natl Acad Sci USA. 1997, 94(2):679-83. Zingarelli B, et al., Cardiovasc Res. 1997, 36(2):205-15. |
| | Langendorfherzmodell an Ratte oder Kaninchen | Beschreibung siehe unten |
| Septischer Schock | Endotoxin Schock in der Ratte Zymosan oder Carrageenan induziertes multiples Or-ganversagen in Ratte oder Maus | Szabo C, et al., J Clin Invest, 1997, 100(3):723-35. Szabo C, et al. J Exp Med. 1997, 186 (7):1041-9. Cuzzocrea S, et al. Eur J Pharmacol. 1998, 342(1):67-76. |
| Rheumatoide Arthritis | Adjuvant oder Collagen induzierte Arthritis in Ratte oder Maus | Szabo C, et al., Proc Natl Acad Sci U S A. 1998, 95(7):3867-72. |
| Diabetes | Streptozotocin und Alloxan induziert bzw. Obesity assoziert | Uchigata Y et al., Dia-betes 1983, 32: 316-318.Masiello P et al., Dia-betologia 1985, 28: 683-686.Shimabukuro M et al., J Clin Invest 1997, 100: 290-295. |
| Krebs | In vitro-Modell; s.u. | Schlicker et al., 1999, 75(1), 91-100. |

a) Modell der NMDA-Exzitotoxizität

Glutamat ist der wichtigste exzitorische Neurotransmitter im Gehirn. Unter normalen Bedingungen wird Glutamat in den synaptischen Spalt ausgeschüttet und stimuliert die post-synaptischen Glutamat-Rezeptoren, spezifisch die Glutamat-Rezeptoren vom "NMDA"- und "AMPA"-Typ. Diese Erregung spielt eine bedeutende Rolle bei zahlreichen Hirnfunktionen einschleißlich Lernen, Gedächtnis und motorische Kontrolle.

Unter Bedingungen der akuten und chronischen Neurodegeneration (z.B. Schlaganfall) erfolgt jedoch ein starker Anstieg der präsynaptischen Glutamat-Ausschüttung, was eine exzessive Stimulation der Rezeptoren zur Folge hat. Dies führt zum Zelltod der entsprechend erregten Zellen. Bei einer Reihe von neurologischen Krankheiten oder psychischen Störungen treten diese erhöhten Glutamat-Aktivitäten auf, die zu Zuständen von Übererregungen oder toxischen Effekten im zentralen Nervensystem (ZNS) aber auch im peripheren Nervensystem führen. Somit ist Glutamat in eine Vielzahl neurodegenerativen Erkrankungen involviert, insbesondere neurotoxischen Störungen nach Hypoxie, Anoxie, Ischämie und nach Läsionen, wie sie nach Schlaganfall und Trauma auftreten, Schlaganfall, Alzheimersche Erkrankung, Huntington-Erkrankung, Amyotrophe Laterale Sklerose (ALS; "Lou Gehrigs"-Erkrankung), Kopf-Trauma, Rückenmarks-Trauma, periphere Neuropathien, AIDS Demenz und die Parkinsonsche Krankheit. Eine weitere Erkrankung in der Glutamat-Rezeptoren von Bedeutung sind, ist Epilepsie. (vgl.

Brain Res Bull 1998; 46(4):281-309, Eur Neuropsychopharmacol 1998, 8(2):141-52.).

Glutamat vermittelt seine Effekte über verschiedene Rezeptoren. Einer dieser Rezeptoren wird nach einem spezifischen Agonisten NMDA-(N-Methyl-D-Aspartat)-Rezeptor genannt. (Arzneim.Forschung 1990, 40, 511-514; TIPS, 1990, 11, 334-338; Drugs of the Future 1989, 14, 1059-1071). N-Methyl-D-aspartat ist ein starker Agonist einer bestimmten Klasse von Glutamat-Rezeptoren ("NMDA"-Typ). Die Erregung des NMDA-Rezeptors führt zum Calcium-Einstrom in die Zelle und zur Erzeugung von Radikalen. Die Radikale führen zur DNA-Schädigung und zur Aktivierung von PARP. PARP wiederum verursacht durch eine Depletion der Zelle an energiereichen Phosphaten (NAD und ATP) den Zelltod. Dies erklärt die Toxizität von NMDA. Behandlung von Tieren mit NMDA kann daher als Modell gesehen werden für die oben genannten Erkrankungen, bei denen Exzitotoxizität eine Rolle spielt.

Aufgrund der Bedeutung der Glutamat-Rezeptoren in der Neurodegeneration waren viele pharmakologische Ansätze bislang darauf gerichtet, eben diese Rezeptoren spezifisch zu blockieren. Aufgrund ihrer Bedeutung in der normalen Reizleitung haben sich diese Ansätze jedoch als problematisch erwiesen (Nebenwirkungen). Zudem ist die Erregung der Rezeptoren ein sehr schnell erfolgendes Ereignis, so daß die Applikation der Rezeptoren oft zu spät kommt ("Zeitfenster"-Problem). Der Bedarf an neuen Wirkprinzipien und Inhibitoren der NMDA-bedingten Neurotoxizität ist daher hoch.

Der Schutz gegen zerebrale Übererregung durch exzitatorische Aminosäuren (NMDA-Antagonismus an der Maus) kann als hinreichender Beleg der Wirksamkeit eines pharmakologischen Effektors von PARP in Erkrankungen beruhend auf Exzitotoxizität gelten. Durch intrazerebrale Applikation von exzitatorischen Aminosäuren EAA (Excitatory Amino Acids) wird eine so massive Übererregung induziert, daß diese in kurzer Zeit zu Krämpfen und zum Tod der Tiere (Maus) führt.

Im vorliegenden Fall wurden 10 $\mu$l einer 0.035%igen wäßrigen NMDA-Lösung 120 Minuten nach intraperitonealer Gabe (ip.-Gabe) der zu prüfenden Substanz einseitig intracerebroventrikulär appliziert. Durch systemische, z.B. intraperitoneale, Gabe von zentralwirksamen Wirkstoffen lassen sich diese Symptome hemmen. Da die exzessive Aktivierung von EAA-Rezeptoren des Zentralnervensystems in der Pathogenese verschiedener neurologischer Erkrankungen eine bedeutende Rolle spielt, kann aus dem nachgewiesenen EAA-Antagonismus in vivo auf eine mögliche therapeutische Verwendbarkeit der Substanzen gegen derartige ZNS-Erkrankungen geschlossen werden. Als Maß für die Wirksamkeit der Substanzen wurde ein ED50-Wert bestimmt, bei dem 50% der Tiere durch eine festgelegte Dosis von NMDA durch die vorangegangene ip-Gabe der Meßsubstanz syptomfrei werden.

b) Langendorfherzmodell (Model für Herzinfarkt)

Für den Test wurden männliche Sprague-Dawley Ratten (Körpergewicht 300-400 g; Herkunft Janvier, Le Genest-St-Isle, France) verwendet. Die Ratten wurden oral mittels Schlundsonde mit der Wirksubstanz oder Placebo behandelt (Volumen: 5 ml/kg). 50 Minuten später wird Heparin intraperitoneal appliziert (Liquemin N Roche, 125 IU/animal in 0.5 ml). Die Tiere werden mit Inactin® T133 (Thiobetabarbital Natrium, 10 %) anaesthetisiert, auf dem Operationstisch fixiert, tracheotomisiert and mit einer "Harvard Atempumpe" (40 Schläge/min, 4.5 ml/Schlag) beatmet. Der Thorakotomie folgt eine sofortige Katheterisierung der Aorta, Exstirpation des Herzens und sofortige retrograde Perfusion. Die Herzen wurden mit einem konstanten Druck von 75 mmHg perfundiert, was mittels einer "Gilson Minipuls 2 Perfusionspumpe" erreicht wird. Zusammensetzung des Perfusats (mmol/1): NaCl 118, KCl 4.7, $CaCl_2$ x 2 $H_2O$ 2.52, $MgSO_4$ x 7 $H_2O$ 1.64, $NaHCO_3$ 24.88, $KH_2PO_4$ 1.18, Glukose 11. Die Temperatur wird während des gesamten Experimentes auf 37 °C gehalten. Funktionelle Parameter wurden mittels eines "Gould 4-Kanal-Schreibers" kontinuierlich aufgezeichnet. Gemessen wurden der linksventrikuläre Druck (LVP; mmHg), LVEDP (mmHg), Enzymfreisetzung (Creatinkinase, mU/ml/g), Koronarflußrate (ml/min), HR (Pulsfrequenz, $min^{-1}$). Der links-ventrikuläre Druck wurde mittels eines flüssigkeitgefüllten Latexballons und eines Druckaufnehmers-Statham23 Db gemessen. Das Volumen des Ballons wurde anfänglich angepaßt, um einen LVEDP (left ventricular end diastolic pressure) von ca.12 mmHg zu erreichen. $Dp/dt_{max}$ (maximale Pumpkraft) wird aus dem Drucksignal mittels eines Differentiatormoduls abgeleitet. Die Herzfrequenz wurde aus dem Drucksignal errechnet. Die Flußrate wurde mittels Tropfenzähler (BMT Messtechnik GmbH Berlin) bestimmt. Nach einer Äquilibrierzeit von 20 Minuten, wurden die Herzen einer 30 minütigen globalen Ischämie unterworfen, die durch den Stop der Perfusatzufuhr bei Temperierung auf 37 °C realisiert wird. Während der folgenden Reperfusionsperiode von 60 Minuten wurden Proben des Perfusats nach 3, 5, 10, 15, 30, 45 und 60 min zur Analyse der Creatinkinase (CK) Aktivität entnommen. Mittelwerte und Standardabweichungen der gemessenen Parameter wurden statistisch analysiert (Dunnett Test). Die Signifikanzgrenze lag bei p=0.05.

Ähnlich wurde der versuch an Kaninchenherzen durchgeführt. Verwendet wurden männliche, weiße Neuseeländer Kaninchen (Bezug: Interfauna). Die Präparation der Herzen erfolgte wie oben im Rattenmodell beschrieben. Der Perfusionsdruck wurde auf maximal 60 mmHg, der Fluß auf ca 25ml/min eingestellt. Die Äquilibrierzeit betrug ca 30 min. Die Substanzapplikation erfolgte per Infusion direkt vor das Herz. 15 min nach Start der Infusion wurde eine 30 minütige globale Ischämie durch stop-flow unter Temperierung des

Herzens gesetzt. Es folgt eine 30 minütige Reperfusion. Abnahme von Perfusat für Untersuchung von CK-Aktivität erfolgte vor Substanzgabe, nach 15 min, und zu verschiedenen Zeitpunkten (5, 10, 15, 20, 30 min) während der Reperfusion. Gemessen wurden die Parameter: LVP (mmHg), LVEDP, LVdp/dt, PP (mmHg), HR (Pulsfrequenz; Schläge/min), CK-Aktivität (U/min/g Herzgewicht).

c) Tiermodell für akutes Nierenversagen

Untersucht wurde die protektive Wirkung von PARP-Inhibitoren bei einer intravenösen Gabe (4 Tage) auf die Nierenfunktion von Ratten mit postischämischem akutem Nierenversagen.

Verwendet wurden männliche Sprague-Dawley Ratten (ca. 330g bei Versuchsbeginn; Züchter: Charles River). Es wurden 10-15 Tiere pro Versuchsgruppe eingesetzt. Die Applikation von Wirksubstanz/Placebo erfolgte kontinuierlich mit osmotischer Mikropumpe in die v. femoralis. Blutabnahme erfolgte orbital (1,5ml Vollblut) unter Inhalationsnarkose mit Enfluran (Ethrane Abbott, Wiesbaden).

Nach Ermittlung der Vorwerte (Blutentnahme) und Bestimmung der in 24h ausgeschiedenen Urinmenge wurden die Ratten narkotisiert ("Nembutal", Pentobarbital-Natrium, Sanofi CEVA; 50mg/kg i.p., Injektionsvolumen 1,0 ml/kg) und auf einem heizbaren OP-Tisch (37°C) befestigt. Es folgte Heparingabe (Liquemin N, Roche) 125 IU/kg i.v. in die Vena caudalis. Der Bauchraum wurde eröffnet und die rechte Niere freigelegt. Die abzweigende Nierenarterie wurde freigelegt und mit Bulldogklemmen (nach Diefenbach 38mm) von oben her abgeklemmt. Die linke Nierenarterie wurde ebenfalls freigelegt und abgeklemmt (von oben her, ca.1/2 Strecke zur Niere). Während der Operation wurde eine osmotische Mikropumpe in die V. femoralis implantiert. Der Darm wurde wieder eingefügt und der Flüssigkeitsverlust mit lauwarmer 0,9 % NaCl ausgeglichen. Die Tiere wurden mit einem feuchtem Tuch abgedeckt und unter Rotlicht warm gehalten. Nach 40 min wurde das Aussehen der Nieren dokumentiert und rechts, danach links die Klammer entfernt. Der Darm wurde zurückgelegt und 2 Tropfen Antibiotikum (Tardomyocel, Bayer) wurden zugegeben. Die Bauchdecke wurde mit sterilem Catgut (Ethicon Nr.4) geschloßen und nochmals mit 1 Tropfen Antibiotikum behandelt. Die Oberhaut wurde mit sterilem Ethibond Exel (Ethicon) Nr.3/0 genäht und die Naht mit Wundspray Nebacetin N (Yamanouchi) besprüht. Ein Zehntel Tagesdosis Wirkstoff/Placebo wird als Bolus i.v. geben.

Für die Versuchstage 1, 2 und 4 erfolgte Proben- und Blutentnahme für die Untersuchung klinisch chemischer Parameter in Serum und Urin: Na, K, Creatinin, Protein (nur in Urin). Ferner wurden Futter- und Wasserverbrauch, Körpergewicht und Urinvolumen festgehalten. Nach 14 Tagen wurden die Tiere getötet und die Nieren begutachtet.

Für die Auswertung wurden alle Tiere, die während des Versuchs an einem Infarkt verstarben, bzw bei Sektion am Tag 14 einen Infarkt aufwiesen, ausgeschlossen. Berechnet wurden die Creatinin-Clearance und die fraktionale Natriumexkretion als Nierenfunktionsparameter unter Vergleich von behandelten Tieren mit Kontrolle und Sham.

d) In vitro-Modell für die Radiosensitisierung (Tumortherapie)

MCF-7-Zellen (humanes Brustkarzinom) wurden in Dulbecco's modifiziertem Eagles Medium mit 10% hitzeinaktiviertem FCS und 2 mM L-Glutamin kultiviert. Zellen wurden über Nacht ausgesäht in Zelldichten von 100, 1000 oder 10000 Zellen pro Well in einer 6-WellPlatte und anschließend einer ionisierenden Strahlung mit einer Dosis in einem Bereich von 0 bis 10 Gy ($^{137}$Cs, Shepard Mark, Model I-68A, Dosisrate von 3,28 Gy/min) ausgesetzt. 10 Tage nach der Bestrahlung wurde der Versuch ausgewertet, wobei Kolonien mit 50 Zellen als positiv gezählt wurden.

e) Schlaganfallmodell (Fokale cerebrale Ischämie; MCA (mittlere cerebrale Arterie) Okklusion an der Ratte

Eine fokale Ischämie wurde durch Kauterisierung der rechten distalen MCA in Sprague-Dawley- oder Long-Evans-Ratten realisiert. Die Ratten können vor oder nach dem Beginn der MCA-Okklusion mit Modulatoren der erfindungsgemäßen Proteine behandelt werden. In der Regel werden Dosierungen von 1-10 mg/kg gewählt (Bolus-Applikation), gegebenenfalls gefolgt von einer Dauerinfusion von 0,5-5 mg/kg/h. Die Ratten werden mit Halothan in einer Mischung aus 70 % Stickstoff und 30 % Sauerstoff anesthesiert (4 % zur Einleitung und 0,8-1,2 % während der Operation). Die Körpertemperatur wurde permanent rektal gemessen und auf 37,5 °C $\pm$ 0,5 °C mittels einer regulierbaren Heizdecke konstant gehalten. Ferner wurden gegebenenfalls über einen Schwanzvenenkatheter der arterielle Druck, arterieller pH, $PaO_2$ und $PaCO_2$ gemessen. Die fokale Ischämie wurde anschließend gemäß der Methode von Chen et al. (Stroke 17: 738-743; 1986) oder Liu et al. (Am. J. Physiol. 256: H589-593; 1989) mittels permanenter Kauterisierung des distalen Teils der rechten MCA durchgeführt. Nach Beendigung der Operation wurden die Tiere in einer warmen Umgebung für weitere 24 Stunden gehalten. Anschließend werden sie unter Verwendung von $CO_2$ getötet und dekapitiert. Die Gehirne werden unverzüglich entnommen, schockgefroren (Trockeneis oder flüssiger Stickstoff) und bei -80 °C gelagert. Die Hirne werden in 0,02 mm dicke Scheiben geschnitten, wobei jeder zwanzigste Schnitt für die anschließende Analyse herangezogen wird. Die entsprechenden Schnitte werden mit Kresylviolett gefärbt (Nissl-Färbung). Alternativ kann mit TTC (2,3,5-Triphenyltetraztoliumchlorid) gefärbt werden. Das Infarktvolumen kann anschließend unter dem Mikroskop ausgewertet werden. Zur exakten Quantifizierung kann eine computerunterstützte Bildauswertungssoftware herangezogen werden (J. Cereb. Blood Flow Metabol. 10: 290-293; 1990).

f) Septischer Schock

Gruppen von jeweils 10 männlichen C57/BL-Mäusen (Körpergewicht 18-20 g) werden mit LPS (Lipopolysaccharid aus E. coli, $LD_{100}$ = 20 mg/Tier i. v.) plus Galactosamin (20 mg/Tier i. v.) behandelt. Die zu testende Substanz wird i. p. oder i. v. über drei aufeinander folgende Tage appliziert (zum Beispiel 1-10 mg/kg), wobei die erste Dosis 30 Minuten nach der LPS-Applikation gegeben wird. Die Sterblichkeitsquote wird alle 12 h ermittelt. Alternativ kann die Substanz auch in mehreren über die Tage verteilten Dosen appliziert werden.

g) Bestimmung der veränderten Genexpression in alternden Zellen

Die Alterung von Zellen wird durch Änderung von Zellkulturmedien von komplettem Medium auf Medium mit einer reduzierten Serumkonzentration simuliert und anschließend mittels quantitativer PCR oder Northern Blot analysiert (Linskens et al., Nucleic Acids Res. 1995; 23(16): 3244-51). Als typische Marker für die Alterung der Haut können z. B. Kollagen oder Elastin fungieren. Zum Einsatz kommen humane Fibroblasten oder Fibroblastenzelllinien, die die Alterung der Haut wiedergeben können. Modulatoren der erfindungsgemäßen Proteine werden in das Medium gegeben und ihre Auswirkung auf die Änderung der Genexpression wird beobachtet. Eine erhöhte Elastinproduktion in Zellen mit durch die Modulatoren reduziertem Alterungsprozess kann beobachtet werden.

## Patentansprüche

**1.** Poly(ADP-ribose)polymerase (PARP)-Homologes und die funktionalen Äquivalente davon, **gekennzeichnet durch** eine Aminosäuresequenz, welche

a) eine funktionale $NAD^+$-Bindungsdomäne und
b) kein Zink-Finger-Sequenzmotiv der allgemeinen Formel

$$CX_2CX_mHX_2C$$

aufweist, worin
m für einen ganzzahligen Wert von 28 oder 30 steht und die Reste X unanbhängig voneinander für eine beliebige Aminosäure stehen;

wobei die funktionale $NAD^+$-Bindungsdomäne das Sequenzmotiv $PX_n$(S/T)$GX_3$GKGIYFA umfasst, worin n für einen ganzzahligen Wert von 1 bis 5 steht und die Reste X unanbhängig voneinander für eine beliebige Aminosäure stehen.

**2.** PARP-Homologes nach Anspruch 1, **dadurch gekennzeichnet, daß** die funktionale $NAD^+$-Bindungsdomäne eines der folgenden allgemeinen Sequenzmotive umfaßt:

(S/T)XGLR(I/V)$XPX_n$(S/T)$GX_3$GKGIYFA oder
LLWHG(S/T)$X_7$IL(S/T)XGLR(I/V)$XPX_n$(S/T)$GX_3$GKGIYFA$X_3$SKSAXY

worin n und X wie in Anspruch 1 definiert sind.

**3.** PARP-Homologes nach einem der vorherigen Ansprüche, umfassend wenigstens ein weiteres der folgenden Teilsequenz-Motive:

$LX_9NX_2YX_2QLLX(D/E)X_{10/11}WGRVG$,
$AX_3FXKX_4KTXNXWX_5FX_3PXK$,
$QXL(I/L)X_2IX_9MX_{10}PLGKLX_3QIX_6L$,
$FYTXIPHXFGX_3PP$; und
$KX_3LX_2LXDIEXAX_2L$,

worin die Reste X unabhängig voneinander für eine beliebige Aminosäure stehen.

**4.** PARP-Homologes nach einem der vorherigen Ansprüche, ausgewählt unter humanen PARP-Homologen, **gekennzeichnet durch** die Aminosäuresequenz gemäß SEQ ID NO:2 oder SEQ ID NO:4 oder 6; oder murinen PARP-Homologen, **gekennzeichnet durch** die Aminosäuresequenz gemäß SEQ ID NO:8 oder SEQ ID NO:10.

**5.** Bindungspartner für PARP-Homologe nach einem der vorherigen Ansprüche, ausgewählt unter Antikörpern und Fragmenten davon.

**6.** Nukleinsäure, umfassend

a) eine für wenigstens ein PARP-Homologes nach einem der Ansprüche 1 bis 4 kodierende Nukleotidsequenz oder die komplementäre Nukleotidsequenz davon;
b) Nukleotidesquenzen, die durch Entartung des genetischen Codes von den in a)definierten Nukleotidsequenzen abgeleitet sind, und für ein Protein mit Poly-ADP-ribosylierender Aktivität kodiert.

**7.** Nukleinsäure nach Anspruch 6, umfassend

a) die Nukleotide + 3 bis + 1715 gemäß SEQ ID NO:1;
b) die Nukleotide + 242 bis + 1843 gemäß SEQ ID NO:3;
c) die Nukleotide + 221 bis + 1843 gemäß SEQ ID NO:5;
d) die Nukleotide + 112 bis + 1710 gemäß SEQ ID NO:7; oder
e) die Nukleotide'+ 1 bis + 1584 gemäß SEQ ID NO:9.

**8.** Expressionskassette, enthaltend unter genetischer Kontrolle wenigstens einer regulativen Nukleotidsequenz wenigstens eine Nukleotidsequenz gemäß einem der Ansprüche 6 und 7.

**9.** Rekombinanter Vektor, umfassend wenigstens eine Expressionskassette nach Anspruch 8.

**10.** Rekombinanter Mikroorganismus, enthaltend wenigstens einen rekombinanten Vektor nach Anspruch 9.

**11.** In vitro Nachweisverfahren für PARP-Inhibitoren **dadurch gekennzeichnet, daß** man

a) ein ungeträgertes oder geträgertes Poly-ADP-ribosylierbares Target mit einem Reaktionsgemisch inkubiert, umfassend

a1) ein PARP-Homologes nach einem der Ansprüche 1 bis 4,
a2) aktivierte DNA als PARP-Aktivator; und
a3) einen PARP-Inhibitor oder einen Analyten, in welchem man wenigstens einen PARP-Inhibitor vermutet;

b) die Poly-ADP-Ribosylierungsreaktion durchführt; und
c) die Poly-ADP-Ribosylierung des Targets qualitativ oder quantitativ bestimmt.

**12.** Verfahren nach Anspruch 11, **dadurch gekennzeichnet, dass** man das PARP-Homologe mit dem PARP-Aktivator und dem PARP-Inhibitor oder einen Analyten, in welchem man wenigstens einen PARP-Inhibitor vermutet, vorinkubiert, bevor man die Poly-ADP-Ribosylierungsreaktion durchführt.

**13.** Verfahren nach einem der Ansprüche 11 und 12, **dadurch gekennzeichnet, dass** das Poly-ADP-ribosylierbare Target ein Histon-Protein ist.

**14.** Verfahren nach einem der Ansprüche 11 bis 13, **dadurch gekennzeichnet, dass** man die Poly-ADP-Ribosylierungsreaktion durch Zugabe von $NAD^+$ startet.

**15.** Verfahren nach einem der Ansprüche 11 bis 14, **dadurch gekennzeichnet, dass** man die Poly-ADP-Ribosylierung des geträgerten Targets mit Anti-Poly-(ADP-ribose)-Antikörper bestimmt.

**16.** Verfahren nach einem der Ansprüche 11 bis 14, **dadurch gekennzeichnet, dass** das nichtgeträgerte Target mit einem Akzeptor-Fluoorophor markiert ist.

**17.** Verfahren nach Anspruch 16, **dadurch gekennzeichnet, dass** man die Poly-ADP-Ribosylierung des nicht geträgerten Targets mit Anti-Poly-(ADP-ribose)-Antikörper bestimmt, der mit einem Donor-Fluorophor markiert ist, das zur Energieübertragung auf das Akzeptor-Fluorophor befähigt ist.

**18.** Verfahren nach einem der Ansprüche 16 und 17, **dadurch gekennzeichnet, dass** das Target biotinyliertes Histon ist und das Akzeptor-Fluorophor über Avidin oder Streptavidin daran gekoppelt ist.

**19.** Verfahren nach einem der Ansprüche 17 und 18, **dadurch gekennzeichnet, dass** der Anti-Poly-(ADP-ribose)-Antikörper ein Europium-Kryptat als Donor-Fluorophor trägt.

**20.** In vitro-Screeningverfahren auf Bindungspartner für ein PARP-Molekül, **dadurch gekennzeichnet, daß** man

a1 wenigstens ein PARP-Homologes nach einem der Ansprüche 1 bis 4 an einem Träger immobilisiert;
b1) das immobilisierte PARP-Homologe mit einem Analyten in Kontakt bringt, in welchem man wenigstens einen Bindungspartner vermutet; und
c1) an das immobilisierte PARP-Homologe gebundene Bestandteile des Analyten, gegebenenfalls nach einer Inkubationsphase, bestimmt;

oder

a2) einen Analyten, welcher wenigstens einen möglichen Bindungspartner für ein PARP-Molekül enthält, an einem Träger immobilisiert;
b2) den immobilisierten Analyten mit wenigstens einem PARP-Homologen nach einem der Ansprüche 1 bis 4 in Kontakt bringt, für welches man einen Bindungspartner sucht; und
c2) den immobilisierten Analyten, gegebenenfalls nach einer Inkubationsphase, auf die Bindung des PARP-Homologen untersucht.

**21.** Verfahren zur qualitativen oder quantitativen Bestimmung von PARP-Homologe nach einem der Ansprüche 1 bis 4 kodierenden Nukleinsäuren, **gekennzeichnet durch**

a) Inkubation einer biologischen Probe mit einer definierten Menge einer exogenen Nukleinsäure nach einem der Ansprüche 6 und 7, Hybridisierung unter stringenten Bedingungen, Bestimmung der hybridisierenden Nukleinsäuren und gegebenenfalls Vergleich mit einem Standard; oder
b) Inkubation einer biologischen Probe mit einem Paar von Oligonucleotid-Primern mit Spezifität für eine ein PARP-Homologes kodierende Nukleinsäure nach einem der Ansprüche 6 und 7, Amplifizierung der Nukleinsäure, Bestimmung des Amplifikationsprodukts und gegebenenfalls Vergleich mit einem Standard.

**22.** Verfahren zur qualitativen oder quantitativen Bestimmung eines PARP-Homologen nach einem der Ansprüche 1 bis 4, **gekennzeichnet durch**

a) Inkubation einer biologischen Probe mit einem für ein PARP-Homologes spezifischen Bindungspartner nach Anspruch 5,
b) Nachweis des Bindungspartner/PARP-Komplexes und gegebenenfalls
c) Vergleich des Ergebnisses mit einem Standard.

**23.** Verfahren nach Anspruch 22, **dadurch gekennzeichnet, daß** der Bindungspartner ein Antikörper oder ein bindendes Fragment davon ist, der gegebenenfalls eine detektierbare Markierung trägt.

**24.** Verfahren nach einem der Ansprüche 21 bis 23 zur Diagnostizierung von Energiedefizienz-vermittelten Erkrankungen.

**25.** Verfahren zur Bestimmung der Wirksamkeit von PARP-Inhibitoren, **dadurch gekennzeichnet daß** man

a) ein PARP-Homologes nach einem der Ansprüche 1 bis 4 mit einem Analyten inkubiert, welcher einen PARP-Inhibitor enthält; den Inhibitor gegebenenfalls wieder abtrennt; und
b) die Aktivität des PARP-Homologen, gegebenenfalls nach Zugabe von Substraten oder Cosubstraten, bestimmt.

**26.** Gentherapeutisches Mittel, **dadurch gekennzeichnet**, das es in einem gentherapeutisch akzeptablen Träger ein Nukleinsäurekonstrukt enthält, das

a) eine Antisense-Nukleinsäure gegen eine kodierende Nukleinsäure nach einem der Ansprüche 6 und 7; oder
b) ein Ribozym gegen eine Nukleinsäure nach einem der Ansprüche 6 und 7 umfasst; oder

kodiert.

**27.** Pharmazeutisches Mittel, enthaltend in einem pharmazeutisch akzeptablen Träger wenigstens ein PARP-Protein nach einem der Ansprüche 1 bis 4, wenigstens einen PARP-Bindungspartner nach Anspruch 5 oder wenigstens eine kodierende Nukleotidsequenz nach Anspruch 6 oder 7.

**28.** Verwendung von PARP-Bindungspartnern nach Anspruch 5 zur Herstellung eines pharmazeutischen Mittels zur Diagnose oder Therapie von Krankheitszuständen, die durch eine Energiedefizienz vermittelt sind.

## Claims

**1.** A poly-(ADP-ribose)-polymerase (PARP) homologue and functional equivalents thereof, **characterized by** an amino acid sequence comprising

a) a functional $NAD^+$ binding domain and
b) no zinc finger sequence motif of the general formula

$$CX_2CX_mHX_2C$$

wherein
m is an integer of 28 or 30 and
X is, independently at each occurrence, any amino acid residue;

wherein the functional $NAD^+$ binding domain comprises the motif

$PX_n(S/T)GX_3GKGIYFA$

wherein
n is an integer from 1 to 5 and
X is, independently at each occurrence, any amino acid residue.

**2.** The PARP homologue according to claim 1, **characterized in that** the functional $NAD^+$ binding domain comprises one of the following general sequence motifs:

$(S/T)XGLR(I/V)XPX_n(S/T)GX_3GKGIYFA$ or
$LLWHG(S/T)X_7IL(S/T)XGLR(I/V)XPX_n(S/T)GX_3GKGIYFAX_3SKSAXY$

wherein n and X are as defined in claim 1.

**3.** The PARP homologue according to any of the preceding claims, comprising at least one of the following partial sequences motifs:

$LX_9NX_2YX_2QLLX(D/E)X_{10/11}WGRVG$,
$AX_3FXKX_4KTXNXWX_5FX_3PXK$,
$QXL(I/L)X_2IX_9MX_{10}PLGKLX_3QIX_6L$,
$FYTXIPHXFGX_3PP$; and
$KX_3LX_2LXDIEXAX_2L$,

wherein X is, independently at each occurrence, any amino acid residue.

**4.** The PARP homologue according to any of the preceding claims, selected among the group consisting of human PARP homologues **characterized by** the amino acid sequences according to SEQ ID NO:2, SEQ ID NO:4 or 6; and murine PARP homologues, **characterized by** the amino acid sequences according to SEQ ID NO:8 or SEQ ID NO:10.

**5.** A binding partner for a PARP homologue according to any of the preceding claims, selected among the group consisting of antibodies and fragments thereof.

**6.** A nucleic acid, comprising

a) a nucleotide sequence encoding at least one PARP homologue according to any of claims 1 to 4, or the complementary nucleotide sequence thereof;
b) a nucleotide sequence derived from the nucleotide sequences defined in a) by virtue of the degeneracy of the genetic code, coding for a protein with poly-ADP ribosylation activity.

**7.** The nucleic acid according to claim 6, comprising

a) nucleotides +3 to +1715 according to SEQ ID NO:1;
b) nucleotides +242 to +1843 according to SEQ ID NO:3;
c) nucleotides +221 to +1843 according to SEQ ID NO:5;
d) nucleotides +112 to +1710 according to SEQ ID NO:7;
e) nucleotides +1 to +1584 according to SEQ ID NO:9.

**8.** An expression cassette, comprising at least one nucleotide sequence according to claim 6 or 7 under the genetic control of at least one regulatory nucleotide sequence.

**9.** A recombinant vector, comprising at least one expression cassette according to claim 8.

**10.** A recombinant microorganism, comprising at least one recombinant vector according to claim 9.

**11.** An in vitro diagnostic method for PARP inhibitors, **characterized in that** it comprises

a) incubating a carrier-coupled or non-carrier-coupled poly-ADP-ribosylable target with a reaction mixture, comprising

a1) a PARP homologue according to any one of claims 1 to 4,
a2) activated DNA as PARP activator; and
a3) a PARP inhibitor or an analyte suspected to comprise at least one PARP inhibitor;

b) performing the poly-ADP ribosylation reaction; and
c) qualitatively or quantitatively determining the target's poly-ADP ribosylation.

**12.** The method according to claim 11, **characterized in that** the PARP homologue is preincubated with the PARP activator and the PARP inhibitor or an analyte suspected to comprise at least one PARP inhibitor prior to performing the poly-ADP ribosylation reaction.

**13.** The method according to claim 11 or 12, **characterized in that** the poly-ADP-ribosylable target is a histone protein.

**14.** The method according to any of claims 11 to 13, **characterized in that** the poly-ADP ribosylation reaction is initiated by addition of $NAD^+$.

**15.** The method according to any of claims 11 to 14, **characterized in that** the poly-ADP ribosylation of the carrier-coupled target is determined using an anti-poly-(ADP-ribose) antibody.

**16.** The method according to any of claims 11 to 14, **characterized in that** the non-carrier-coupled target is labelled with an acceptor fluorophore.

**17.** The method according to claim 16, **characterized in that** the poly-ADP ribosylation of the non-carrier-coupled target is determined using an anti-poly-(ADP-ribose) antibody labelled with a donor fluorophore capable of transferring energy to the acceptor fluorophore.

**18.** The method according to claim 16 or 17, **characterized in that** the target is biotinylated histone and the acceptor fluorophore is coupled to it via avidine or streptavidine.

**19.** The method according to claim 17 or 18, **characterized in that** the anti-poly-(ADP-ribose) antibody carries a europium cryptate as donor fluorophore.

**20.** An in vitro screening method for binding partners for a PARP molecule, **characterized in that** it comprises

a1) immobilizing at least one PARP homologue according to any of claims 1 to 4 to a carrier;
b1) contacting the immobilized PARP homologue with an analyte suspected to comprise at least one PARP inhibitor; and
c1) determining the components of the analyte bound to the immobilized PARP homologue, optionally after a period of incubation;

or

a2) immobilizing an analyte suspected to comprise at least one PARP inhibitor to a carrier;
b2) contacting the immobilized analyte with at least one PARP homologue according to any of claims 1 to 4 which a binding partner is being sought for; and
c2) assaying the immobilized analyte for binding of the PARP homologue, optionally after a period of incubation.

**21.** A method for qualitatively or quantitatively determining nucleic acids encoding PARP homologues according to any one of claims 1 to 4, **characterized in that** it comprises

a) incubating a biological sample with a defined amount of an exogenous nucleic acid according to claim 6 or 7, hybridizing under stringent conditions, detecting the hybridizing nucleic acids and, optionally, comparing the result to a reference standard; or
b) incubating a biological sample with a pair of oligonucleotide primers with specificity for a nucleic acid encoding a PARP homologue according to claim 6 or 7, amplifying the nucleic acid, detecting the amplification product, and, optionally, comparing the result to a reference standard.

**22.** A method for qualitatively or quantitatively determining a PARP homologue according to any one of claims 1 to 4, **characterized in that** it comprises

a) incubating a biological sample with a binding partner for a PARP homologue according to claim 5,
b) detecting the binding partner/PARP complex and, optionally,
c) comparing the result to a reference standard.

**23.** The method according to claim 22, **characterized in that** the binding partner is an antibody or a binding fragment thereof, optionally carrying a detectable label.

**24.** The method according to any of claims 21 to 23 for diagnosis of disorders mediated by energy deficiency.

**25.** A method for determining the efficiency of PARP inhibitors, **characterized in that** it comprises

a) incubating a PARP homologue according to any one of claims 1 to 4 with an analyte comprising a PARP inhibitor; optionally removing the inhibitor again; and
b) determining the activity of the PARP homologue, optionally after adding substrates or cosubstrates.

**26.** An composition for gene therapy, **characterized in that** it comprises, within a carrier acceptable for gene therapy, a nucleic acid construct encoding

a) an antisense nucleic acid for a coding nucleic acid according to claim 6 or 7; or
b) a ribozyme for a nucleic acid according to claim 6 or 7.

**27.** A pharmaceutical composition, comprising, within a pharmaceutically acceptable carrier, at least a PARP protein according to any one of claims 1 to 4, at least one PARP binding partner according to claim 5 or at least one coding nucleotide sequence according to claim 6 or 7.

**28.** Use of a PARP binding partner according to claim 5 for the manufacture of a pharmaceutical composition for the diagnosis or therapy of disorders mediated by energy deficiency.

**Revendications**

**1.** Homologues de poly(ADP-ribose)polymérase (PARP) et leurs équivalents fonctionnels, **caractérisés par** une séquence d'acide aminé, qui présente

a) un domaine de liaison $NAD^+$ fonctionnel et
b) aucun motif de séquence de Finger - Zink de formule générale

$$CX_2CX_mHX_2C$$

dans laquelle
m représente un nombre entier de 28 ou 30 et
les radicaux X représentent, indépendamment les uns des autres, un acide aminé quelconque ;

où le domaine de liaison $NAD^+$ fonctionnel comprend le motif de séquence

$PX_n(S/T)GX_3GKGIYFA$,

dans laquelle
n représente un nombre entier de 1 à 5 et les radicaux X, indépendamment les uns des autres, représentent un acide aminé quelconque.

**2.** Homologues de PARP selon la revendication 1, **caractérisés en ce que** le domaine de liaison $NAD^+$ fonctionnel d'un des motifs de séquence généraux suivants comprend :

$(S/T)XGLR(IIV)XPX_n(S/T)GX_3GKGIYFA$ ou
$LLWHG(S/T)X_71L(S/T)XLGR(I/V)XPX,(S/T)GX_3GKGIYFAX_3SKSAXY$

dans lesquels n et X sont définis comme dans la revendication 1.

**3.** Homologues de PARP selon l'une des revendications précédentes, comprenant au moins un autre des motifs de séquence partielle suivants :

$LX_9NX_2YX_2QLLX(D/E)X_{10}/_{11}$ WGRVG,
$AX_3FXKX_4KTXNXWX_5FX_3PXK$,
$QXL(I/L)X_2IX_9MX_{10}PLGKLX_3QIX_6L$,
$FYTXIPHXFGX_3PP$ ; et
$KX_3LX_2LXDIEXAX_2L$,

dans lesquels les radicaux X, indépendamment les uns des autres, représentent un acide aminé quelconque.

**4.** Homologues de PARP selon l'une des revendications précédentes, choisis parmi les homologues de PARP humains, **caractérisés par** la séquence d'acide aminé selon la SEQ ID N°2 ou SEQ ID N°4 ou 6 ; ou les homologues de PARP murins, **caractérisés par** la séquence d'acide aminé selon la SEQ ID N°8 ou la SEQ ID N°10.

**5.** Partenaire de liaison pour les homologues de PARP selon l'une des revendications précédentes, choisi parmi les anticorps et fragments de ceux-ci.

**6.** Acide nucléique, comprenant

a) une séquence de nucléotide codant au moins un homologue de PARP selon l'une des revendications 1 à 4 ou la séquence de nucléotide complémentaire de celle-ci ;
b) des séquences de nucléotide, qui sont dérivées, par dégénération du code génétique, des séquences de nucléotide définies au a) et qui codent une protéine ayant une activité poly-ADP-ribosylante.

**7.** Acide nucléique selon la revendication 6, comprenant :

a) les nucléotides + 3 à + 1715 selon la SEQ ID N°1 ;
b) les nucléotides + 242 à + 1843 selon la SEQ ID N°3 ;
c) les nucléotides + 221 à + 1843 selon la SEQ ID N°5 ;
d) les nucléotides + 112 à + 1710 selon la SEQ ID N°7 ;
ou
e) les nucléotides + 1 à + 1584 selon la SEQ ID N°9.

**8.** Cassette d'expression, contenant au moins une séquence de nucléotide selon l'une des revendications 6 et 7 sous le contrôle génétique d'au moins une séquence de nucléotide régulatrice.

**9.** Vecteur recombinant, comprenant au moins une cassette d'expression selon la revendication 8.

**10.** Micro-organisme recombinant, contenant au moins un vecteur recombinant selon la revendication 9.

**11.** Procédé de détection *in vitro* pour des inhibiteurs de PARP, **caractérisé en ce que** l'on

a) incube une cible poly-ADP-ribosylable non supportée ou supportée avec un mélange réactionnel comprenant :

a1) un homologue de PARP selon l'une des revendications 1 à 4,
a2) un ADN activé comme activateur de PARP ; et
a3) un inhibiteur de PARP ou un analyte, dans lequel on suppose la présence d'au moins un inhibiteur de PARP ;

b) réalise la réaction de poly-ADP-ribosylation ; et
c) détermine qualitativement ou quantitativement la poly-ADP-ribosylation.

**12.** Procédé selon la revendication 11, **caractérisé en ce que** l'on pré-incube l'homologue de PARP avec l'activateur de PARP et l'inhibiteur de PARP ou un analyte, dans lequel on suppose la présence d'au moins un inhibiteur de PARP, avant de réaliser la réaction de poly-ADP-ribosylation.

**13.** Procédé selon l'une des revendications 11 et 12, **caractérisé en ce que** la cible poly-ADP-ribosylable est une protéine d'histone.

**14.** Procédé selon l'une des revendications 11 à 13, **caractérisé en ce que** l'on démarre la réaction de poly-ADP-ribosylation par l'addition de $NAD^+$.

**15.** Procédé selon l'une des revendications 11 à 14, **caractérisé en ce que** l'on détermine la poly-ADP-ribosylation de la cible supportée avec un anticorps anti-poly-(ADP-ribose).

**16.** Procédé selon l'une des revendications 11 à 14, **caractérisé en ce que** la cible non supportée est marquée avec un fluorophore accepteur.

**17.** Procédé selon la revendication 16, **caractérisé en ce que** l'on détermine la poly-ADP-ribosylation de la cible non supportée avec un anticorps anti-poly-(ADP-ribose), qui est marqué avec un fluorophore donneur, qui est capable de transférer de l'énergie sur le fluorophore accepteur.

**18.** Procédé selon l'une des revendications 16 et 17, **caractérisé en ce que** la cible est l'histone biotinylée et que le fluorophore accepteur y est couplé par le biais de l'avidine ou de la streptavidine.

**19.** Procédé selon l'une des revendications 17 et 18, **caractérisé en ce que** l'anticorps anti-poly-(ADP-ribose) porte un cryptate d'europium comme fluorophore donneur.

**20.** Procédé de criblage *in vitro* des partenaires de liaison pour une molécule de PARP, **caractérisé en ce que** l'on

a1) immobilise sur un support au moins un homologue de PARP selon l'une des revendications 1 à 4 ;
b1) met en contact l'homologue de PARP immobilisé avec un analyte, dans lequel on suppose la présence d'au moins un partenaire de liaison ; et
c1) détermine les composants de l'analyte liés à l'homologue de PARP immobilisé, le cas échéant après une phase d'incubation;

ou

a2) immobilise sur un support un analyte, qui contient au moins un partenaire de liaison possible pour une molécule de PARP ;
b2) met en contact l'analyte immobilisé avec au moins un homologue de PARP, selon l'une des revendications 1 à 4, pour lequel on cherche un partenaire de liaison ; et
c2) analyse l'analyte immobilisé sur le plan de la liaison à l'homologue de PARP, le cas échéant après une phase d'incubation.

**21.** Procédé pour la détermination qualitative ou quantitative d'acides nucléiques codant des homologues de PARP selon l'une des revendications 1 à 4, **caractérisé par**

a) l'incubation d'un échantillon biologique avec une quantité définie d'un acide nucléique exogène selon l'une des revendications 6 et 7, l'hybridation dans des conditions stringentes, la détermination des acides nucléiques hybridants et le cas échéant la comparaison avec un standard ; ou
b) l'incubation d'un échantillon biologique avec une paire d'amorces oligonucléotidiques ayant une spécificité pour un acide nucléique codant un homologue de PARP selon l'une des revendications 6 et 7, l'amplification de l'acide nucléique, la détermination du produit d'amplification et le cas échéant la comparaison avec un standard.

**22.** Procédé pour la détermination qualitative ou quantitative d'un homologue de PARP selon l'une des revendications 1 à 4, **caractérisé par**

a) l'incubation d'un échantillon biologique avec un partenaire de liaison spécifique pour un homologue de PARP selon la revendication 5,
b) la détection du complexe partenaire de liaison/PARP et le cas échéant
c) la comparaison du résultat avec un standard.

**23.** Procédé selon la revendication 22, **caractérisé en ce que** le partenaire de liaison est un anticorps ou un fragment liant de celui-ci, qui porte le cas échéant un marquage détectable.

**24.** Procédé selon l'une des revendications 21 à 23 pour diagnostiquer des maladies induites par une déficience énergétique.

**25.** Procédé pour la détermination de l'efficacité des inhibiteurs de PARP, **caractérisé en ce que** l'on

a) incube un homologue de PARP selon l'une des revendications 1 à 4 avec un analyte, qui contient un inhibiteur de PARP ; sépare de nouveau l'inhibiteur le cas échéant ; et
b) détermine l'activité de l'homologue de PARP, le cas échéant après addition de substrats ou de co-substrats.

**26.** Agent de thérapie génique, **caractérisé en ce qu'**il contient, dans un support acceptable sur le plan de la thérapie génique, un hybride d'acide nucléique, qui code

a) un acide nucléique antisens contre un acide nucléique codant selon l'une des revendications 6 et 7 ; ou
b) un ribozyme contre un acide nucléique selon l'une des revendications 6 et 7.

**27.** Produit pharmaceutique contenant dans un support pharmaceutiquement acceptable au moins une protéine de PARP selon l'une des revendications 1 à 4, au moins un partenaire de liaison de PARP selon la revendication 5 ou au moins une séquence de nucléotide codante selon la revendication 6 ou 7.

**28.** Utilisation de partenaires de liaison de PARP selon la revendication 5 pour la fabrication d'un produit pharmaceutique pour le diagnostic ou la thérapie des états pathologiques, qui sont induits par une déficience énergétique.

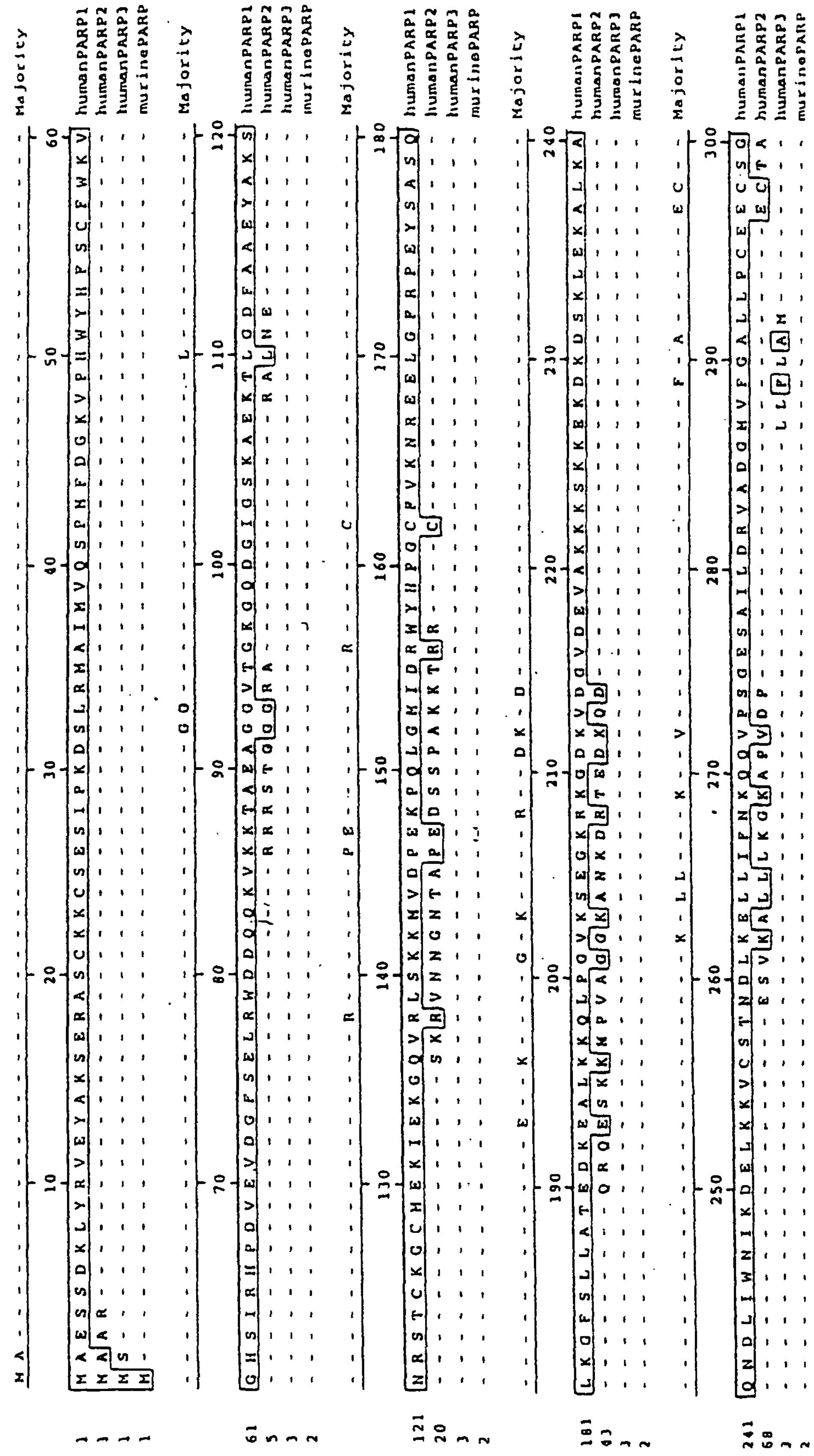


Fig. 1(1)

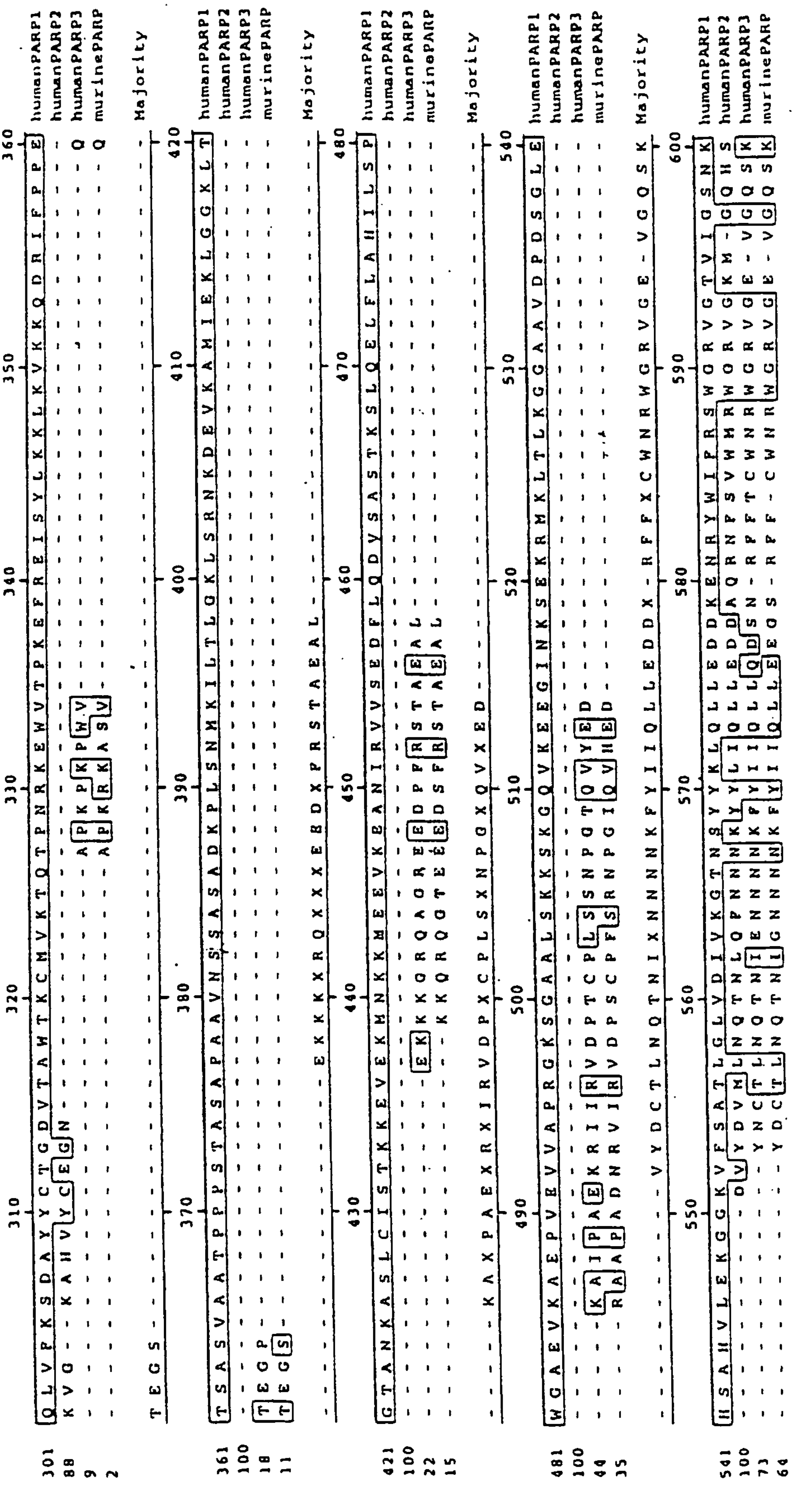

Fig. 1(2)

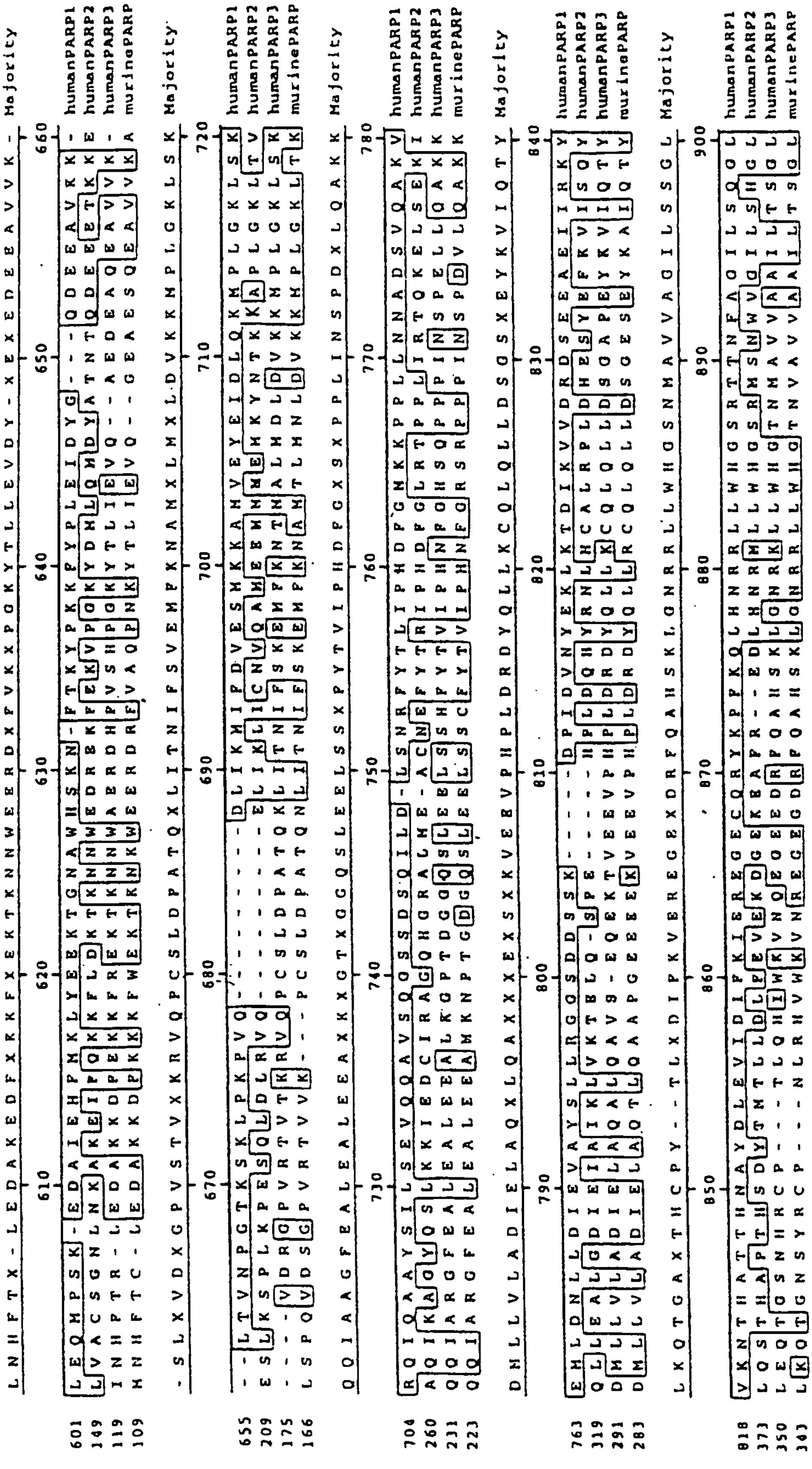

Fig. 1(3)

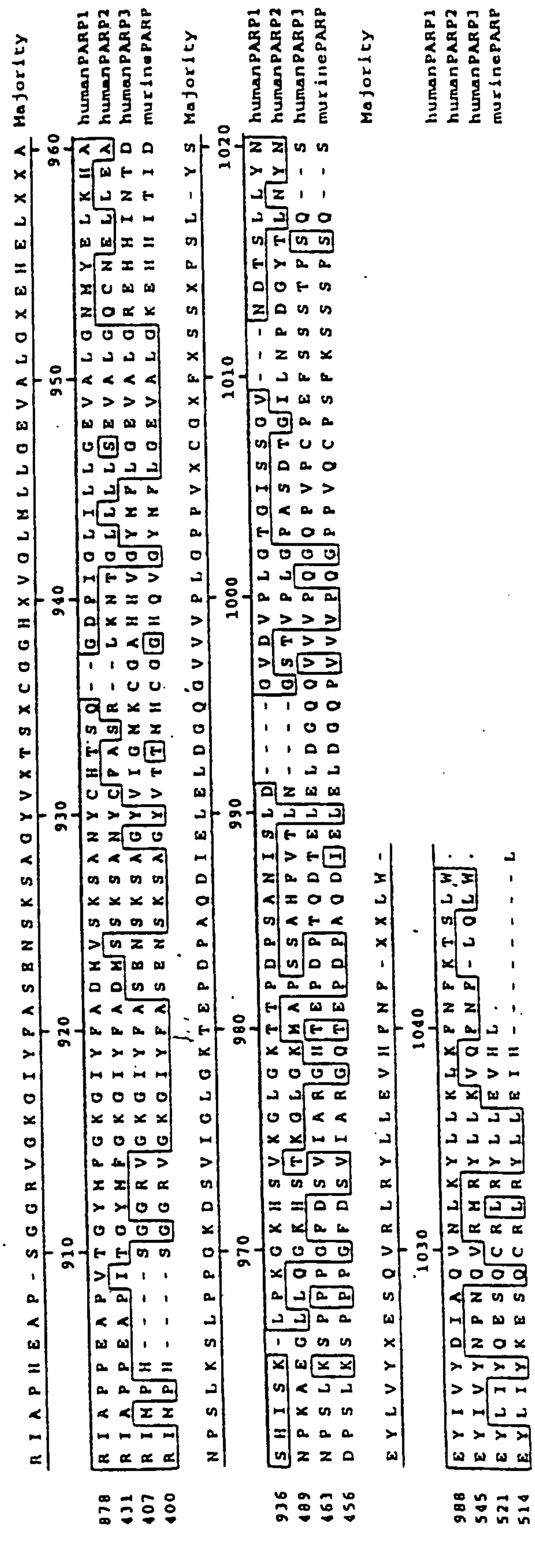

Fig. 1(4)

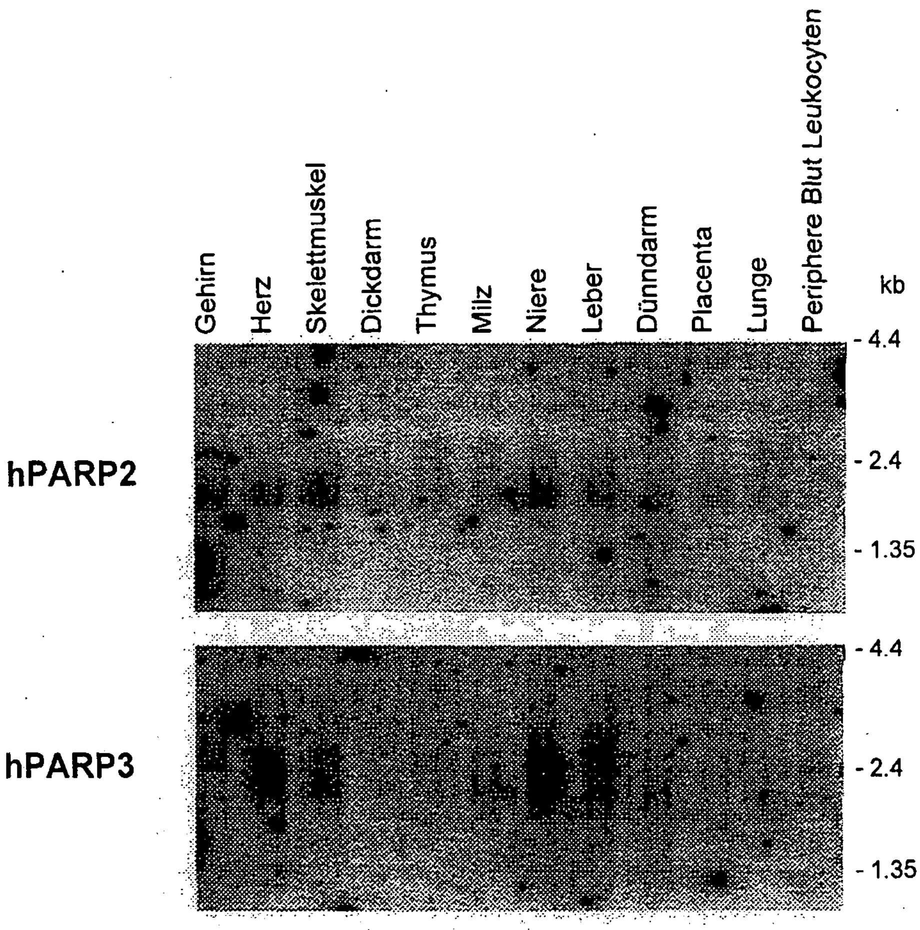


Fig. 2

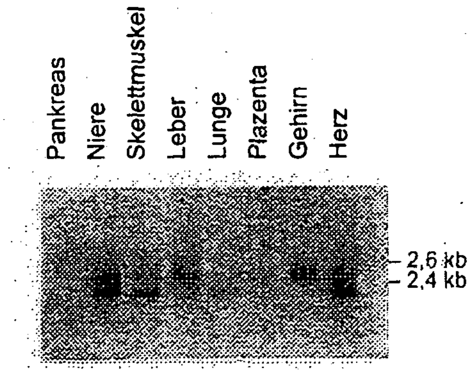


Northern Blot Analyse
Human PARP3 Gewebeverteilung

Fig. 3

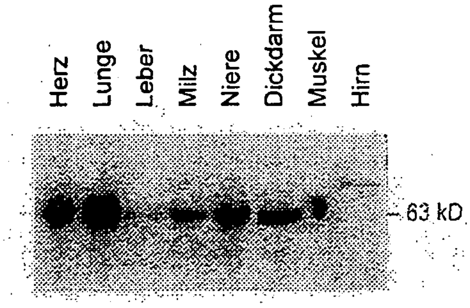


Western Blot Analyse
PARP3 Gewebeverteilung

Fig. 4

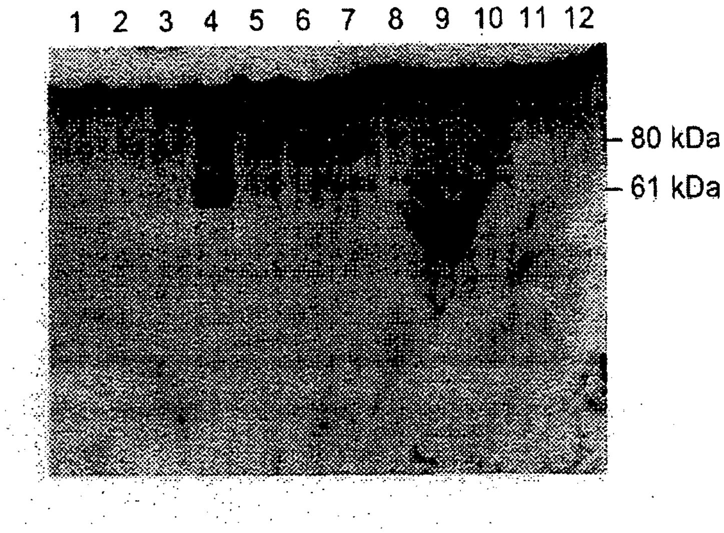


Fig. 5

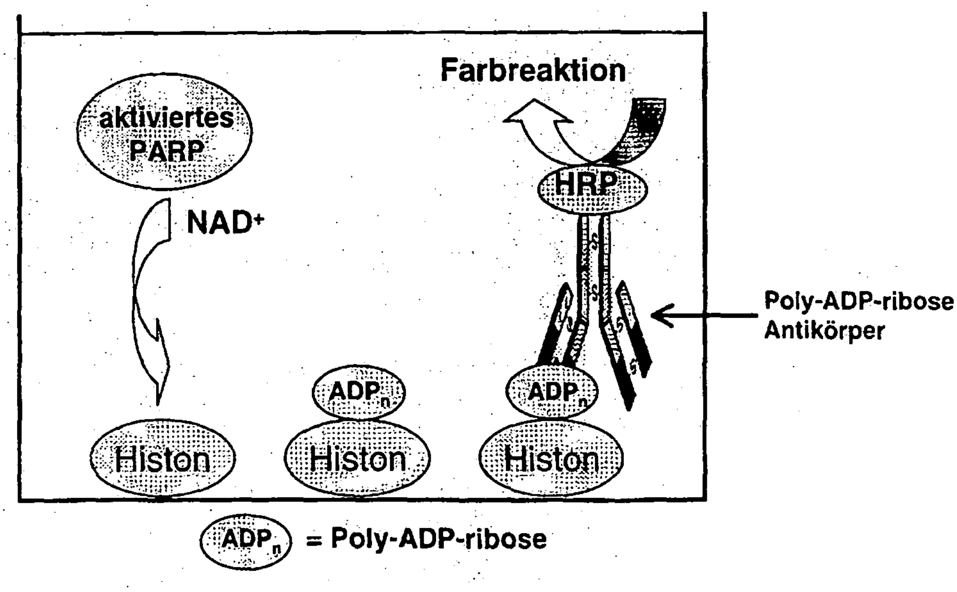


HRP = Meerettich-Peroxidase

Fig. 6

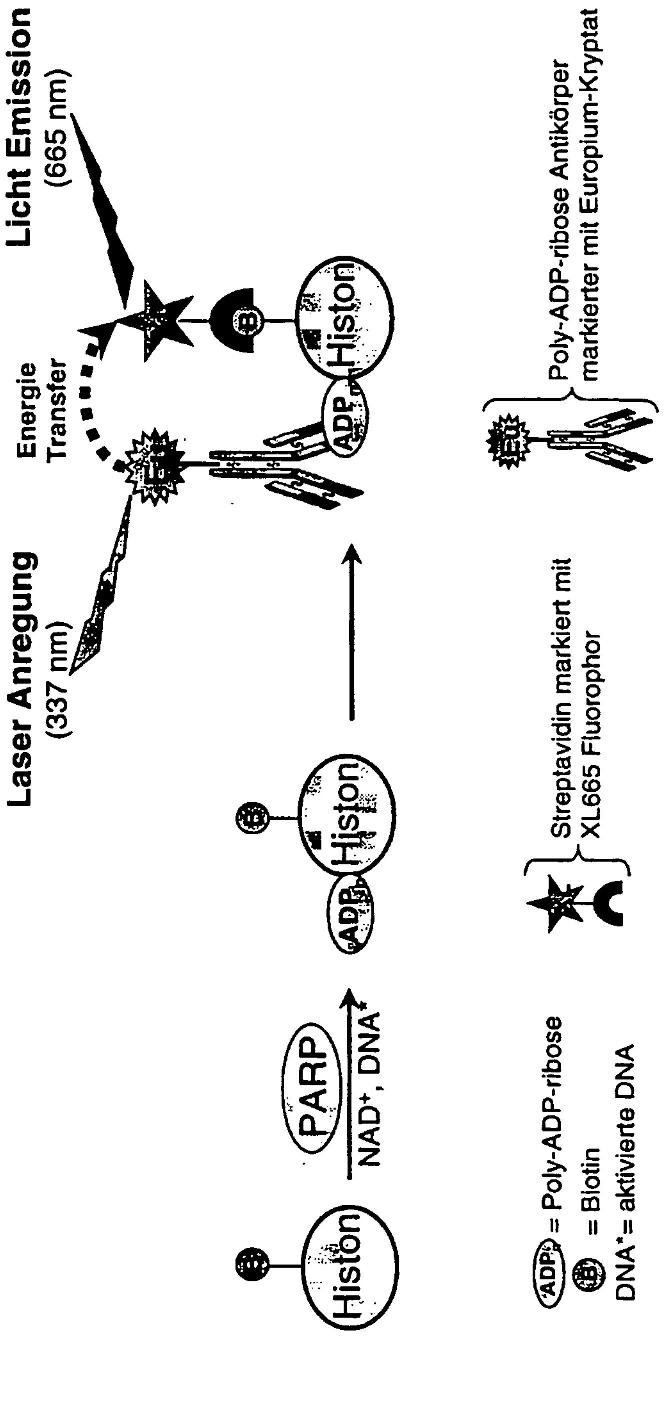
Laser Anregung
(337 nm)
Energie
Transfer
Licht Emission
(665 nm)
Histon
PARP
NAD+, DNA*
ADP
B
Eu
= Poly-ADP-ribose
= Biotin
DNA*= aktivierte DNA
Streptavidin markiert mit
XL665 Fluorophor
Poly-ADP-ribose Antikörper
markierter mit Europium-Kryptat

Fig. 7